(19) Europäisches Patentamt
European Patent Office
Office européen des brevets

(11) EP 0 257 993 B1

(12) **EUROPEAN PATENT SPECIFICATION**

(45) Date of publication and mention of the grant of the patent: **27.11.1996 Bulletin 1996/48**

(51) Int Cl.[6]: **C07H 21/04**, C12N 15/82, C12N 9/88, C12N 1/20, A01H 1/00, A01H 5/10, A01N 25/00, A01N 47/36

(21) Application number: **87307384.5**

(22) Date of filing: **20.08.1987**

(54) **Nucleic acid fragment encoding herbicide resistant plant acetolactate synthase**

Herbizid-resistante Pflanzen-Acetolactatsynthase kodierendes Nucleinsäurefragment

Fragment d'acide nucléique codant la synthase acétolactate végétale résistante aux herbicides

(84) Designated Contracting States:
**AT BE CH DE ES FR GB GR IT LI LU NL SE**

(30) Priority: **26.08.1986 US 900609**

(43) Date of publication of application:
**02.03.1988 Bulletin 1988/09**

(60) Divisional application: **96105411.1**

(73) Proprietor: **E.I. DU PONT DE NEMOURS AND COMPANY**
**Wilmington Delaware 19898 (US)**

(72) Inventors:
- **Bedbrook, John Robert**
  **Piedmont, CA 94611 (US)**
- **Chaleff, Roy Scott**
  **Chadds Ford, PA 19317 (US)**
- **Falco, Saverio Carl**
  **Arden, DE 19810 (US)**
- **Mazur, Barbara Jean**
  **Wilmington, DE 19803 (US)**
- **Yadav, Narendra Singh**
  **Wilmington, DE 19810 (US)**

(74) Representative: **Hildyard, Edward Martin et al**
**Frank B. Dehn & Co.,**
**European Patent Attorneys,**
**179 Queen Victoria Street**
**London EC4V 4EL (GB)**

(56) References cited:
**EP-A- 0 154 204** **EP-A- 0 292 435**

- **SCIENCE, vol. 224, 29 June 1984, pages 1443-1445; R.S. CHALEFF et al.: "Acetolactate synthase is the site of action of two sulfonylurea herbicides in higher plants"**
- **ULCA SYMP. SER., vol. 48, UCLA SYMP. MOLECULAR STRATEGIES FOR CROP PROTECTION HELD, 30th March - 6th April 1986, pages 415-425, Steamboat Springs Co., US; R.S. CHALEFF et al.: "Developing plant varieties resistant to sulfonylurea herbicides"**
- **WORLD BIOTECH REPORT, vol. 2, PROCEEDINGS OF BIOTECH'85, Washington, DC, October 1985, pages 97-108; B.J. MAZUR et al.: "Cloning herbicide resistance genes into and out of plants"**
- **TRENDS IN GENETICS, April 1986, pages 89-93; M.A. ESTELLE et al.: "The mutants of Arabidopsis"**
- **NUCLEIC ACIDS RESEARCH, vol. 13, no. 11, 1985; S.C. FALCO et al., p. 4011**
- **NATO ASI SER. A, vol. 140 (Plant. Mol. Biol.), D.v. Wettstein et al. (eds.), Proceedings of Nato Advanced Study Institute on Plant Molecular Biology, Cansberg, 10-19 June 1987; B.J. MAZUR et al.**
- **BIOLOGICAL ABSTRACTS/RMM; C.R. SOMERVILLE et al.; AN 30:49417**
- **PROCEEDINGS OF THE NATL. ACADEMY OF SCIENCES USA, vol. 83, June 1986, Washington, DC (US); N. YADAV et al., pp. 4418-4422**

Remarks:
The file contains technical information submitted after the application was filed and not included in this specification

EP 0 257 993 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to nucleic acid fragments encoding a herbicide-resistant form of the enzyme acetolactate synthase (ALS).

Sulfonylurea herbicides such as sulfometuron methyl (I) and chlorsulfuron (II) inhibit growth of some bacteria, yeast and higher plants by blocking acetolactate synthase [ALS, EC 4.1.3.18], the first common enzyme in the biosynthesis of the branched-chain amino acids valine, leucine and isoleucine. The biosynthesis of branched-chain amino acids and, hence. the toxicity of sulfonylurea herbicides is restricted to plants and microbes. ALS is also inhibited by a structurally unrelated class of herbicides, the imidazolinones.

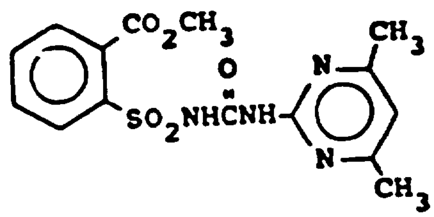

I

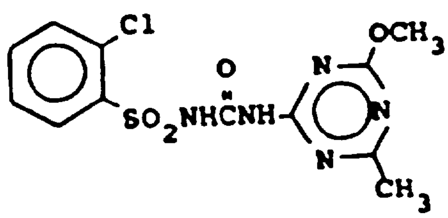

II

Three major isozymes of ALS, designated I. II and III, have been identified in enteric bacteria. Isozymes I and III, but not II, are sensitive to end-product inhibition by valine. Each of the three bacterial isozymes comprises a large and a small protein subunit. ALS enzymes from the yeast Saccharomyces cerevisiae and from some higher plants have been partially characterized and show some degree of end-product inhibition. It is not known if the yeast and plant ALS enzymes consist of one or more different polypeptides. Evidence suggests that the cellular locations of the yeast and plant ALS enzymes are in the mitochondria and chloroplasts, respectively.

Genes encoding ALS enzymes have been isolated from the enteric bacteria Salmonella typhimurium and Escherichia coli, and the yeast S. cerevisiae. The nucleotide sequences of the genes coding for the two subunits of E. coli ALS isozymes I, II and III show that they are organized as operons ilvBN, ilvGM and ilvIH, respectively. Comparison of the deduced amino acid sequences of the large subunits of the E. coli ALS isozymes shows three regions with about 50% conserved amino acids, comprising about two-thirds of the proteins, and separated by regions sharing little discernible homology. Amino acid sequence conservation, though less extensive, is also evident among the small subunits of the bacterial isozymes. In the yeast S. cerevisiae, a single gene. ILV2. essential for ALS activity was identified. Nucleotide sequence analysis of the ILV2 gene has revealed that the polypeptide encoded by it is homologous to the large subunits of the bacterial ALS isozymes. The deduced amino acid sequence of the yeast ALS shows the same degree of structural organization and the same degree of homology as is observed between the large subunits of the bacterial isozymes, except for about ninety amino acids at the amino terminus of the yeast protein that are believed to be involved in the translocation of the protein into the mitochondrion. No information on the structure of plant genes encoding ALS or the amino acid sequence of plant ALS enzymes was available prior to the inventions disclosed herein.

Yadav et al (Proc. Natl. Aca. Sci. USA 83 4418-4422) discloses yeast ALS enzymes which have no operability in plants. Mazur et al (World Biotech Report 2, Proceedings of Biotech '85, 97-108) reported that a nucleic acid fragment encoding sulfonylurea-resistant yeast ALS enzyme had been introduced into plant cells. Low level resistance of the plant cells to the herbicide was reported, but this was subsequently found not to be reproducible. Further work has shown that the DNA fragments disclosed do not, in fact, transform plant cells.

EP-A-0154204 relates to plants which are resistant to inhibition of certain herbicides, but gives no guidance as to the particular sequences of nucleic acid which confer such properties.

Enteric bacterial isozyme I is the only ALS in nature that is known to be insensitive to inhibition by sulfometuron methyl and chlorsulfuron. Therefore. enteric bacteria are sensitive to these herbicides only in the presence of valine, which inhibits isozyme I. Sulfonylurea herbicide-resistant mutant forms of the enteric bacteria Salmonella typhimurium and E. coli (selected in the presence of valine), the yeast S. cerevisiae and the higher plants Nicotaiana tabacum (tobacco). Arabidopsis thaliana and Zea mays (corn) have been identified. These mutant phenotypes cosegregate with herbicide-resistant forms of ALS through genetic crosses. In S. typhimurium the herbicide-resistance mutations are genetically linked to a gene encoding ALS, and in E. coli and S. cerevisiae, these mutations reside in the structural genes for ALS. In the higher plants the mutations responsible for the resistance are inherited as single, dominant or semidominant nuclear traits. In tobacco, these mutations map to either of two unlinked genetic loci.

The chemical control of undesirable weeds associated with agronomically useful crops requires the use of highly selective chemical herbicides. In some cases, it is difficult to identify any chemical which kills weeds without injury to the crop plant. The introduction of herbicide-resistance as a biological trait in crop plants would overcome this difficulty.

Although many genes involved in the structure and function of differentiated plant tissues and organs are not expressed in undifferentiated tissues, those involved in basic cellular functions are expressed and can be selected for in a disorganized callus or cell suspension culture. This has been demonstrated in many cases by the selection of a phenotype in tissue culture from which plants expressing the same phenotype have been regenerated. Examples include the in vitro selection of plants resistant to herbicides, pathotoxins or diseases, antibiotics, amino acid analogues, salt tolerance, etc. (reviewed in Chaleff, 1981; Evans et al, 1983; Vasil, 1986).

Since acetolactate synthase is an enzyme involved in the basic cellular metabolic activity of amino acid biosynthesis, it was expected and has been demonstrated that genes encoding this enzyme are expressed in callus tissue as well as the whole plant. The sulfonylurea resistant tobacco mutants described in this patent, S4, C3 and Hra, were first selected in tissue culture and subsequently regenerated into whole plants in which the resistant phenotypes were retained in a genetically stable manner (Chaleff and Ray, 1984). Callus tissues derived from regenerated plants or their progeny continue to grow on concentrations of the herbicide which inhibit the growth of wild type callus. Thus resistance to a sulfonylurea herbicide at the plant cellular level is predictive of resistance at the whole plant level. In addition, it has been demonstrated in bacteria, yeast and higher plants that mutations resulting in the production of herbicide resistant ALS are sufficient to confer resistance at the cellular level and, in the case of plants, at the whole plant level. Therefore, the observation of herbicide-resistant ALS in extracts of plant cells is also predictive of herbicide resistant growth of culture plant cells and herbicide resistant growth of whole plants.

There are limitations to obtaining herbicide-resistant crop plants through tissue culture or seed mutagenesis: 1) the tissue culture method is restricted to those crop species that can be manipulated in and regenerated from tissue culture, 2) plants derived from seed mutagenesis, and possibly from tissue culture, might have non-desirable phenotypes which would require multiple genetic backcrosses to eliminate, and 3) transfer of the resistance gene by breeding would be restricted to cultivars of the same species as well as require multiple genetic back crosses. Therefore, the isolation of a nucleic acid fragment able to confer herbicide resistance and its subsequent introduction into crops through genetic transformation should allow rapid, cross-species transfer of herbicide resistance and overcome many of the above mentioned limitations.

Although genes isolated from one plant have been introduced and expressed in other plants, non-plant genes have been expressed in plants only as chimeric genes in which the encoding sequences of the non-plant genes have been fused to plant regulatory sequnces required for gene expression. However, it would be difficult to introduce herbicide resistance into plants by introducing chimeric genes consisting of bacterial or yeast genes for herbicide-resistant forms of ALS, since (a) these microbial ALS enzymes are believed to lack a specific signal (transit) peptide sequence required for uptake into plant chloroplasts, the cellular location of plant ALS, (b) the bacterial isozymes consist of two different polypeptide subunits, and (c) the microbial ALS enzymes may not function optimally in the foreign cellular environment of higher plants. Therefore, there is a need for nucleic acid fragments (1) which encode a herbicide-resistant form of plant ALS, and (2) which can confer herbicide resistance when introduced into herbicide sensitive plants.

According to one aspect of the present invention, there is provided a nucleic acid fragment comprising a nucleotide sequence, said nucleotide sequence corresponding at least in part to a plant acetolactate synthase gene and : said nucleic acid fragment conferring increased sulfonylurea herbicide resistance on a plant in which said fragment is oparative because the nucleotide sequence comprises at least one sequence which encodes one of the substantially conserved amino acid sub-sequences designated A, B, C, D, E, F and G in Figure 6. The nucleic acid fragment is further characterized in that at least one of the following conditions is met:

a) the nucleic acid fragment has a sequence which encodes an amino acid sub-sequence A wherein $\varepsilon_1$ is an amino acid other than alanine, and/or $\varepsilon_2$ is an amino acid other than glycine,

b) the nucleic acid fragment has a sequence which encodes an amino acid sub-sequence B wherein $\alpha_1$ is an amino acid other than proline,

c) the nucleic acid fragment has a sequence which encodes an amino acid sub-sequence C wherein $\delta_2$ is an amino acid other than alanine,

d) the nucleic acid fragment has a sequence which encodes an amino acid sub-sequence D wherein $\lambda_1$ is an amino acid other than lysine,

e) the nucleic acid fragment has a sequence which encodes an amino acid sub-sequence E wherein $\gamma_1$ is an amino acid other than aspartic acid,

f) the nucleic acid fragment has a sequence which encodes an amino acid sub-sequence F wherein $\beta_3$ is an amino acid other than the tryptophan, and/or $\beta_8$ is an amino acid other than valine and/or $\beta_7$ is an amino acid other than phenylalanine, and

g) the nucleic acid fragment has a sequence which encodes an amino acid sub-sequence G wherein $\sigma_1$ is an amino acid other than methionine.

In another embodiment, the instant invention provides the above nucleic acid fragment encoding plant acetolactate synthase which is capable of being incorporated into a nucleic acid construct suitable for transforming a plant containing wild-type acetolacetate synthase which is sensitive to a sulfonylurea herbicide compound, said nucleic acid fragment having at least one of the above identified point mutation relative to the wild-type nucleic acid fragment encoding plant acetolactate synthase such that upon transformation with said nucleic acid construction said plant contains and nucleic acid fragment and exhibits increased resistance to the application of said sulfonylurea herbicide compound.

In still another embodiment, the present invention provides nucleic acid constructs, monocotyledonous and dicotyledonous plants, and tissue cultures which contain the above specified nucleic acid fragment. The invention further provides methods for transforming plants with these specified fragments, selecting transformed plant cells, and growing transformed plants.

The present invention also provides a method for selecting plant cells transformed with the nucleic acid fragment of the present invention The method comprises introducing the fragment into plant cells whose growth is sensitive to inhibition by herbicides to which the ALS encoded by the fragment is resistant to form a transformed plant cell. The transformed plant cells whose growth is resistant to the selected herbicide are identified by selection at a herbicide concentration which inhibits the growth of the untransformed plant cells.

In another aspect, the present invention is a method for controlling unwanted vegetation growing at a locus where a herbicide-resistant, agronomically useful plant (transformed with the nucleic acid fragment of the present invention) has been cultivated. The method comprises applying to the locus to be protected an effective amount of herbicide. In still another aspect, the present invention provides a nucleic acid construct wherein a nucleic acid fragment encoding plant acetolactate synthase with increased sulfonylurea herbicide resistance as hereinbefore described is linked to a second nucleic acid fragment conferring a second trait such that when said nucleic acid construct is utilized to transform a plant, the expression of herbicide resistance by said plant upon application of sulfonylurea compounds can be utilized to detect the presence of said second nucleic acid fragment in said plant.

## Brief Description of the Drawings

Figure 1 is a physical map of nucleic acid insert fragments containing ALS genes isolated from a genomic library of DNA from the tobacco Hra mutant.

Figure 2 is a diagram of plasmid pAGS152 showing a physical map of the nucleic acid fragment from tobacco encoding a herbicide-resistant ALS.

Figure 3 is a physical map of a nucleic acid insert fragment in phage clone 35 isolated from genomic library of DNA from the tobacco C3 mutant.

Figure 4 is a nucleotide sequence, and the cognate deduced amino acid sequence, of a gene from the Hra mutant of tobacco encoding a herbicide-resistant form of ALS from tobacco.

Figure 5 is a nucleotide sequence, and the cognate deduced amino acid sequence, of a gene from the C3 mutant of tobacco encoding a herbicide-resistant form of ALS.

Figure 6 is a comparison of deduced amino acid sequence of the large subunits of bacterial ALS and the yeast and plant ALS enzymes.

Figure 7 is a physical map of a nucleic acid insert fragment and sub-fragments derived from phage clone 21 isolated from genomic library of sugarbeet DNA.

Figure 8 is a comparison of deduced amino acid sequences of plant ALS enzymes.

The present invention provides specified nucleic acid fragments which confer herbicide resistance when introduced into herbicide-sensitive plants. As used herein, the term "nucleic acid fragment" refers to a linear segment of single- or double-stranded deoxyribonucleic acid (DNA) or ribonucleic acid (RNA), which can be derived from any source.

Preferably, the nucleic acid fragment of the present invention is a segment of DNA. The term "plant" refers to a photosynthetic organism including algae, mosses, ferns, gymnosperms, and angiosperms. The term, however, excludes, prokaryotic and eukaryotic microorganisms such as bacteria, yeast, and fungi. "Plant cell" includes any cell derived from a plant, including undifferentiated tissue such as callus or gall tumor, as well as protoplasts, and embryonic and gametic cells. The term "plant acetolactate synthase" refers to the specified enzyme when expressed in a plant or plant cell. The term "nucleotide sequence" refers to a polymer of DNA or RNA which can be single- or double-stranded, optionally containing synthetic, non-natural, or altered nucleotides capable of incorporation into DNA or RNA polymers. As used herein, the expression "substantially conserved amino acid sequences" refers to regions of amino acid homology between polypeptides comprising ALS enzymes from different sources. In the present invention seven substantially conserved amino acid sequences, designated A, B, C, D, E, F, and G are shown in Figure 6. One skilled in the art could align the amino acid sequnces of ALS enzymes from different sources to the schematic of Figure 6 to identify the segments therein which are the substantially conserved amino acid sequences defined herein. The skilled person could then determine whether the identified segments have the characteristics disclosed and claimed in the present application. It is to be understood that the expression includes modification of the segments which do not adversely affect the activity of the ALS enzyme. The term "nucleic acid construct" refers to a plasmid, virus, autonomously replicating sequence, phage or linear segment of a single- or double-stranded DNA or RNA, derived from any source, which is capable of introducing a nucleic acid fragment into a biological cell.

"Regulatory nucleotide sequence", as used herein, refers to a nucleotide sequence located 5′ and/or 3′ to a nucleotide sequence whose transcription and expression is controlled by the regulatory nucleotide sequence in conjuction with the protein synthetic apparatus of the cell. As used herein, a "regulatory nucleotide sequence" can include a promoter region, as that term is conventionally employed by those skilled in the art. A promoter region can include an association region recognized by an RNA polymerase, one or more regions which control the effectiveness of transcription initiation in responst to physiological consitions, and a transcription initiation sequence.

"Transit peptide" refers to a signal polypeptide which is translated in conjunction with a polypeptide which by a product nucleotide sequence, forming a polypeptide precursor. In the process of transport to a selected site within the cell, for example, a chloroplast, the transit peptide can be cleaved from the remainder of the polypeptide precursor to provide an active or mature protein.

"Herbicide," as used herein, refers to an antibiotic compound which inhibits the metabolism, growth, or replication of plant cells or whole plants. Cells transformed with a construct of the present invention exhibit selectable cross-resistance to certain structurally related sulfonamide compounds effective as broad-spectrum preemergent and postemergent herbicides. As used herein in a generic sense, "sulfonylurea herbicides" refer to N-(heterocyclicaminocarbonyl)arylsulfonamide compounds exhibiting broad-spectrum herbicidal activity and low mammalian toxicity "Selective concentration" refers to a concentration of an inhibitor or antibiotic compound, for example, a herbicide, which is capable of inhibiting the metabolism, growth, or multiplication of wild-type cell or organism. Such an organism, as well as clones thereof, is referred to as a "sensitive" organism or cell. "Resistance" refers to a capability of an organism or cell to grow in the presence of selective concentrations of an inhibitor. In relation to particular enzymes or proteins, "sensitive" indicates that the enzyme or protein is susceptible to specific inhibition by a particular inhibiting compound, for example, an antibiotic or herbicide. In relation to particular enzymes or proteins, "resistant" indicates that the enzyme or protein, as a result of a different chemical structure, expresses activity in the presence of a selective concentration of a specific inhibitor which inactivates sensitive variants of the enzyme or protein. The term "selectable genetic market" refers to a nucleotide sequence which, when incorporated into the genome of an organism, allows growth of that organism and its progeny under conditions which inhibit growth of the organism lacking the selectable genetic marker. For example, a gene which encodes an enzyme that is resistant to specific inhibition by a particular antibiotic compound, such as a herbicide, can function as a selectable genetic marker by allowing an organism, such a plant, to grow and propagate in the presence of a selective concentration of the compound. A second nucleic acid fragment, controlling a property which is difficult to assay, can be covalently linked to the selectable genetic marker, in which case the presence of the selectable marker, indicated by growth of an organism under selective conditions, can be used to detect an organism containing the second nucleic acid fragment.

Preparation of DNA Fragments Encoding Herbicide-Resistant ALS

Callus cultures of sensitive tobacco (Nicotiana tabacum var. Xanthi) were exposed to sulfometuron methyl at 2 ppb according to the method described by Chaleff, U.S. 4,443,971. Resistant cell lines designated C3 and S4 were selected. Standard genetic analysis of plants regenerated from these cells lines indicated that the C3 and S4 lines each carried a single semi-dominant nuclear gene mutation responsible for the herbicide resistance trait and that the C3 and S4 mutations were not genetically linked, i.e. were in different genes designated SURA and SURB, respectively. The C3 and S4 lines were shown to produce ALS enzyme activity one-hundred fold more resistance to the sulfonylurea herbicides chlorsulfuron and sulfomenturon methyl than ALS from wild type. Production of herbicide resistance ALS

activity cosegregated in genetic crosses with resistance to growth inhibition by the herbicides. The observation of two different genes that had mutated to form herbicide resistance ALS was not unexpected because N. tabacum is believed to be an allotetraploid plant formed from N. tomentosiformis and N. sylvestris, essentially containing two complete genomes. Thus, the S4 and C3 cell lines each contain one mutant and one wild type ALS gene. The S4 cell line was exposed to sulfometuron methyl at 200 ppb, a selective concentration which completely inhibits the growth of S4. Cell lines resistant to 200 ppb were identified; one such line was designated Hra. Hra was shown to tolerate concentrations of sulfometuron methyl one thousand times greater than that required to completely inhibit the growth of wild type callus. Hra was shown to be cross resistant to chlorsulfuron. Plants were regenerated from Hra callus cultures. Genetic analysis of the plants demonstrated that the Hra and S4 mutations were linked indicating that the Hra line contained a second mutation in the mutant gene of the progenitor S4 line. ALS activity in extracts of leaves of wild type and homozygous Hra mutant tobacco plants was determined. The ALS activity in the extract from Hra mutant plants was about one thousand fold more resistant to chlorsulfuron than was the activity of the wild type plants. Hra mutant plants were further shown to be cross resistant to the foliar application of the following compounds:

- 2-[4,5-dihydro-4-methyl-4-(1-methylethyl)-5-oxo-1H-imidazol-2-yl]-2-pyridinecarboxylic acid, (1-methylethanamine) salt;
- 5-ethyl-4,5-dihydro-2-[4-methyl-4-(1-methylethyl)-5-oxo-1H-imidazol-2-yl]-3-pyridinecarboxylic acid;
- 2-(2-chloroethoxy)-N-[(4-methoxy-6-methyl-1,3,5-triazin-2-yl)aminocarbonyl]benzenesulfonamide;
- 2-chloro-N-[(4-methoxy-6-methyl-1,3,5-triazin-2-yl)aminocarbonyl]benzenesulfonamide;
- 2-[[(4-chloro-6-methoxypyrimidin-2-yl)aminocarbonyl]aminosulfonyl]benzoic acid, ethyl ester;
- N-[(4,6-dimethoxypyrimidin-2-yl)aminocarbonyl]-2,3-dihydro-2-methylbenzo[β]thiophene-7-sulfonamide, 1,1-dioxide;
- 7-chloro-N-[(4,6-dimethoxypyrimidin-2-yl)aminocarbonyl]-3,4-dihydro-2-methyl-2H-1,2-benzothiazine-8-sulfonamide, S,S-dioxide;
- 2-[[(4-methoxy-6-methylpyrimidin-2-yl)aminocarbonyl]aminosulfonyl]-6-methylbenzoic acid, methyl ester;
- 5,7-dimethyl-N-(2-methyl-6-nitrophenyl)[1,2,4]triazolo[1,5-A]pyrimidin-2-sulfonamide;
- 2-[4,5-dihydro-4-methyl-4-(1-methylethyl)-5-oxo-1H-imidazol-2-yl]-3-quinolinecarboxylic acid;
- 6-(4-isopropyl-4-methyl-5-oxo-2-imidazolin-2-yl)-m-toluic acid and p-toluic acid, methyl esters;
- 2-[[(4,6-dimethylpyrimidin-2-yl)aminocarbonyl]aminosulfonyl]benzoic acid, methyl ester;
- N-(2,6-dichlorphenyl)-5,7-dimethyl[1,2,4]triazolo [1,5-A]pyrimidin-2-sulfonamide;
- N-(2-chloro-6-methylphenyl)-5,7-dimethyl[1,2,4]triazolo[1,5-A]pyrimidin-2-sulfonamide.

In order to clone a herbicide resistant ALS gene, tobacco DNA was isolated from the S4 homozygous mutant line of Nicotiana tabacum. 50 g portions of callus tissue were frozen in liquid $N_2$, and then lyophilized. The resulting dried tissue was then ground at about 23°C in a blender, using 15 second bursts, until powdered. Ten volumes of a sucrose buffer (0.3 M sucrose, 50 mM Tris-HCl pH 8.0, 5mM $MgCl_2$) were added, and the resulting suspension was incubated at 0°C for 5 minutes. The suspension was then filtered through cheesecloth, and centrifuged at 350 x g for 10 minutes. The nuclear pellet was then resuspended in lysis buffer (20 mM EDTA, 50 mM Tris-HCl pH 8.0, 1% Sarkosyl), CsCl added to provide 0.95 g per mL buffer, and the resulting mixture centrifuged at 17,000 x g for 20 minutes at 4°. Ethidium bromide was added to the resulting supernatant to a concentration of 400 μg per mL, the reflective index was adjusted to 1.39, and the resulting solution centrifuged at 90,000 x g in a Beckman Ti70 rotor at 20°C for 3 days. The resulting fluorescent DNA band was removed from the gradient, and treated with isopropanol to extract the ethidium bromide. Finally, the DNA was dialyzed against TE buffer and precipitated by addition of ethanol.

A Nicotiana genomic library was prepared from this DNA as follows, using the phage lambda vector EMBL4 described by Frischauf et al., J. Mol. Bio. 170: 827 (1983). EMBL4 phage was prepared from agarose plate stocks prepared by the method of Davis et al; Advanced Bacterial Genetics, (Cold Spring Harbor Laboratory, New York, 1980). Phage DNA was prepared as described by Silhavy et al., Experiments with Gene Fusions, (Cold Spring Harbor Laboratory, New York, 1984), by concentrating phage with polyethylene glycol, removing the polyethylene glycol by chloroform extraction, and purifying phage using glycerol step gradients. The resulting purified phage was then treated with deoxyribonuclease and ribonuclease prior to phenol extraction. Phage DNA was spooled from ethanol. To prepare arms of the EMBL4 phage, phage DNA was sequentially digested with Sal I and Bam HI endonucleases. The arms were annealed and then separated from the central fragment on a 10-40% sucrose gradient, as described by Maniatis et al., Molecular Cloning: A Laboratory Manual. (Cold Spring Harbor Laboratory, New York, 1982). The arms were completely denatured and reannealed prior to ligation to tobacco DNA. Tobacco DNA, prepared as previously described, was partially digested with Sau3A endonuclease and sedimented through a 10-40% sucrose gradient. Fractions from the sucrose gradient were then analyzed by electrophoresis on 0.5% agarose gels. Fractions containing fragments in the 20-40 kb size range were dialyzed, precipitated, and ligated to the lambda phage DNA arms. The DNA was ligated at a concentration of 135μg per mL vector and 45 μg per mL insert DNA. The resulting ligated concatamers were then

packaged using lambda DNA packaging extracts. The resulting yield of phage was approximately 4.5 x $10^5$ phage per µg insert DNA. A library of approximately 400,000 phage was constructed, representing an estimated 99% complete library for tobacco, which has an approximate genomic content of 1.65 picograms, or 1.52 x $10^9$ base pairs (Zimmerman, et al., Chromosoma 59:227 1977).

The resulting phage library of Nicotiana DNA was grown and plated on E. coli stain LE392 (ATCC 33572), as disclosed by Silhavy et al., Experiments with Gene Fusions, (Cold Spring Harbor Laboratory, New York, 1984). Phage were plated to provide 2000-5000 plaques on 90 mm petri dishes or 50,000 plaques on 150 mm petri dishes. Plaque lifts were done by the method of Benton et al., Science 196:180 (1977). Following transfer of phage DNA to nitrocellulose filters, the filters were prehybridized by incubation for about 4 hours at 56°C in 6 x SSPE containing 0.5% SDS, 100 µg per mL denatured calf thymus DNA, and 10 x Denhardt's solution. Hybridization was then accomplished as described by Maniatis, et al., Molecular Cloning: A Laboratory Manual, (Cold Spring Harbor Laboratory, New York, 1982) p. 326. In this step, a fresh aliquot of hybridization solution was added, together with about $10^8$ cpm of the radioactive yeast ALS gene probe. Hybridization was allowed to occur for about 24-48 hours at 56°C. At this point, the filters were first rinsed for about 4 hours in 6 x SSPE at 56°C, then rinsed three additional times for 20 minutes each in 2 x SSPE at about 23°C. The filters were then dried and exposed at -70°C for 2 days, using Kodak XAR or XRP x-ray film and a Du Pont Cronex® Lightning Plus™ intensifying screen. Exposed shots on the film indicated the position of plaques potentially containing Nicotiana ALS genes.

The autoradiograms prepared as described above were then oriented over the original bacteriophage-containing petri dishes. Using the wide end of a sterile Pasteur pipette, plaques corresponding to the darkest spots on the autoradiograms were excised. The plaques selected were then eluted into SM buffer and plated onto fresh 90 mm petri dishes. Each dish received about 100-200 phage. The complete phage location process was then reiterated, using freshly prepared probe. In this manner, the phage location and isolation steps were repeated until the majority of plaques indicated the presence of phage containing DNA capable of hybridization to the yeast ALS gene probe.

Mini-preparations of DNA from the plaque-purified phage designation NtA13 were isolated as described and worked up as described by Maniatis et al, Molecular Cloning; A Laboratory Manual (Cold Spring Harbor Laboratory, New York, 1982), p. 371. EcoRI restriction endonuclease digests of the DNA mini-preparations were electrophoresed through 0.7% agarose gels and blotted onto nitrocellulose filters. Fragments containing the ALS gene were then identified by hybridization with the yeast ALS gene probe. Fragments capable of hybridization to the probe were then isolated and subcloned into vectors pBR322, M13mp9, or M13mp18. These fragments were then sequenced using oligonucleotide primers in a dideoxy chain termination procedure conducted substantially as described by Sanger et al., Proc. Natl. Acad. Sci. USA 74:5463 (1977). A kit available from New England Biolabs (Beverly, Massachusetts, U. S.A.) was employed. Use of synthetic oligonucleotide primers allowed extension of a DNA sequence along a cloned fragment in overlapping segments. Computer analysis of the DNA sequence identified a 667 codon open reading frame. The deduced amino acid sequence of this open reading frame was substantially homologous to the sequences previously determined from the Sacharomyces cerevisiae ILV2 gene and the E. coli ilvG gene, indicating that the DNA fragment recovered from the Nicotiana genomic library contained a tobacco ALS gene. To determine whether this ALS gene encoded the wild type herbicide sensitive enzyme or the mutant herbicide resistant enzyme from the S4 line, the gene was introduced into wild type herbicide sensitive tobacco by Agrobacterium tumefaciens medicated transformation.

A genetic marker for selection of transformed plant cells was required. Resistance to the antibiotic G-418 resulting from expression in plants of a bacterial gene, NPT II, encoding neomycin phosphotransferase was used. To allow expression of NPT II, plant regulatory sequences were fused to the NPT II coding region in the vector pKNK. Vector pKNK was derived from the commonly used plasmid pBR322 by removing the Hind III BamH I sites and inserting at the Cla I site and approximately 2.3 kb Cla I fragment which consisted of the following:

a) a 320 bp Cla I-Bgl II sequence containing the promoter region of the neomycin phosphotransferase (NPT II) gene of transposon Tn 5 derived by the conversion of a Hind III site to the Cla I site [Beck, E., Ludwig, G., Auerswald, E. A., Reiss, B. & Schaller, H. (1982) Gene 19:327-336].
b) a 296 bp Sau 3A-Pst I sequence containing the nopaline synthase promoter (NOSO) derived from the nopaline synthase gene (NOS) (nucleotides -263 to +33, with respect to the transcription start site [Depicker, A., Stachel, S., Dhaese, P., Zambryski, P & Goodman, H. J. (1982) J. Mol. Appl. Genet. 1:561-574] by the creation of a Pst I site at the initiation codon.
c) the 998 bp Hind III- BamH I sequence containing the coding sequence for the NPT II gene derived from Tn 5 by the creation of Hind III and BamH I sites at nucleotides 1540 and 2518 [Beck, E., Ludwig, G., Auerswald, E. A., Reiss, B. & Schaller, H. (1982) Gene 19:327-336], respectively.
d) the 702 bp BamH I-Cla I sequence containing the 3′ region of the NOS gene (nucleotides 848 to 1550) [Depicker, A., Stachel, S., Dhaese, P., Zambryski, P. & Goodman, H. J. (1982) J. Mol. Appl. Genet. 1:561-574].

The nucleotide sequence at the fusion of the NOSP and the NPT II coding sequence is:

```
   NOS Sequence                         NPT II Sequence
... ...AATAATCTGCAGCAAGCTTGCGGGGATCGTTCGC ATG ... ...
              Pst 1  HindIII
```

Vector pKNK was cleaved by restriction enzyme Sal I (BRL) and the resultant linear vector, following phenol extraction, was joined in the presence of T4 DNA ligase (New England Biolabs) to purified 2.1 kb Sal I fragment carrying the bacterial neomycin phosphotransferase I (NPT I) gene as a selectable marker for bacterial resistance to kanamycin. The ligation mixture was used to transform competent E. coli HB101 cells, and the transformants were selected on plates containing 100 mg/L kanamycin. The resultant recombinant plasmid, designated pKNKS, was purified.

Plasmid pKNKS was cleaved with restriction enzyme Eco RI (BRL), and its ends made blunt by Klenow fragment (BRL). The resultant linear DNA was joined in the presence of T4 DNA ligase (New England Biolabs) to phosphorylated Bgl II linkers. Following extensive digestion with Bgl II restriction enzyme (BRL) to remove the excess linkers, the DNA was passed through a Sephadex G-150 (fine) (Pharmacia) gel filtration column to separate it from the linkers. The DNA fragment was isolated and the volume adjusted to yield a DNA concentration of approximately 78 μg/ml. 1 μl of the DNA was self-ligated in the presence of T4 DNA ligase (New England Biolabs) in total volume of 5 μl and used to transform competent E. coli HB101 cells. Ampicillin-resistant cells were shown to contain the plasmid, pKNKSG, which is identical to pKNKS except for the replacement of the EcoRI restriction site with that of Bgl II.

A Sma I fragment of phage NtA13 was shown to contain the region that hybridized to the yeast ALS gene. Phage NtA13 was partially digested by restriction enzyme Sma I (New England Biolabs), and following phenol extraction, joined to phosphorylated BamH I linkers (New England Biolabs) in the presence of T4 DNA ligase (New England Biolabs). Following digestion with BamH I and removal of excess linkers by electrophoresis on an agarose gel, a 16 kb BamH I restriction fragment containing the tobacco ALS gene from phage NtA13 was isolated from the gel by electroelution and used for further cloning.

Vector pKNKSG was linearized with restriction enzyme Bgl II (BRL) and, following dephosphorylation by calf intestine phosphatase (Boehringer Mannheim), it was joined in the presence of T4 DNA ligase (New England Biolabs) to the 16 kb BamH I restriction fragment derived from phage NtA13. The ligation mixture was used to transform competent E. coli HB101 cells and an ampicillin-resistant transformant was shown to contain the recombinant plasmid designated pIV13. The orientation of the insert fragment in pIV13 was such that the open reading frames of the NOS: NPT II gene in the vector and the ALS gene in the insert were in opposite directions. HB101 (pIV13), following purification by three single colony streaking, was used for triparental mating.

Three ml overnight cultures of E. coli HB101 (pIV13) and E. coli HB101 (pRK2013) (ATCC number 37159) in LB liquid medium containing 25 mg/L kanamycin were grown at 37°C, and of Agrobacterium tumefaciens GV3850 in LB medium at 28-29°C. The cells were harvested at room temperature in a clinical centrifuge, washed once in LB medium without drug, harvested, and resuspended in 3 ml of LB. 0.25 ml aliquots of all three strains were mixed in a tube and the mixture was transferred onto a Millipore filter (2.5 cm HAWP, 0.45 μm) placed on top of three Whatman No. 1 filters in a petri dish. After all of the liquid medium was absorbed by the Whatman filter (about 30 min), the Millipore filter with bacteria on its top surface was laid (bacteria side up) onto a LB plate without drug. After incubation overnight at 28-29°C, the Millipore filter was transferred to 5 ml of 10 mM $MgSO_4$ and vortexed to resuspend the bacteria in the solution. 0.1 ml aliquots were plated on selective plates [M9 minimal plates containing 20% sucrose, 1 mM $MgSO_4$, 1 mM $CaCl_2$, and 1 mg/ml kanamycin (Sigma)]. Several large colonies showed up after about four days of incubation at 28-29°C. Several transconjugants were purified by three successive single-colony streakings on the same selective plates. Only Agrobacteria containing the plasmid pIV13 recombined with the endogenous pGV3850 plasmid through their common pBR322 sequences were expected to grow. This was confirmed by Southern analysis before using the engineered Agrobacterium, GVKNT13, for plant transformations.

For plant cell transformations, standard aseptic techniques for the manipulation of sterile media and axenic plant/bacterial cultures were followed, including the use of a laminar flow hood for all transfers. Recipes for media are given in Example VI. Potted tobacco plants for leaf disk infections were grown in a growth chamber maintained for a 12 hr. 24°C day, 12 hr, 20°C night cycle, with approximately 80% relative humidity, under mixed cool white fluorescent and incandescent lights. Tobacco leaf disk infections were carried out essentially by the method of Horsch et al. (1985) Science 227, 1229.

Young leaves, not fully expanded and approximately 4-6 inches in length, were harvested with a scalpel from approximately 4-6 week old tobacco plants (Nicotiana tabacum var. Xanthi). The leaves were surface sterilized for 30 minutes by submerging them in approximately 500 ml of a 10% Chlorox, 0.1% SDS solution and then rinsed 3 times with sterile deionized water. Leaf disks, 6 mm in diameter, were prepared from whole leaves using a sterile paper punch.

Leaf disks were inoculated by submerging them for several minutes in 20 ml of a 1:10 dilution of an overnight Agrobacterium culture carrying the plasmid GCKNT13. Agrobacterium cultures were started by inoculating 10 ml of YEB broth with a single bacterial colony removed form a R-agar plate. The culture was grown for approximately 17-20 hours in 18 mm glass culture tubes in a New Brunswick platform shaker maintained at 28°C.

After inoculation, the leaf disks were placed in petri dishes containing CN agar medium. The dishes were sealed with paraflm and incubated under mixed fluorescent and "Gro and Sho" plant light (General Electric) for 2-3 days in a culture room maintained at approximately 25°C.

To rid the leaf disks of Agrobacterium and to select for the growth of transformed tobacco cells, the leaf disks were transferred to fresh CN medium containing 500 mg/L cefotaxime and 100 mg/L kanamycin. Cefotaxime was kept as a frozen 100 mg/ml stock solution and added aseptically (filter sterilized through a 0.45 μm filter) to the media after autoclaving. A fresh kanamycin stock (50 mg/ml) was made for each use and was filter sterilized into the autoclaved media.

Leaf disks were incubated under the growth conditions described above for 3 weeks and then transferred to fresh media of the same composition.

Approximately 1-2 weeks later, shoots developing on medium containing kanamycin were excised with a sterile scalpel and planted in A medium containing either 10 ppb chlorsulfuron or 100 mg/L kanamycin. Root formation on selective and non-selective media was recorded within 3 weeks.

Within 2 weeks of planting, small leaves were removed from excised shoots to determine levels of resistance to chlorsulfuron and kanamycin in a callus induction assay on selective media. To induce callus formation, small leaves were excised and cut into several sections with a scalpel and planted in B medium containing either 10 ppb chlorsulfuron or 50 mg/L kanamycin. Callus growth on selective and non-selective media was recorded within 3 weeks.

The results shown in Table 1 indicated that transformation of tobacco had been achieved with the GVKNT13 strain based on production of kanamycin resistant callus. The kanamycin resistant callus remained sensitive to the sulfonylurea herbicide chlorsulfuron, indicating that the ALS gene isolated from the tobacco S4 mutant in phage NtA 13 encoded the wild type herbicide sensitive enzyme. This plant ALS gene has been used as a DNA hybridization probe to isolate other plant ALS genes, including genes which encode herbicide resistant ALS.

Table 1

| Results from Callus Tests of GVKNT13 Infected Tobacco | | | |
|---|---|---|---|
| Number of transformed shoot explants producing callus on selective and non-selective media. | | | |
| | GVKNT13[1] | GVKK[2] | GV3850[3] |
| EXP. #1 | | | |
| No selection | 59/62 | 12/13 | 10/10 |
| Kanamycin, 50 mg/L | 53/62 | 8/13 | 0/10 |
| Chlorsulfuron, 10 ppb | 0/62 | 0/13 | 0/10 |
| Exp. #2 | | | |
| No selection | 96/102 | 21/23 | 22/25 |
| Kanamycin, 50 mg/L | 81/102 | 16/23 | 0/25 |
| Chlorsulfuron, 10 ppb | 0/102 | 0/23 | 0/25 |

[1] Agrobacterium strain containing Ti plasmid carrying tobacco ALS gene and NOS/NPTII gene (Kanamycin resistance)
[2] Agrobacterium strain containing Ti plasmid carrying only NOS/NPTII gene
[3] Agrobacterium strain containing Ti plasmid (devoid of either tobacco ALS or NOS/NPTII genes)

A genomic library of DNA from the Hra mutant of tobacco was made in bacteriophage lambda and screened for clones that hybridized to the wild type tobacco ALS gene from the S4 mutant. Several phage clones were isolated. Physical mapping of the tobacco DNA inserts using restriction endonucleases revealed the presence of two distinct classes of DNA fragments representative of the two tobacco ALS genes SURA and SURB. Comparison of the physical maps of the SURA and SURB genes of N. tabacum to maps from the progenitor species showed that the SURA gene came from N. sylvestris and the SURB gene came from N. tomentosiformis. The wild type ALS gene isolated previously from the S4 mutant was designated SURA. The genetic linkage of the high level herbicide resistance mutation in Hra to the S4 mutation indicated that the Hra mutation was in the same ALS gene as the S4 mutation, namely SURB. Therefore, it was expected that the SURB gene isolated from the Hra mutant would be a mutant gene, designated SURB-Hra, encoding a herbicide resistant ALS. One phage clone containing the SURB-Hra gene was chosen for further analysis. This phage clone, designated 3, has been deposited at the ATCC, Rockville, MD under accession

number ATCC 40237. The phage clone was digested with Spe I restriction endonuclease to give an 8.3 Kb DNA fragment which was inserted into the Xba I site of plasmid pMuc19, and the resulting recombinant plasmid, pAGS148, has been deposited at the ATCC, Rockville, MD under accession number ATCC 67124. Plasmids pAGS148 and pAGS135 were ligated to each other as described below, and the resulting recombinant plasmid pAGS152 (Figure 2) was introduced into Agrobacterium tumefaciens LBA 4404. The resultant Agrobacterium tumefaciens LBA 4404 (pAGS152) has been deposited at the ATCC, under accession number ATCC 67126.

A genomic library of DNA from the tobacco C3 mutant was made in bacteriophage lambda and screened for clones which hybridized to the previously isolated ALS genes from tobacco. Several phage clones were isolated and the tobacco DNA inserts where physically mapped with restriction endonucleases. Two different DNA fragment types, corresponding to the SURA-C3 gene and the SURB gene, were identified. Two phage clones designated 35 and 38, carrying the SURA-C3 gene were chosen for further analysis.

Phage clone 35 was digested with Spe I and Sal I restriction endonucleases to give the 6.3 kb DNA fragment shown in Figure 3. This DNA fragment has been inserted into the plasmid vector pUC119 digested with restriction endonucleases Xba I and Sal I, and the resulting recombinant plasmid, pALS35, has been deposited at the ATCC, Rockville, Maryland, under accession number 67424.

In addition to the four tobacco ALS genes, SURA and SURB encoding wild type herbicide sensitive ALS, and SURA-C3 and SURB-Hra encoding mutant herbicide resistant ALS, ALS genes have been isolated from Arabidopsis thaliana, Beta vulgaris (sugarbeet) and part of an ALS gene from Zea mays (corn). the latter ALS genes, from herbicide sensitive plants, were obtained from genomic DNA libraries made in bacteriophage lambda by screening for DNA hybridizing to a previously isolated ALS gene from yeast or tobacco. The wild type ALS gene from sugarbeet was isolated in a phage designated $\phi$21 and physically mapped with restriction endonucleases. The DNA fragment isolated in this phage and two DNA fragments which were subcloned into the plasmid vector pUC119 are shown in Figure 7. Plasmid pSBALS216 has been deposited at the ATCC, Rockville, Maryland under accession number 67425.

Figure 1 shows restriction endonuclease maps of DNA fragments containing ALS genes isolated from the Hra mutant of tobacco. Based on these maps, two classes of DNA fragments can be distinguished. An approximately 18 kilobase nucleic acid insert in phage clone 3 carries the SURB-Hra gene. The insert contains a preferred DNA fragment of the present invention which encodes a herbicide-resistant ALS from tobacco mutant Hra. This nucleic acid fragment consists of double-stranded DNA of 8.3 ± .5 kilobases and has a molecular weight of 5.5 ± .3 mega daltons, and has 5′ overhang sequences of CTAG at both ends. The 8.3 kilobase nucleic acid fragment between the two Spe I sites hybridized to the ALS gene probe used to screen the genomic library. Restriction endonuclease Spe I can be used to excise the fragment from the phage using well-known techniques.

Figure 2 shows a physical map of plasmid pAGS152. Plasmids pAGS135 and pAGS148 are not drawn to scale. Restriction endonuclease sites EcoR I (RI), BamH I (B), Xba I (X), Pst I (P), Sal I (S), Spe I (Sp), Nco I (N), Hind III (H), BstE II (Bs), Sma I (Sm), Kpn I (K), Sst I (St), Sph I (Sh) and Bgl II (G) are shown. pAGS152 results from the ligation of the BamH I- cleaved plasmids pAGS135 (approximately 27 kilobases) and pAGS148 (approximately 12.1 kilobases). Plasmid pAGS135, drawn as a circle, is a wide host range plasmid containing a plant kanamycin resistance gene (NOS:NPT II) and a BamH I cloning site, flanked by the left border (LB) and the right border (RB) of T-DNA. Plasmid pAGS148, shown as a linear BamH I fragment, consists of the Spe I (Sp) fragment (approximately 8.3 kilobases) of the aspect of the invention (shown flanked by X/Sp and Sp/X), containing the coding sequence for the herbicide-resistant form of ALS, from the Hra mutant, inserted in the Xba I site (X) of plasmid pMuc19 (open box). Although Spe I and Xba I restriction enzymes recognize different sequences, their action results in DNA fragments with the same 5′ overhanging sequence, viz 5′-CTAG-3′. Thus, Spe I and Xba I digestd fragments can be ligated to each other, but the ligation results in a loss of both sides. The hatched box on the insert fragment corresponds to the coding region of the ALS gene and the arrow denotes the 5′→3′ direction of the coding sequence. The nucleic acid fragment is flanked by Hind III, Sph I, Pst I and Sal I sites at one end and by BamH I, Sma I, Kpn I, Sst I and EcoR I sites at the other end. These enzymes can be used to excise the fragment from the plasmid by complete or partial digestion using well-known techniques. After digestion, the ends of the fragment will be characteristic of the endonuclease used to excise the fragment:

| 5' Overhanging Sequence | | 3' Overhanging Sequence | |
|---|---|---|---|
| Spe I | 5'-CTAGT-3'<br>3'-A-5' | Sph I | 5'-C-3'<br>3'-GTACG-5' |
| Hind III | 5'-AGCTT-3'<br>3'-A-5' | Pst I | 5'-G-3'<br>3'-ACGTC-5' |
| Sal I | 5'-TCGAC-3'<br>3'-G-5' | Kpn I | 5'-C-3'<br>3'-CATGG-5' |
| BamH I | 5'-GATCC-3'<br>3'-G-5' | Sst I | 5'-C-3'<br>3'-TCGAG-5' |
| EcoR I | 5'-AATTC-3'<br>3'-G-5' | | |
| | Blunt end | | |
| Sma I | 5'-GGG-3'<br>3'-CCC-5' | | |

The 8.3 kilobase fragment can be isolated from the restriction digest using agarose gel electrophoresis. The fragment can be characterized by the restriction map shown in Figure 2, and contains the coding sequence for ALS from mutant plant Hra of Nicotiana tabacum cv. 'Xanthi' which is resistant to inhibition by chlorsulfuron and sulfometuron methyl. The fragment also contains regulatory nucleotide sequences required to express the gene in plants.

Figure 3 shows a restriction endonuclease map of the approximately 6.8 kb preferred nucleic acid fragment which carries the SURA-C3 gene. This DNA fragment was obtained from lambda phage clone 35 by digestion with restriction endonucleases SpeI and SalI and was inserted into the plasmid vector pUC119 which had been digested with restriction endonucleases Xba I and Sal I, as described in the legend to Figure 2.

Figure 4 shows a partial nucleotide sequence of a preferred DNA fragment encoding a herbicide-resistant form of ALS from SURB-Hra gene of tobacco. Nucleotides are indicated by their bases by the following standard abbreviations:

A = adenine;
C = cytosine;
T = thymine;
G = guanine.

The begining of the nucleotide sequence corresponds to Pst I Site (P) 885 nucleotide bases preceding the coding sequence, shown on Figure 2; the sequence ends at base number 2946, which is 67 bases past the end of the coding sequence shown in Figure 2. The nucleotide sequence from nucleotide one to nucleotide 884 is believed to contain 5′ regulatory sequence(s) required for expression of the encoded ALS. Figure 4 also shows the deduced amino acid sequence of the ALS protein.

Amino acid residues are indicated by the following abbreviations:

A = alanine;
C = cysteine;
D = aspartic acid;
E = glutamic acid;
F = phenylalanine;
G = glycine;
H = histidine;

I = isoleucine;
K = lysine;
L = leucine;
M = methionine;
N = asparagine;
P = proline;
Q = glutamine;
R = arginine;
S = serine;
T = threonine;
V = valine;
W = tryptophan; and
Y = tyrosine.

The term "amino acids" as used herein is meant to denote the above-recited natural amino acids and functional equivalents thereof.

Figure 5 shows a partial nucleotide sequence and its cognate deduced amino acid sequence, of a preferred DNA fragment encoding a herbicide-resistant form of ALS from the C3 gene of tobacco. The beginning of the nucleotide sequence corresponds to the BamH I site shown in Figure 3. The coding sequence begins at nucleotide 176 and ends at nucleotide 2175. The nucleotide sequence from nucleotide one to nucleotide 175 is believed to contain 5′ regulatory sequence(s) necessary, but not sufficient, for expression of the encoded ALS. Nucleotides and amino acids are indicated by the standard abbreviations, as shown above.

Figure 6 shows the deduced amino acid sequences of the large subunits of ALS isozymes I, II and III from E. coli (Lines E, F and G respectively), wild type ALS proteins of yeast (Line D), Arabidopsis thaliana (Line C) and Nicotiana tabacum (tobacco) (Lines A and B), encoded by the SURB and SURA genes, respectively. Amino acid residues are indicated by standard abbreviations as shown above. The first amino acid, methionine, of the deduced amino acid sequences of the yeast (line D, Figure 6) and higher plant (lines A-C) ALS proteins is the putative start of the transit peptides believed to be involved in translocating the enzymes into mitochondria, in the case of the yeast enzyme, or chloroplasts, in the case of the plant enzymes. These transit peptides are believed to be cleaved off during translocation of the proteins into the organelles and are believed not to be required for ALS activity. The extend of these transit peptides is difficult to determine in the absence of data on the in vivo N-termini of the ALS proteins of yeast and higher plants. Based on the homology with the bacterial ALS proteins the chloroplast and mitochondrial transit sequences may be estimated to extend for 90 amino acids.

The dotted lines in the sequences are spacing marks inserted to best align regions of homology. Vertical lines highlight the amino acid residues that are conserved between adjacent sequences of Figure 6. The homology between tobacco and Arabidopsis ALS proteins (lines A to C), which derive from two different plant families, is striking. Even more unexpected, considering the evolutionary distance betwen microbes and higher plants, is the finding that the amino acid residues which are conserved between the bacterial (lines E to G) and the yeast (line D) ALS proteins are largely conserved between these proteins and the plant ALS proteins.

Figure 7 shows a restriction endonuclease map of the approximately 17.5 kilobase nucleic acid insert in phage clone ϕ21 carrying the sugarbeet ALS gene. Two smaller DNA fragments which also contain the sugarbeet ALS gene and which were subcloned into the pUC119 plasmid vector are also shown.

Figure 8 shows the deduced amino acid sequences of wild type ALS proteins from the plants Nicotiana tobacum (tobacco) (Lines A and B), Arabidopsis thaliana (Line C) Beta vulgaris cv. sennica (sugarbeet) (Line D) and a partial sequence of the ALS protein from maize (Line E). The dotted lines in the sequences are spacing marks to best align regions of homology. Vertical lines highlight the amino acid sequences that are conserved between adjacent sequences. The homology between all of the plant ALS proteins is very extensive. Based upon this, a mutation in one plant ALS gene causing an amino acid substitution that results in sulfonylurea herbicide resistant ALS would be expected to have an analogous effect if it were present in any other plant ALS gene.

The amino acid residues which are conserved in all of the ALS sequences in Figure 6 are believed to be important for the binding of substrates, herbicides, coenzymes, etc. These sequences are believed to be substantially conserved in all ALS proteins. The residues which are partially conserved in the different ALS proteins may participate in less conserved aspects of enzyme function, such as those which govern its herbicide sensitivity and its end-product inhibition. Examples of this would include the resistance of bacterial isozyme I to sulfometuron methyl and chlorsulfuron, and of bacterial isozyme II to end-product inhibition by valine. Finally, those residues which are not conserved between the proteins probably reside in the framework of the ALS protein where sequence divergence is less disruptive to enzyme function.

Although not wishing to be bound by theory, binding of sulfonylurea herbicides to ALS during acetolactate synthesis

is believed to be facilitated by the binding of a first pyruvate molecule to the enzyme. However, the binding of a sulfonylurea herbicide molecule is competitive with the binding of a second pyruvate molecule to the enzyme. Sulfonylurea herbicide sensitivity is conserved through evolution in most ALS enzymes. From these facts, it was deduced that the binding of the sulfonylurea herbicide occurs at or proximal to one or more of the conserved amino acids in the ALS proteins. In fact, Applicant has discovered that substitutions for one or more of 10 specific amino acid residues in one or more of the 7 substantially conserved sub-sequences A through G will confer herbicide residence and are claimed. It is expected that substitution at other amino acid residues in the substantially conserved sub-sequences will also confer herbicide resistance.

Sulfonylurea herbicide resistance in bacteria, yeast and higher plants, which resistance cosegregates with herbicide-resistant forms of ALS, results from mutations in the structural genes for ALS. Comparing the nucleotide sequences of ALS genes of organisms encoding herbicide sensitive and herbicide-resistant forms of ALS allows one to determine which amino acid residues are important for herbicide inhibition of the enzyme. One mutation in the E. coli ilvG gene, which results in an enzyme with increased resistance to sulfometuron methyl inhibition, and with reduced catalytic activity, was determined to result in an alanine-to-valine substitution at position 122 (Figure 6). Another sulfometuron methyl resistance mutation in this gene was determined to result in a alanine-to-serine substitution at the same position. This alanine residue is conserved in all ALS enzymes except bacterial isozyme I (Figure 6), which is naturally resistant.

Many genes encoding herbicide-resistant ALS enzymes have been isolated from spontaneous sulfonylurea-resistant yeast mutants. Sequencing of these genes has shown the molecular basis of resistance to be base changes which result in amino acid substitutions at ten different positions in the protein (Table 2), residues 121, 122, 197, 205, 256, 359, 384, 588, 591 and 595 (numbering relative to the positions in Figure 6).

Table 2

| Spontaneous Mutations of the Yeast ALS Gene Resulting in Sulfonylurea Herbicide Resistance | | | | |
|---|---|---|---|---|
| Amino Acid Positions | Wild Type Codon | Wild Type Amino Acid | Mutant Codon | Amino Acid Substitution |
| 121 | GGT | Gly | AGT | Ser |
| 122 | GCT | Ala | CCT | Pro |
| | | | GAT | Asp |
| | | | GTT | Val |
| | | | ACT | Thr |
| 197 | CCA | Pro | TCA | Ser |
| | | | CGA | Arg |
| 205 | GCT | Ala | GAT | Asp |
| | | | ACT | Thr |
| 256 | AAG | Lys | GAG | Glu |
| | | | ACG | Thr |
| | | | AAC | Asn |
| 359 | ATG | Met | GTG | Val |
| 384 | GAC | Asp | GAA | Glu |
| | | | GTC | Val |
| | | | AAC | Asn |
| 588 | GTT | Val | GCT | Ala |
| 591 | TGG | Trp | CGG | Arg |
| | | | AGG | Arg |
| | | | TGT | Cys |
| | | | TGC | Cys |

Table 2 (continued)

| Spontaneous Mutations of the Yeast ALS Gene Resulting in Sulfonylurea Herbicide Resistance | | | | |
|---|---|---|---|---|
| Amino Acid Positions | Wild Type Codon | Wild Type Amino Acid | Mutant Codon | Amino Acid Substitution |
| | | | GGG | Gly |
| | | | TTG | Leu |
| | | | TCG | Ser |
| | | | GCG | Ala |
| 595 | TTC | Phe | TTA | Leu |

At six of these positions, 122, 197, 205. 256, 384 and 591 (Table 2), more than one substitution that confers herbicide resistance has been obtained. At position 122, at which an alanine residue is present in all known wild type ALS enzymes except E. coli isozyme I, substitutions of aspartic acid, proline, threonine or valine result in sulfonylurea-resistant ALS. At position 197, at which a proline residue is present in all known wild-type ALS enzymes except E. coli isozymes II and III, substitutions of serine or arginine result in sulfonylurea-resistant ALS. At position 205, at which an alanine residue is present in all known wild type ALS enzymes, substitutions of aspartic acid of threonine result in sulfonylurea-resistant ALS. At position 256, at which a lysine residue is present in all known wild type ALS enzymes, substitutions of glutamic acid, asparagine or threonine result in sulfonylurea-resistant ALS. At position 384 at which an aspartic acid is present in all known wild type ALS enzymes, substitutions of glutamic acid, asparagine or valine result in sulfonylurea-resistant ALS. At position 591, at which a tryptophan is present in all known wild type ALS enzymes except E. coli isozyme I, substitutions of alanine, cysteine, glycine, leucine, arginine or serine result in sulfonylurea-resistant ALS.

Mutants resistant to sulfonylurea herbicides resulting from single amino acid substitutions at the other four positions, 121, 359, 588, 595, have been obtained. At position 121, at which glycine is present in all known ALS enzymes, substitution of serine results in a sulfonylurea-resistant ALS. At position 359, at which methionine is present in all known ALS enzymes, substitition of valine results in a sulfonylurea-resistant ALS. At position 588, at which valine is present in all known ALS enzymes, substitution of alanine results in sulfonylurea-resistant ALS. At position 595, at which phenylalanine is present in all known ALS enzymes except E. coli isozyme III, substitution of leucine results in sulfonylurea-resistant ALS.

Oligonucleotide-directed site specific mutations, which result in amino acid substitutions at positions 122, 205, 256, 359, 384 and 591, have been made in the yeast gene encoding ALS (Table 3).

Table 3

Site-Directed Mutations of the Yeast ALS Gene Resulting in Sulfonylurea Herbicide Resistance

| Amino Acid Positions | Wild Type Codon | Wild Type Amino Acid | Mutant Codon | Amino Acid Substitution |
|---|---|---|---|---|
| 122 | GCT | Ala | TCT | Ser |
| | | | GTT | Val |
| | | | ACT | Thr |
| | | | CCT | Pro |
| | | | AAT | Asn |
| | | | ATT | Ile |
| | | | CAT | His |
| | | | CGT | Arg |
| | | | CTT | Leu |
| | | | TAT | Tyr |
| | | | TGT | Cys |
| | | | TTT | Phe |
| | | | GAA | Glu |
| | | | ATG | Met |
| | | | AAA | Lys |
| | | | CAA | Gln |
| | | | TGG | Trp |
| 205 | GCT | Ala | CGT | Arg |
| | | | TGT | Cys |
| | | | GAA | Glu |
| | | | TGG | Trp |
| 256 | AAG | Lys | GAC | Asp |
| | | | GGG | Gly |
| | | | CTG | Leu |
| | | | CCG | Pro |
| | | | TGG | Trp |

Table 3 (continued)

| Amino Acid Positions | Wild Type Codon | Wild Type Amino Acid | Mutant Codon | Amino Acid Substitution |
|---|---|---|---|---|
| 359 | ATG | Met | CCA | Pro |
| | | | CAG | Glu |
| 384 | GAC | Asp | CCA | Pro |
| | | | TGG | Trp |
| | | | TCC | Ser |
| | | | GGT | Gly |
| | | | TGC | Cys |
| | | | AAA | Lys |
| 591 | TGG | Trp | GAC | Asp |
| | | | GAG | Glu |
| | | | TTC | Phe |
| | | | CAC | His |
| | | | TAC | Tyr |
| | | | ATA | Ile |
| | | | GTG | Val |
| | | | AAG | Lys |
| | | | AGG | Arg |
| | | | ATG | Met |
| | | | AAC | Asn |
| | | | CAG | Gln |
| | | | ACG | Thr |

At position 122, mutations resulting in eighteen amino acid substitutions for alanine, which is present in wild type ALS, have been made. The nineteenth substitution (aspartic acid) was isolated previously as a spontaneous mutation and was therefore not remade. Each substitution, except for glycine, results in sulfonylurea-resistant ALS. At position 205, mutations resulting in substitutions for alanine, the wild type residue, of cysteine, glutamic acid, arginine or tryptophan, result in sulfonylurea-resistant ALS. At position 256, mutations resulting in substitutions for lysine, the wild type residue, of aspartic acid, glycine, leucine, proline or tryptophan, result in sulfonylurea-resistant ALS. At position 359, mutations resulting in substitutions for methionine, the wild type residue, of glutamic acid or proline result in sulfonylurea-resistant ALS. At position 384, mutations resulting in amino acid substitutions for aspartic acid, the wild type residue, of cysteine, glycine, proline, lysine, serine or tryptophan result in sulfonylurea-resistant ALS. At position 591, mutations resulting in amino acid substitutions for tryptophan, the wild type residue, of aspartic acid, glutamic acid, phenylalanine, histidine, isoleucine, lysine, arginine, valine, methionine, asparagine, glutamine, threonine or tyrosine result in sulfonylurea-resistant ALS.

All mutations described in Tables 2 and 3 resulted in enzymes which were active and less inhibited by sulfonylurea herbicides than the wild type. Taken in total these results indicate that most substitutions at these 10 positions result in enzymatically active herbicide resistant ALS.

The deduced amino acid sequences of the wild type ALS proteins from tobacco, Arabidopsis, sugarbeet and corn are shown in Figure 7. The amino acid residues at positions 121, 122, 197, 205, 256, 359, 384, 588, 591 and 595 (numbering of positions from Figure 6) in all the plant enzymes are the same as those present in the wild type herbicide sensitive yeast protein (Figure 6). The deduced amino acid sequence of the tobacco ALS gene SURB-Hra, which encodes a herbicide-resistant ALS, is shown in Figure 4. The mutant gene of Figure 4 was derived from a tissue culture line which had undergone two successive spontaneous mutations. The two mutations have been shown to be genetically linked, and introduction of this fragment into sensitive tobacco cells confers upon the cells the same level of herbicide resistance as is found for the original highly resistant mutant tobacco plant from which the fragment was derived. Based on these facts, it was expected that there would be two amino acid substitutions in the enzyme encoded by the fragment. A comparison of the deduced amino acid sequence of the mutant ALS with the deduced amino acid sequence of the wild type ALS reveals that the mutant ALS has a proline-to-alanine substitution at position 197 (Figure

6) and a tryptophan-to-leucine substitution at position 591 (Figure 6). Based on the foregoing, it was determine that substitutions at proline 197 and tryptophan 591 residues confer herbicide resistance. The deduced amino acid sequence of a second mutant tobacco ALS gene, SURA-C3, which encodes a sulfonylurea herbicide resistant ALS, is shown in Figure 5. A comparison of the deduced amino acid sequences of the mutant and wild type ALS enzymes (Figure 5 and Figure 6, line B) reveals that the mutant ALS has a single substitution, proline-to-glutamine, at position 197. The C3 cell line from which the SURA-C3 gene was obtained showed selective herbicide resistance. That is, the C3 mutation conferred resistance to the sulfonylurea herbicides chlorsulfuron and sulfomenturon methyl, but not to an imidazolinone herbicide.

The identification of amino acid substitutions in herbicide-resistant ALS enzymes from plants at positions 197, from the C3 and Hra mutants, and 591 from the Hra mutant, indicates that substitutions at positions operable in yeast ALS are also operable in plant ALS.

While the amino acid residues present at positions, 121, 122, 197, 205, 256, 359, 384, 588, 591 and 595 are conserved in all wild type herbicide sensitive ALS enzymes so far characterized from eucaryotes, some substitutions at these positions are found in wild type bacterial ALS enzymes. E. coli isozyme I has a serine rather than alanine at position 122 and a glutamine rather than tryptophan at position 591, E. coli isozyme II has a serine rather than proline at position 197 and E. coli isozyme III has an alanine rather than proline at position 197 and an isoleucine rather than phenylalanine at position 595. Each of these E. coli ALS isozymes is more resistant (from 50-fold to greater than 10,000-fold) to inhibition by (particular) sulfonylurea herbicides than plant or yeast ALS. Furthermore, a site-directed mutation causing a serine-to-proline substitution at position 197 in E. coli ALS II rendered the mutant enzyme 100 fold more sensitive to inhibition, i.e., as senstive as wild type higher plant enzymes. Thus, proline at position 197 is involved in herbicide binding in E. coli ALS II as well as in yeast and higher plant ALS.

In addition, site-directed mutations which result in tryptophan-to-leucine and glutamine-to-tryptophan substitutions at position 591 in ALS II and ALS I, respectively, of E. coli have been made. The mutation in ALS II makes the enzyme more herbicide resistant than the wild type ALS II, while the mutation in ALS I makes it more sensitive than wild type ALS I.

The site-directed mutations at positions 197 and 591 in ALS I and ALS II of E. coli affect inhibition by herbicide of the mutant enzymes in a manner predicted from the herbicide-resistant mutant yeast and plant ALS proteins. These experimental findings support the universality of the amino acid residues involved in herbicide binding to ALS enzymes from diverse sources.

## Characterization of Nucleic Acid Fragments Encoding Herbicide-Resistant ALS

According to the present invention, the amino acid residues of ALS that correspond to $\varepsilon_1$ and $\varepsilon_2$ in amino acid sub-sequence A, $\alpha_1$ in amino acid sub-sequence B, $\delta_2$ in amino acid sub-sequence C, $\lambda_1$ in amino acid sub-sequence D, $\gamma_1$ in amino acid sub-sequence E, $\beta_3$, $\beta_7$ and $\beta_8$ in amino acid sub-sequence F and $\sigma_1$ in amino acid sub-sequence G of Figure 6 (referred to hereinafter as positions 122, 121, 197, 205, 256, 384, 591, 595, 588 and 359 respectively) are important in herbicide sensitivity or resistance of ALS enzymes regardless of the biological source of these enzymes, and any nucleotide sequence encoding a plant ALS can be altered to direct synthesis of a herbicide-resistant ALS by virtue of amino acid substitutions at these residues. The nucleic acid fragment of the present invention is characterized in that at least one of the following conditions is met:

a) The nucleic acid fragment encodes an amino acid other than glycine at position 121. Preferably the amino acid is serine. threonine, or cysteine. Most preferably the amino acid is serine.
b) The nucleic acid fragment encodes an amino acid other than alanine at position 122. Most preferably, the amino acid is any other than glycine.
c) The nucleic acid fragment encodes an amino acid other than proline at position 197. Preferably, the amino acid is alanine, glycine, arginine, lysine, histidine, serine, threonine, cysteine, glutamine, or asparagine. Most preferably, the amino acid is alanine, arginine, serine or glutamine.
d) The nucleic acid fragment encodes an amino acid other than alanine at position 205. Preferably, the amino acid is threonine, serine, cysteine, tyrosine, aspartic acid, glutamic acid, tryptophan, histidine, phenylalanine, arginine or lysine. Most preferably, the amino acid is threonine, cysteine, aspartic acid, glutamic acid, tryptophan or arginine.
e) The nucleic acid fragment encodes an amino acid other than lysine at position 256. Preferably, the amino acid is glycine, alanine, leucine, isoleucine, valine, threonine, serine, cysteine, tryosine, glutamic acid, aspartic acid, proline, asparagine, glutamine, tryptophan or phenylalanine. Most preferably, the amino acid is glycine, leucine, threonine, glutamic acid, aspartic acid, proline, asparagine or tryptophan.
f) The nucleic acid fragment encodes an amino acid other than methionine at position 359. Preferably, the amino acid is glutamic acid, aspartic acid, proline, valine, leucine or isoleucine. Most preferably the amino acid is glutamic acid, proline or valine.

g) The nucleic acid fragment encodes an amino acid other than aspartic acid at position 384. Preferably, the amino acid is glycine, alanine, valine, leucine, isoleucine, serine, threonine, cysteine, tyrosine, glutamic acid, proline, asparagine, glutamine, lysine, arginine, tryptophan, histidine or phenylalanine. Most preferably, the amino acid is glycine, valine, serine, cysteine, glutamic acid, proline, asparagine, lysine or tryptophan.
h) The nucleic acid fragment encodes an amino acid other than valine at position 588. Preferably the amino acid is alanine or glycine. Most preferably the amino acid is alanine.
i) The nucleic acid fragment encodes an amino acid other than tryptophan at position 591. Most preferably, the amino acid is other than proline.
j) The nucleic acid fragment encodes an amino acid other than phenylalanine at position 595. Preferably, the amino acid is leucine, isoleucine and valine. Most preferably the amino acid is leucine.

In one embodiment, position 121 resides within amino acid sub-sequence A as follows:

$$PG\varepsilon_2A$$

wherein P, G and A are as defined above. To confer herbicide resistance $\varepsilon_2$ is an amino acid other than glycine. Most preferably $\varepsilon_2$ is the amino acid serine. This sub-sequence begins about 24 residues from the begining of a substantially conserved amino acid sequence

HEQ,

i.e.,

$$PG\varepsilon_2A...HEQ.$$

In one embodiment, position 122 resides within amino acid sub-sequence A as follows:

$$PGG\varepsilon_1$$

wherein P and G are as defined above. To confer herbicide resistance $\varepsilon_1$ is a natural amino acid other than alanine. Most preferably $\varepsilon_1$ is any amino acid except glycine. This sub-sequence begins about 24 residues from the begining of a substantially conserved amino acid sequence

HEQ,

i.e.,

$$PGG\varepsilon_1...HEQ.$$

In one embodiment, position 197 resides within amino acid sub-sequence B as follows:

$$GQV\alpha_1$$

wherein G, Q, and V are as defined above, to confer herbicide resistance $\alpha_1$ is an amino acid other than proline. Most preferably, $\alpha_1$ is alanine, arginine, serine or glutamine. This sub-sequence, begins about 20 residues from the end of one substantially conserved amino acid sequence

SGPGATN

and about 55 residues from the beginning of a second substantially conserved amino acid sequence

SGRPGP,

i.e.,

SGPGATN...GQV$\alpha_1$....SGRPGP.

In one embodiment, position 205 resides within an amino acid sub-sequence C as follows:

IG$\delta_1$D$\delta_2$FQE

wherein I, G, D, F, Q, and E are as defined above, $\delta_1$ represents an amino acid residue which can vary according to the source of the enzyme, but is most commonly T. To confer herbicide resistance $\delta_2$ is an amino acid other than alanine. Most preferably, $\delta_2$ is threonine, cysteine, aspartic acid, glutamic acid, arginine, or tryptophan. This sub-sequence begins about 5 residues from the end of a substantially conserved amino acid sequence

GQV

and about 43 residues from the begining of a second substantially conserved amino acid sequence

SGRPGP,

i.e.,

GQV...IG$\delta_1$D$\delta_2$FQE...SGRPGP.

In one embodiment, position 256 resides within an amino acid sub-sequence D as follows:

P$\lambda_1$D

wherein P and D are as defined above. To confer herbicide resistance $\lambda_1$ is an amino acid other than lysine. Preferably, $\lambda_1$, is glycine, leucine, threonine, glutamic acid, aspartic acid, asparagine, tryptophan or proline. This sub-sequence D begins about 6 residues from the end of a substantially conserved amino acid sequence

SGRPGP

i.e.,

SGRPGP...p$\lambda_1$D.

In one embodiment, position 359 resides within an amino acid sub-sequence G as follows

MLG$\sigma_1$HG

wherein M, L, G and H are defined as above. To confer herbicide resistance, $\sigma_1$ is an amino acid other than methionine. Most preferably, $\sigma_1$ is proline, glutamic acid or valine. This sub-sequence ends about 20 residues from the beginning of a substantially conserved amino acid sequence

RFDDR

i.e.,

MLG$\sigma_1$HG...RFDDR.

In one embodiment, position 384 resides within an amino acid sub-sequence E as follows

RFD$\gamma_1$R

wherein R, F, and D are as defined above. To confer herbicide resistance, $\gamma_1$ is an amino acid other than aspartic acid. Most preferably, $\gamma_1$ is glycine, valine, cysteine, serine, lysine, glutamic acid, proline, asparagine or tryptophan.

This sub-sequence begins about 20 residues from the end of a substantially conserved amino acid sequence

MLGMHG.

i.e.,

MLGMHG...RFD$\gamma_1$R.

In one embodiment, position 588 resides within an amino acid sub-sequence F as follows

G$\beta_1\beta_8\beta_2$Q$\beta_3\beta_4\beta_5\beta_6\beta_7$

wherein G and Q are defined above, $\beta_1$ to $\beta_8$ will vary depending upon the source of the enzyme. $\beta_1$ is usually methionine, $\beta_3$ is usually tryptophan and $\beta_7$ is usually phenylalanine. To confer herbicide resistance $\beta_8$ is an amino acid other than valine. Most preferably $\beta_8$ is alanine. This sub-sequence begins about 49 residues from the end of a substantially conserved amino acid sequence

GLPAA

i.e.,

GLPAA...G$\beta_1\beta_8\beta_2$Q$\beta_3\beta_4\beta_5\beta_6\beta_7$.

In one embodiment, position 591 resides within an amino acid sub-sequence F as follows

G$\beta_1$V$\beta_2$Q$\beta_3\beta_4\beta_5\beta_6\beta_7$

wherein G, V and Q are defined above, $\beta_1$ to $\beta_7$ will vary depending depending upon the source of the enzyme. $\beta_1$ is usually methionine and $\beta_7$ is usually phenylalanine. To confer herbicide resistance $\beta_3$ is any amino acid other than tryptophan. Most preferably $\beta_3$ is any amino acid other than proline. This sub-sequence begins about 49 residues from the end of another substantially conserved amino acid sequence

GLPAA.

i.e..

$$GLPAA.....G\beta_1V\beta_2q\beta_3\beta_4\beta_5\beta_6\beta_7.$$

In one embodiment, position 595 resides within an amino acid sub-sequence F as follows

$$G\beta_1V\beta_2Q\beta_3\beta_4\beta_5\beta_6\beta_7$$

wherein G. V and Q are defined above. $\beta_1$ to $\beta_7$ will vary depending upon the source of the enzyme. $\beta_1$ is usually methionine and $\beta_3$ is usually tryptophan. To confer herbicide resistance, $\beta_7$ is an amino acid other than phenylalanine. Most preferably, $\beta_7$ is leucine. This sub-sequence begins about 49 amino acids from the end of a substantially conserved amino acid sequence

GLPAA

i.e.,

$$GLPAA...G\beta_1V\beta_2Q\beta_3\beta_4\beta_5\beta_6\beta_7.$$

Herbicide resistance can be achieved by any one of the above described amino acid substitutions and by combinations thereof.

The precise amino acid substitutions required for herbicide resistance can be achieved by mutating a nucleic acid fragment encoding a herbicide sensitive ALS from any plant of interest generally as follows:

(1) isolate genomic DNA or mRNA from the plant;
(2) prepare a genomic library from the isolated DNA or a cDNA library from the isolated RNA;
(3) identify those phages or plasmids which contain a DNA fragment encoding ALS;
(4) sequence the fragment encoding the ALS;
(5) sub-clone the DNA fragment carrying the ALS gene into a cloning vehicle which is capable of producing single-stranded DNA;
(6) synthesize an oligonucleotide of about 15 to 20 nucleotides which is complementary to a particular ALS nucleotide sequence encoding one of the amino acid sub-sequences recited above except for the nucleotide change (s) required to direct a mutation to a codon for an amino acid selected for its ability to confer herbicide resistance;
(7) anneal the oligonucleotide to the single-stranded DNA containing the region to be mutated and use it to prime synthesis in vitro of a complementary DNA strand forming a heteroduplex;
(8) transform bacterial cells with the heteroduplex DNA;
(9) screen the transformed bacterial cells for those cells which contain the mutated DNA fragment by a) immobilizing the DNA on a nitrocellulose filter, b) hybridizing it to the 5'-$^{32}$P labelled mutagenic oligonucleotide at ambient temperature, and c) washing it under conditions of increasing temperature so as to selectively dissociate the probe from the wild-type gene but not the mutant gene;
(10) isolate a double-stranded DNA fragment containing the mutation from the cells carrying the mutant gene; and
(11) confirm the presence of the mutation by DNA sequence analysis.

An amino acid substitution required for herbicide resistance can also be achieved by substituting a nucleotide sequence of a plant ALS gene which encodes a sequence of amino acids containing the amino acid to be substituted with another nucleotide sequence, which encodes the corresponding stretch of amino acids containing the desired substitution, derived from any natural ALS gene (including microbial) or from a synthetic source.

Preparation of Herbicide-Resistant Plants

The nucleic acid fragments of the present invention can be used to introduce herbicide resistance into plants. In order to introduce a nucleic acid fragment which includes a gene encoding herbicide resistant ALS into different plants, a wide variety of techniques are used depending on the species or cultivar desired. In general, explants or protoplasts can be taken or produced from either in vitro or soil grown plants. Explants or protoplasts may be produced from cotyledons, stems, petioles, leaves, roots, immature embryos, hypocotyls, inflorescences, etc. In theory, any tissue which can be manipulated in vitro to give rise to new callus or organized tissue growth can be used for genetic transformation.

To achieve transformation, explants or protoplasts may be cocultured with Agrobacterium, which can be induced to transfer nucleic acid fragments located between the T-DNA borders of the Ti plasmid to the plant cells. Another method, less commonly used, is direct DNA uptake by plant protoplasts. With this method, the use of Agrobacterium is bypassed and DNA is taken up directly by the protoplasts under the appropriate conditions.

In the examples, a variety of explants from different plants have been cocultured with Agrobacterium to achieve transformation to herbicide resistance. These explants were cultured to permit callus growth. The callus was then tested directly for resistance to sulfonylureas, or plants were regenerated and the plants were tested for sulfonylurea resistance. Testing consisted of an enzyme assay of plant cell extracts for the presence of ALS activity resistant to herbicide and/or growth of plant cells in culture or of whole plants in the presence of normally inhibitory concentrations of herbicide.

The DNA fragments are comprised of a region coding for the synthesis of herbicide-resistant ALS and a region providing for expression of the coding sequence in plants. The 8.3 kb DNA fragment shown in Figure 2 which codes for the herbicide-resistant ALS protein shown in Figure 4 contains about 800 bp in the 5′ direction (upstream) of the coding region, sufficient for expression of the protein in plants. This DNA fragment can confer resistance to chlorsulfuron up to 2000 ppb in transformed tobacco calluses. Plants regenerated from the transformed cells also show resistance at the whole plant level. The 6.3 kb DNA fragment shown in Figure 3 which codes for the herbicide resistant ALS protein shown in Figure 5 contains 2.5 kb in the 5′ direction (upstream) and 1.8 kb in the 3′ direction (downstream) of the coding region sufficient for expression of the protein in plants. This DNA fragment can confer resistance to chlorsulfuron at 2ppb in transformed tobacco calluses.

In work which is on-going, DNA fragments containing site-directed mutations in the SURA gene that are expected to code for herbicide resistant ALS have been made. These mutations result in the following amino acid substitutions: Ala 122 to Ser, known to be operable in E. coli ALS isozyme II and yeast ALS, Ala 122 to Val, known to be operable in E. coli ALS isozyme II and yeast ALS, Ala 122 to Pro, known to be operable in yeast ALS, Pro 197 to Ser, known to be operable in yeast ALS, and E. coli ALS II enzyme, Pro 197 to Ala, known to be operable in ALS encoded by the SURB-Hra gene of tobacco, Lys 256 to Glu, known to be operable in yeast ALS, Asp 384 to Val, known to be operable in yeast ALS and Trp 591 to Leu, known to be operable in yeast ALS and ALS encoded by the SURB-Hra gene of tobacco. By combining the above mutations, double mutations, resulting in two amino acid substitutions such as Ala 122 to Ser and Pro 197 to Ser, or Ala 122 to Ser and Pro 197 to Ala, or Pro 197 to Ala and Trp 591 to Leu, or Pro 197 to Ser and Trp 591 to Leu have also been made. These mutations were made in a DNA fragment that included only about 180 bp in the 5′ direction (upstream) and only about 600 bp in the 3′ direction (downstream) of the ALS coding sequence. These DNA fragments were introduced into tobacco by transformation. Herbicide resistance was not expressed in these transformants. It is believed that utilization of the regulatory sequences found sufficient for expression of herbicide resistance with the SURA-C3 mutant coding region, namely 2.5 kb in the 5′ direction (upsteam) and 1.8 kb in the 3′ direction (downstream) of the coding region, would have resulted in expression of herbicide resistance with all of the site-directed mutations in the SURA coding region.

Site directed mutations that are expected to code for herbicide resistant ALS have also been made in the sugarbeet ALS gene. These mutations result in the following amino acid substitutions: Ala 122 to Pro, known to be operable in yeast ALS, Pro 197 to Ala, known to be operable in ALS encoded by the SURB-Hra gene of tobacco, Trp 591 to Leu, known to be operable in yeast ALS and in ALS encoded by the SURB-Hra gene of tobacco and the double mutant, Pro 197 to Ala and Trp 591 to Leu, known to be operable in ALS encoded by the SURB-Hra gene of tobacco.

The nucleic acid fragments of the invention generally can be introduced into plants directly or in a nucleic acid construct comprising the desired nucleic acid fragment. The nucleic acid construct can be derived from a bacterial plasmid or phage, from the Ti- or Ri-plasmids, from a plant virus or from an autonomously replicating sequence. One preferred means of introducing the nucleic acid fragment into plant cells comprises use of Agrobacterium tumefaciens containing the nucleic acid fragment between T-DNA borders either on a disarmed Ti-plasmid (that is, a Ti-plasmid from which the genes for tumorigenicity have been deleted) or in a binary vector in trans to a Ti-plasmid with Vir functions. The Agrobacterium can be used to transform plants by inoculation of tissue explants, such as stems or leaf discs, or by co-cultivation with plant protoplasts. Another preferred means of introducing the present nucleic acid fragment comprises direct introduction of the fragment or a vector containing the fragment into plant protoplasts or cells, with or without the aid of electroporation, polyethylene glycol or other agents or processes known to alter membrane permeability to macromolecules.

The nucleic acid fragments of the invention can be used to transform protoplasts or cell cultures from a wide range of higher plant species to form plant tissue cultures of the present invention. These species include the dicotyledonous plants tobacco, petunia, cotton, sugarbeet, potato, tomato, lettuce, melon, sunflower, soybean, canola (rapeseed) and other Brassica species and poplars; and the monocotyledonous plants corn, wheat, rice, Lolium multiflorum and Asparagus officinalis. It is expected that all protoplast-derived plant cell lines can be stably transformed with the fragments of the invention.

The nucleic acid fragments of the invention can also be introduced into plant cells with subsequent formation of transformed plants of the present invention. Transformation plants of the present invention. Transformation of whole plants is accomplished in plants whose cells can be transformed by foreign genes at a stage from which whole plants can be regenerated. In the present invention, transformed plants are monocotyledonous and dicotyledonous plants. Preferably, the transformed plants are selected from the group consisting of tobacco, petunia, cotton, sugarbeets, potato, tomato, lettuce, sunflower, soybean, canola and other Brassica species, poplars, alfalfa, clover, sugarcane, barley, oats and millets; see "Handbook of Plant Cell Culture" Vols. 1-3, Evans, D. A. et al., Sharp et al., and Ammirato et al., respectively, MacMillan, N.Y. (1983, 84). Most preferably, the transformed plants are selected from the group consisting of tobacco, petunia, potato, tomato, sunflower, sugarbeet, alfalfa, lettuce or Brassica species. The range of crop species in which foreign genes can be introduced is expected to increase rapidly as tissue culture and transformation methods improve and as selectable markers such as the fragments of the invention (see discussion below) become available.

One could further increase the level of expression of the nucleic acid fragments of the invention by replacing their native regulatory nucleotide sequences, 5′ and 3′ to the ALS coding sequence, with synthetic or natural sequences known to provide high level and/or tissue specific expression. One may also substitute the nucleotide sequences of the nucleic acid fragments of the invention with other synthetic or natural sequences which encode transit peptides which will allow efficient chloroplast uptake of the nucleic acid fragments of the invention.

The nucleic acid fragments of the present invention also have utility as selectable markers for both plant genetic studies and plant cell transformations. A gene of interest, generally conferring some agronomically useful trait, e.g. disease resistance, can be introduced into a population of sensitive plant cells physically linked to a nucleic acid fragment of the present invention. Cells can then be grown in a medium containing a herbicide to which the ALS encoded by a fragment of the invention is resistant. The surviving (transformed) cells are presumed to have acquired not only the herbicide resistance phenotype, but also the phenotype conferred by the gene of interest. The nucleic acid fragments can be introduced by cloning vehicles, such as phages and plasmids, plant viruses, and be direct nucleic acid introduction. Subsequently, in a plant breeding program, the agronomically useful trait can be introduced into various cultivars through standard genetic crosses, by following the easily assayed herbicide resistance phenotype associated with the linked selectable genetic marker.

Transformed plants of the present invention are resistant to many of the sulfonylurea, triazolopyrimidine sulfonamide and imidazolinone herbicides. These herbicides are disclosed in the following patents and published patent applications as follows:

Sulfonylureas

| | |
|---|---|
| U.S. 4,127,405 | U.S. 4,383,113 |
| U.S. 4,169,719 | U.S. 4,394,153 |
| U.S. 4,190,432 | U.S. 4,394,506 |
| U.S. 4,214,890 | U.S. 4,420,325 |
| U.S. 4,225,337 | U.S. 4,452,628 |
| U.S. 4,231,784 | U.S. 4,481,029 |
| U.S. 4,257.802 | U.S. 4,586,950 |
| U.S. 4,310,346 | U.S. 4.435,206 |
| U.S. 4,544,401 | U.S. 4,514,212 |
| U.S. 4,435,206 | U.S. 4,634,465 |
| | EP-A-204,513 |

Triazolopyrimidine sulfonamides

South African Application 84/8844 (published 5/14/85)

Imidazolinones

U.S. 4,188,487
EP-A-41.623 (published 12/16/81)

The nucleic acid fragments of the present invention encode ALS which is resistant to the following sulfonylurea

herbicides:

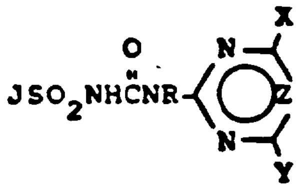

I

wherein

R is H or $CH_3$;
J is

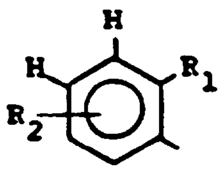 , 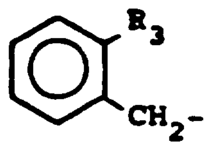 , 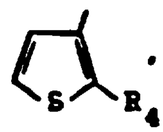 ,

J-1 J-2 J-3

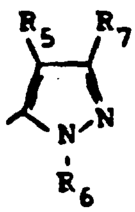 , 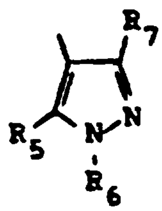 , 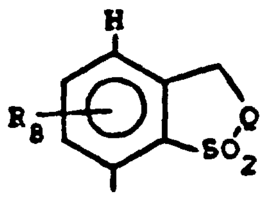

J-4 J-5 J-6

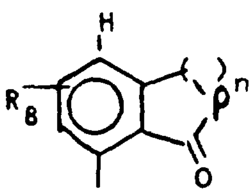 ; 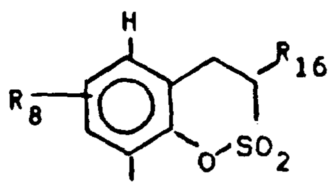 or 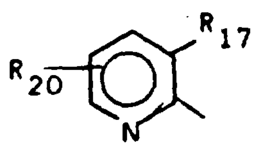 ;

J-7 J-8 J-9

$R_1$ is Cl, Br, $NO_2$, $C_1$-$C_4$ alkyl, $C_2$-$C_4$ alkenyl, $CF_3$, $C_1$-$C_4$ alkoxy, $C_1$-$C_4$ haloalkoxy, $C_3$-$C_4$ alkenyloxy, $C_2$-$C_4$ haloalkenyloxy, $C_3$-$C_4$ alkynyloxy, $CO_2R_9$, $CONR_{10}R_{11}$, $S(O)_mR_{12}$, $OSO_2R_{12}$, phenyl, $SO_2N(OCH_3)CH_3$, $SO_2NR_{10}R_{11}$,

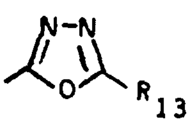 , 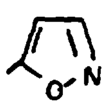 , 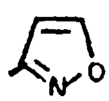 ,

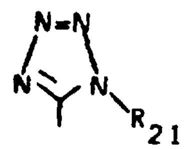 , 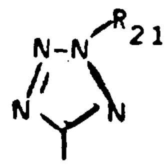 , 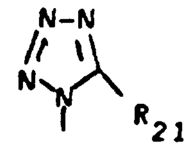 or 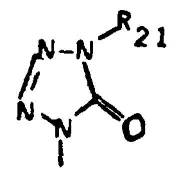

$R_2$ is H, Cl, Br, F, $CH_3$, $NO_2$, $SCH_3$, $OCF_2H$, $OCH_2CF_3$ or $OCH_3$;
$R_3$ is Cl, $NO_2$, $CO_2CH_3$, $CO_2C_2H_5$, $SO_2N(CH_3)_2$, $SO_2CH_3$ or $SO_2C_2H_5$;
$R_4$ is $C_1$-$C_3$ alkyl, Cl, Br, $NO_2$, $CO_2R_9$, $CON(CH_3)_2$, $SO_2N(CH_3)_2$, $SO_2N(OCH_3)CH_3$ or $S(O)_mR_{12}$;
$R_5$ is $C_1$-$C_3$ alkyl, $C_4$-$C_5$ cycloalkylcarbonyl, F, Cl, Br, $NO_2$, $CO_2R_{14}$, $SO_2N(CH_3)_2$, $SO_2R_{12}$ or phenyl;
$R_6$ is H, $C_1$-$C_3$ alkyl, or $CH_2CH{=}CH_2$;
$R_7$ is H, $CH_3$, $OCH_3$, Cl or Br;
$R_8$ is H, F, Cl, Br, $CH_3$, $OCH_3$, $CF_3$, $SCH_3$ or $OCF_2H$;
$R_9$ is $C_1$-$C_4$ alkyl, $C_3$-$C_4$ alkenyl or $CH_2CH_2Cl$;
$R_{10}$ is H or $C_1$-$C_3$ alkyl;
$R_{11}$ is H or $C_1$-$C_2$ alkyl;
$R_{12}$ is $C_1$-$C_3$ alkyl;
$R_{13}$ is H or $CH_3$;
$R_{14}$ is $C_1$-$C_3$ alkyl or $CH_2CH{=}CH_2$;
m is 0, 1 or 2;
n is 1 or 2;
Q is $CH_2$, $CHCH_3$ or $NR_{15}$;
$R_{15}$ is H or $C_1$-$C_4$ alkyl;
P is O or $CH_2$;
$R_{16}$ is H or $CH_3$;
$R_{17}$ is $C(O)NR_{18}R_{19}$;
$R_{18}$ is H or $CH_3$;
$R_{19}$ is $CH_3$;
$R_{20}$ is H, Cl, F, Br, $CH_3$, $CF_3$, $OCH_3$ or $OCF_2H$;
$R_{21}$ is H or $CH_3$;
X is $CH_3$, $OCH_3$, $OC_2H_5$ or $NHCH_3$;
Y is $CH_3$, $C_2H_5$, $OCH_3$, $OC_2H_5$, $OCF_2H$, $OCH_2CF_3$, Cl, $CH_2OCH_3$ or cyclopropyl;
Z is CH or N;

and their agriculturally suitable salts; provided that

a) when Y is Cl, then Z is CH and X is $OCH_3$;
b) when Y is $OCF_2H$, then Z is CH;
c) when J is J-1 and $R_1$ is $OSO_2R_{12}$ or phenyl, then Y is other than $OCF_2H$;
d) when J is J-2, then Y is other than $OCF_2H$ or $OCH_2CF_3$; and
e) when J is J-3 and $R_4$ is $S(O)_mR_{12}$, then Y is other than $OCH_2CF_3$.

Sulfonylurea herbicides to which the ALS is particularly resistant include

1) Compounds of Formula I where

J is J-1;
$R_1$ is Cl, $CH_3$, $C_1$-$C_4$ alkoxy, $C_1$-$C_2$ haloalkoxy, allyloxy, propargyloxy, $CO_2R_9$, $CONR_{10}R_{11}$, $SO_2N(OCH_3)CH_3$, $SO_2NR_{10}R_{11}$, $S(O)_mR_{12}$, $OSO_2R_{12}$, phenyl or

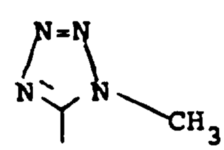 .

2) Compounds of Formula I where

J is J-2;
R is H; and
$R_3$ is $SO_2N(CH_3)_2$, $CO_2CH_3$ or $CO_2C_2H_5$.

3) Compounds of Formula I where

J is J-3
R is H; and
$R_4$ is $CO_2CH_3$ or $CO_2C_2H_5$;

4) Compounds of Formula I where

J is J-4;
R is H;
$R_5$ is Cl, Br, $CO_2CH_3$, $CO_2C_2H_5$ or

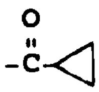 ;

$R_6$ is $CH_3$; and
$R_7$ is H, Cl or $OCH_3$;

5) Compounds of Formula I where

J is J-5;
R is H;
$R_5$ is $CO_2CH_3$ or $CO_2C_2H_5$; and
$R_7$ is H or $CH_3$.

6) Compounds of Formula I where

J is J-6;
Q is $CHCH_3$ or $NR_{15}$;
R is H; and
$R_8$ is H, F, Cl, $CH_3$, $OCH_3$, $CF_3$ or $SCH_3$.

7) Compounds of Formula I where

J is J-7;
R is H;
P is O; and
$R_8$ is H, F, Cl, $CH_3$, $OCH_3$, $CF_3$ or $SCH_3$.

8) Compounds of Formula I where

J is J-8;
R is H;
$R_{16}$ is $CH_3$; and
$R_8$ is H, F, Cl, $CH_3$, $OCH_3$, $CF_3$ or $SCH_3$.

9) Compounds of Formula I where

J is J-9;
R is H; and

$R_{17}$ is $C(O)N(CH_3)_2$.

10) Compounds of Formula I where

R is H;
$R_1$ is Cl, $C_1$-$C_4$ alkoxy, $OCF_2H$, $OCH_2CH_2Cl$, $CO_2R_9$, $CON(CH_3)_2$, $SO_2N(CH_3)_2$, $SO_2R_{12}$ or $OSO_2R_{12}$; and
$R_2$ is H, Cl, $CH_3$, or $OCH_3$.

The nucleic acid fragments of the present invention encode ALS which is resistant to the following triazolopyrimidine sulfonamides:

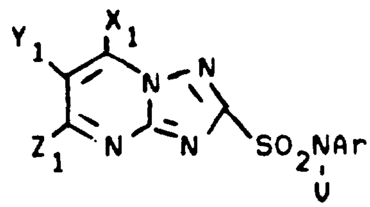

II

wherein

Ar is

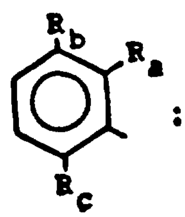

| | |
|---|---|
| $R_a$ | is $C_1$-$C_4$ alkyl, F, Cl, Br, I, $NO_2$, $S(O)_pR_d$, $COOR_e$ or $CF_3$; |
| $R_b$ | is H, F, Cl, Br, I, $C_1$-$C_4$ alkyl or $COOR_e$; |
| $R_c$ | is H, $C_1$-$C_4$ alkyl, F, Cl, Br, I, $CH_2OR_d$, phenyl, $NO_2$ or $COOR_e$; |
| $R_d$ | is $C_1$-$C_4$ alkyl; |
| $R_e$ | is an alkyl, alkenyl, alkynyl, group, each having up to 4 carbon atoms or 2-ethoxyethyl; |
| V | is H, $C_1$-$C_3$ alkyl, allyl, propargyl, benzyl or ($C_1$-$C_3$ alkyl)carbonyl; |
| $X_1$, $Y_1$, and $Z_1$, | are independently H, F, Cl, Br, I, $C_1$-$C_4$ alkyl $C_1$-$C_2$ alkylthio or $C_1$-$C_4$ alkoxy; and |
| p | is 0, 1 or 2. |

Triazolopyrimidinesulfonamide herbicides to which the ALS is particularly resistant include

1) Compounds of Formula II where
V is H.
2) Compounds of Preferred 1 where

$X_1$ is H or $CH_3$;
$Y_1$ is H;
$Z_1$ is $CH_3$; and $R_a$ and $R_c$ are not simultaneously H.

The nucleic acid fragments of the present invention encode ALS which is resistant to the following imidazolinones:

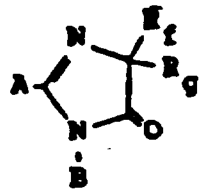

III

wherein

A is

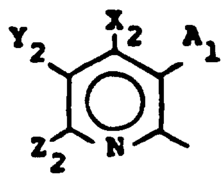

.

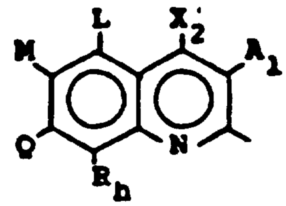

or

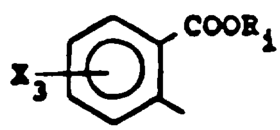

;

| | |
|---|---|
| $R_f$ | is $C_1$-$C_4$ alkyl; |
| $R_g$ | is $C_1$-$C_4$ alkyl or $C_3$-$C_6$ cycloalkyl; |
| $A_1$ | is $COOR_i$, $CH_2OH$ or CHO; |
| $R_i$ | is H; $C_1$-$C_{12}$ alkyl optionally substituted by $C_1$-$C_3$ alkyl, $C_3$-$C_6$ cycloalkyl or phenyl; $C_3$-$C_5$ alkenyl optionally substituted by phenyl or 1-2 $C_1$-$C_3$ alkyl, F, Cl, Br or I; or $C_3$-$C_5$ alkynyl optionally substituted by phenyl or 1-2 $C_1$-$C_3$ alkyl, F. Cl, Br or I; |
| B | is H; $C(O)C_1$-$C_6$ alkyl or C(O)phenyl optionally substituted by Cl, $NO_2$ or $OCH_3$; |
| $X_2$ | is H, F, Cl, Br, I, OH or $CH_3$; |
| $Y_2$ and $Z_2$ | are independently H, $C_1$-$C_6$ alkyl, $C_1$-$C_6$ alkoxy, F, Cl, Br, I, phenyl, $NO_2$, CN, $CF_3$ or $SO_2CH_3$; |
| $X_3$ | is H, $C_1$-$C_3$ alkyl, F, Cl, Br, I or $NO_2$; and |
| L, M, Q and $R_h$ | are independently H, F, Cl, Br, I, $CH_3$, $OCH_3$, $NO_2$, $CF_3$, CN, $N(CH_3)_2$, $NH_2$, $SCH_3$ or $SO_2CH_3$ provided that only one of M or Q may be a substituent other than H, F, Cl, Br, I, $CH_3$ or $OCH_3$. |

Imidazolinone herbicides to which the ALS is particularly resistant include

1) Compounds of Formula III where

B is H; and
$A_1$ is $COOR_i$.

2) Compounds of Preferred 1 where

| | |
|---|---|
| $R_f$ | is $CH_3$; |
| $R_g$ | is $CH(CH_3)_2$; |
| $X_2$ | is H; |
| $Y_2$ | is H, $C_1$-$C_3$ alkyl or $OCH_3$; |
| $Z_2$ | is H; |
| $X_3$ | is H, $CH_3$, Cl or $NO_2$; and |

L, M, Q and $R_h$ are H.

Any of the aforementioned compounds may be applied alone or in combination to the site, pre- and/or post-emergence. Because the crop plant itself is resistant to the herbicide(s), the spectrum of herbicide activity can be chosen for its efficacy in controlling the unwanted vegetation.

The present invention is further defined in the following Examples, in which all parts and percentages are by weight and degrees are Celsius: unless otherwise stated. It should be understood that these examples, while indicating preferred embodiments of the invention, are given by way of illustration only. From the above disclosure and these examples one skilled in the art can ascertain the essential characteristics of this invention, and without departing from the spirit and scope thereof, can make various changes and modifications of the invention to adapt it to various usages and conditions.

EXAMPLE I

Tobacco (Nicotiana tabacum cv. Xanthi) DNA from the Hra mutant was made according to the procedure of Dunsmuir et al. (J. Mol. App. Genetics, 1983, 2, 285). 2 x 13 g of 1-2 inch tobacco leaves were removed from plants and immediately ground in 2 x 20 mL buffer A (10 mM Tricine-KOH pH 7.6 - 1.14 M sucrose - 5 mM $MgCl_2$ - 5 mM 2-mercaptoethanol) in the cold room, using mortars and pestles. An additional 40-50 mL of buffer A was added, and the slurries were filtered through 16 layers of cheesecloth. The filtrates were centrifuged at 2500 rpm in a Sorvall GSA rotor at 4°C for 5 minutes. The pellets were resuspended in 10 mL buffer A, another 100 mL of buffer A was mixed in, and the cells were centrifuged as above. The pellets were then resuspended in 100 mL buffer A + 0.4% Triton X-100, and left on ice for 10 minutes, and centrifuged as above. The pellets were washed twice more in the latter buffer. The final pellets were resuspended in 5 mL of resuspension buffer (50 mM Tris HCl pH 8, 20 mM EDTA), 1 mL of resuspension buffer, 10% sarkosyl was added, and the volumes were then adjusted to 10 mL with suspension buffer. Proteinase K was added to 100 µg/mL to the lysates, and the lysates were digested at 37°C overnight. The lysates were then brought to a density of 1.55 g/mL CsCl, and to a final concentration of 300 µg/mL ethidium bromide. The solutions were centrifuged in a Beckman Ti70.1 rotor at 40000 rpm at 15°C for 24 hours, and the fluorescent DNA band was removed after visualization with longwave UV light. To remove the DNA, holes were punched in the sides of the polyallomer tubes with an 18 gauge needle, and the viscous DNA was allowed to drip into collection tube. Great care was taken at all stages after cell lysis to prevent shearing of the DNA. The DNA was again gently resuspended in a CsCl solution of 1.55 g/mL density and 300 µg/mL ethidium bromide, and centrifuged at 40000 rpm at 15°C for 48 hours, in a Sorvall TFT65.13 rotor. The DNA was again collected by side puncture of the tube. It was gently extracted 10 times with TE (10 mM Tris HCl pH 8, 1 mM EDTA) saturated-isoamyl alcohol, and then dialyzed extensively against TE.

The standard techniques of recombinant DNA and molecular cloning used here are described in R. W. Davis, D. Botstein and J. R. Roth, Advanced Bacterial Genetics, Cold Spring Harbor Laboratory, Cold Spring Harbor, NY (1980) and T. Maniatis, E. F. Fritsch and Sambrook, Molecular Cloning:A Laboratory Manual, Cold Spring Harbor Laboratory, Cold Spring Harbor, NY (1982).

A tobacco DNA library was constructed following the procedures of Maniatis et al (see above). Tobacco DNA was digested with the restriction enzyme Sau 3A to give a majority of fragments in the 20 Kilobase size range, as assayed by agarose gel electrophoresis. The fragments were loaded onto 10-40% sucrose (in 1 M NaCl, 20 mM Tris pH 8, 1 mM EDTA) gradients and size-fractionated by centrifugation in a Beckman SW28 rotor at 26000 rpm at 17°C for 16 hours. Fractions from the sucrose gradients were collected and analyzed by agarose gel electrophoresis, and fractions containing fragments in the 20 kilobase size range were dialyzed against TE and ethanol precipitated. They were then ligated to BamH I cut phage lambda EMBL3 arms, at a 2:1 molar ratio, and packaged into lambda phage heads, following the instructions supplied by the manufacturer of the lambda arms and packaging reactions (Stratagene Cloning Systems, San Diego, CA).

A tobacco DNA library of 400000 phage was plated on the host strain E. coli LE 392 (Silhavy, T. J., Berman, M. L. and Enquist, L. W. (1984), "Experiments with Gene Fusions," Cold Spring Harbor Laboratory, Cold Spring Harbor, N. Y.) at a density of 50000 phage per 150 mm Petri dish, on 10 plates. Duplicate nitrocellulose filter lifts of the phage plaques were made according to the procedure of Maniatis et al., and were hybridized with $^{32}P$-labeled probes carrying either 5′ or 3′ ALS gene fragments produced in a riboprobe labeling system. Riboprobes were synthesized according to the procedures accompanying the riboprobe kit sold by Promega Biotech (Madison, WI). Plaques that gave positive signals on films from both sets of filters were picked and the purification process was reiterated, until well-isolated hybridizing plaques were obtained.

Minipreps of the DNA from plaque purified phage were analyzed by restriction enzyme digestions. Two classes of cloned tobacco DNA fragment inserts were distinguished as shown in Figure 1. Phages 1, 2, 17 and 18 contained inserts related to the previously isolated ALS gene from the SURA locus, encoding herbicide sensitive ALS. Phage 3 contained an insert distinct from the above which was expected to contain the SURB-Hra gene encoding herbicide

resistant ALS. EcoR I fragments that encompassed the hybridizing regions of phage 3 were subcloned into M13 phage vectors and subjected to DNA sequence analysis, using oligonucleotides to extend the sequenced regions in overlapping segments. A single open reading frame of 1992 nucleotides was found, and was identified as an ALS gene by comparison of the deduced amino acid sequence with conserved regions of the amino acid sequences of ALS proteins from other species.

ALS genes isolated from the herbicide-resistant mutant tobacco, Hra, were introduced into sensitive tobacco cells via the "binary vector" system employing Agrobacterium tumefaciens. The ALS genes were first introduced into a binary vector in A. tumefaciens via plasmid conjugation, and the engineered A. tumefaciens were then used to transform plant cells with the foreign genes via co-cultivation.

A) Introduction of the Isolated Tobacco ALS Genes into A. tumefaciens:

i) Construction of Binary Vectors: The standard techniques of recombinant DNA and molecular cloning used here are described in R. W. Davis, D. Botstein and J. R. Roth, Advanced Bacterial Genetics, Cold Spring Harbor Laboratory, Cold Spring Harbor, NY (1980) and T. Maniatis, E. F. Fritsch and Sambrook, Molecular Cloning:A Laboratory Manual, Cold Spring Harbor Laboratory, Cold Spring Harbor, NY (1982). The purified 8.3 kilobase Spe I nucleic acid fragment of the invention, which was isolated from the Hra tobacco mutant and which contains a coding sequence for a herbicide-resistant form of an ALS gene, was inserted into the Xba I site of the plasmid vector pMuc19 (J. D. G. Jones, P. Dunsmiur and J. Bedbrook, EMBO Journal 4:2411-2418 (1985)). (Although, Spe I and Xba I restriction enzymes recognize different DNA sequences, the products of these digestions carry the same 5′ overhanging sequence). The orientation of the insert fragment in one of the resultant plasmids, pAGS148, was determined by restriction enzyme analyses (Fig. 2).

The binary vector pAGS135 was used to move plasmid pAGS148 into A. tumefaciens. Plasmid pAGS135 is derived from plasmid pAGS112 (P. Van den Elzen, K. Y. Lee, J. Townsend and J. Bedbrook, Plant Mol. Biol., 5:149-154 (1985)) by digestion of plasmid pAGS112 DNA with Xho I restriction endonuclease, treatment with the Klenow fragment of E. coli DNA polymerase I, and self-ligation of the DNA which effects the removal of the Xho I site outside the T-DNA right border. Plasmid pAGS112 is derived fro the wide-host range vector pLAFR (A. M. Friedan, S. R. Long, S. E. Brown. S. E. Buikema and F. M. Ausubel, Gene, 18:289-296 (1982)) by the insertion into pLAFR of an EcoR I fragment in which the T-DNA borders flank a gene for expressing kanamycin resistance in plants and a unique BamH I site for cloning (Van den Elzen et. al., Plant Mol. Biol., 5:149-154 (1985)). CsCl purified plasmids pAGS148 and pAGS135 were digested with BamH I, and the resultant BamH I-cleaved plasmids were ligated. The ligation mixtures were packaged into lambda phage particles in vitro and used to infect Escherichia coli strain HB101. Transformants were selected on ampicillin. The physical map of a recombinant plasmid, pAGS152, from one of the transformants was determined by restriction analyses and is shown in Fig. 2.

ii) Conjugation of Plasmid pAGS152 from E. coli into A. tumefaciens: Plasmid pAGS152 was introduced into A. tumefaciens by conjugation essentially by the three-way mating method of Ruvkun, G. and Ausubel, F. M., Nature, 289:85-88 (1981). E. coli strain HB101 harboring plasmid pAGS152 and E. coli strain HB101 harboring the mobilizing vector pRK2013 (ATCC 37159) (D. Figurski and D. R. Helinski, Proc. Natl. Acad. Sci U.S.A., 76:1648-1652 (1979)) were mixed with A. tumefaciens strain LBA4404 harboring plasmid pAL4404 (A. Hoekema, P. R. Hirsch, P. J. J. Hooykaas and R. A. Schilperoort, Nature, 303:179-180 (1983)) and allowed to mate on solid LB medium (J. H. Miller, Experiments in Molecular Genetics, Cold Spring Harbor Laboratory, Cold Spring Harbor, NY (1972)) at 28°C for 16 hours. Transconjugants were selected on plates containing rifampicin at 100 mg/liter and tetracycline at 1 mg/liter. A. tumefaciens LBA4404:pAGS152 was restreaked on minimal A medium containing tetracycline at 1 mg/liter.

Essentially, a similar method was used to obtain both A. tumefaciens LBA4404 containing plasmid pAGS112, the binary vector without any plant nucleic acid fragment insert, and A. tumefaciens LBA4404 containing plasmid pAGS145, the binary vector containing a nucleic acid fragment from phage clone 1. The latter fragment was also isolated from Hra mutant tobacco plants and carries a gene for a herbicide-sensitive form of ALS; this gene is not the wild type allele of the gene for the herbicide-resistant ALS in the nucleic acid fragment of the invention but the SURA gene from the second genetic locus.

B) Introduction of the Isolated ALS Genes Into Sensitive Tobacco by Co-cultivation of the Plant Cells with A. tumefaciens LBA4404 (pAGS145) and LBA4404 (pAGS152).

All manipulations of sterile media and plant materials were done in laminar flow hoods, under suitable containment. Plant growth and plant cell cultures were carried out at 27°C. All protoplast manipulations were carried out at room temperature unless otherwise mentioned.

Day 1 (afternoon):

Protoplast isolation medium was prepared by adding the following to K3/S(1) Medium: 0.1% (w/v) of MES buffer (Sigma Chemical Co.), 1% (w/v) of Cellulase (Onozuka or Cellulysin), and 0.1% of Macerase (Onozuka). After gentle stirring for approximately 30 minutes, the pH was brought to 5.6 with KOH and the medium was filter-sterized.

Sterile tobacco (Nicotiana tabacum var. Wisconsin 38) plants were cultured from 1 cm apical or auxillary explants on OMS Medium in Magenta Boxes under a cycle of a 16 hour light period (6,000-8,000 lux) followed by an 8 hour dark period. When the plants were 5-7 weeks old, fully expanded leaves (3-6 leaves down from the apex) were removed, and two leaves each were placed, top surface down, on 20 mL of protoplast isolation medium in a 100 x 25 mm petri dish. The leaves were then submerged and finely divided with a sharp surgical blade. The midrib was held and the cuts were made outward from it towards the leaf margin at approximately 2 mm intervals. The petri dishes were then sealed with parafilm and the macerated tissue incubated overnight (14-17 hours) in darkness at 27-29°C with gentle gyrotory agitation (20-25 rpm).

Day 2 (morning):

A 75 mm filtering funnel, lined with four layers of cheesecloth, was clamped to a ringstand. A glass tube (approximately 15 cm long and with an outer diameter of <5 mm) was attached to the funnel with latex tubing. The funnel, cheesecloth, latex and glass tubing were wrapped in aluminum foil and sterilized in an autoclave as a unit.

The glass tubing was placed in a Babcock bottle, and the cheesecloth was wetted with K3/S(1) Medium. The digested leaf tissue from two petri dishes was carefully poured into the funnel. The cheesecloth was rinsed and expressed into the bottle. The loaded bottles were topped with K3/S(1) Medium, covered with foil and centrifuged at approximately 100 x g for 10 minutes. The floating protoplasts (1-2 mL) were collected with a 1 mL serological pipette and placed in 20 mL of K3/S(1) Medium in another Babcock bottle. After resuspending the protoplasts by gently swirling, the Babcock bottles were topped and centrifuged as before. The floating protoplasts (1-2 mL) were collected as described above and placed in 30 mL of K3/G(1) Medium. The protoplasts were counted in a hemacytometer, and the volume was adjusted to give 1 x $10^{+5}$ protoplasts/mL. 5 mL aliquots of the protoplasts were plated in petri dishes [100 x 20 mm tissue-culture petri dishes (Corning); these dishes were used in all subsequent protoplast manipulations] and cultured in darkness.

Day 2 (afternoon):

A single colony of A. tumefaciens, containing and desired plant transformation vector, viz., pAGS112 (plasmid vector above), pAGS152 (containing the nucleic acid fragment of the present invention) or pAGS145 (containing a nucleic acid encoding a sensitive form of ALS), growing on a Minimal A plate was inoculated into 5 mL of Minimal A Medium in an 18 mm test tube and cultured overnight on a roller drum at 40-60 rpm at 27-28°C.

Day 3 (morning):

The optical density of the A. tumefaciens cultures was measured at 550 nm and adjusted to 0.15 with Minimal A Medium, and the bacteria were allowed to continue growing as described above.

Day 3 (afternoon):

When the optical density (at 550 nm) of the A. tumefaciens cultures was 0.6 (log phase culture), approximately 6 hours after dilution, the bacteria were added to plant cells at a multiplicity of approximately 50 bacteria/plant cell (an optical density of 1.0 at 550 nm = 1.4 x $10^9$ bacteria). The bacteria and plant cell mixture was co-cultivated for 66 hours at 24°C in low light (approximately 500 lux). Non-transformed protoplast controls were incubated similarly, but without agrobacteria. The following protocol is carried out for each co-cultivation (transformed cells with different agrobacteria, as well as non-transformed cells).

Day 6 (morning):

Co-cultivation was terminated by adding 20 mL of a 1:1 mixture of K3/G(2) Medium:C Medium supplemented with 500 mg/liter of cefotaxime (to select against the agrobacteria) to 5 mL of the co-cultivation mixture. The co-cultivated cells were gently and thoroughly resuspended in the new medium by mixing with a 5 or 10 mL serological pipette. The cell density was 2 x $10^4$ protoplast equivalents/mL (protoplast equivalents = initial protoplasts, assuming 100% recovery and cell survival) and the osmoticum was 0.35 M. Three 5 mL aliquots of each culture were dispensed into fresh petri

dishes.

From this juncture until the cells were embedded in solid medium, the cells were cultured in low light (500-1500 lux) without motion and were aseptically transferred to different media. At the indicated times, cells from one plate of each culture were transferred to non-selective media, while cells from the other two plates of each culture were transferred to selective media containing either 50 mg/liter of kanamycin or 2 ng/mL chlorsulfuron in order to select for transformed plant cells. For these transfers, the contents of each plate were collected with a 5 mL serological pipette, placed in separate 15 mL polystyrene conical centrifuge tubes and centrifuged at approximately 50 x g for 5-10 minutes. The supernatant fluid was removed with a pipette without disrupting the loose pellet. Pellets of co-cultivated cells from each plate were then gently resuspended in the appropriate fresh medium.

Day 10:

The cells were transferred into 5 mL of C Medium supplemented with 500 mg/liter cefotaxime in the case of non-selected plant cells or with 500 mg/liter cefotaxime and either 50 mg/liter of kanamycin or 2 ng/mL chlorsulfuron in the case of selected cells. Each of these cultures was returned to the petri dishes from which they were taken; in this way not all cells needed to be pelleted to effect a medium exchange with minimal cell loss.

Day 13:

Non-selected cells were transferred to 20 mL of a 3:1 mixture of C Medium:MSP Medium supplemented with 500 mg/liter of cefotaxime, and a 5 mL aliquot was dispensed into a fresh petri dish (at a density of 5 x $10^3$ protoplast equivalents/mL). The selected cells were resuspended in 5 mL of a 3:1 mixture of C Medium:MSP Medium supplemented with 500 mg/liter cefotaxime and 50 mg/liter kanamycin or 2 ng/mL chlorsulfuron and returned to the original plates for further culture.

Day 16-17:

The cells were transferred to 5 mL of a 1:1 mixture of C Medium and MSP Medium supplemented with 500 mg/liter cefotaxime alone (in the case of non-selected plant cells) or with 500 mg/liter cefotaxime and either 50 mg/liter of kanamycin or 2 ng/mL chlorsulfuron (in the case of selected cells) and cultured as before.

Day 20:

The non-selected cells were transferred to 25 mL of 1:1 mixture of C Medium:MSP Medium, and the mixture added to 25 mL of a 1:1 mixture of a 2 X MSP Medium and 1% (w/v) type VII agarose solution (50°C). The resultant culture was mixed quickly with a 25 mL wide-mouth serological pipette and dispensed in 5 mL aliquots into fresh petri dishes. The suspended micro calluses in the agar solution were spread carefully and evenly across the plates with agitation by hand. The plates were covered and left in the hood for one hour to solidity before they were wrapped in parafilm and removed to the culture chamber. The cell density was about 5 X $10^2$ protoplasts equivalents/mL and the osmoticum was 0.15 M. The embedded cells were counted on a colony counter approximately 10 days later (Tables 1 and 2, below).

The selected cells were transferred to 20 mL of a 1:3 mixture of C Medium:MSP Medium containing 50 mg/liter of kanamycin or 2 ng/mL chlorsulfuron. Five mL aliquots of the resuspended cultures (5 X $10^{+3}$ protoplast equivalents/mL) were dispensed into four fresh petri dishes per selected culture and cultured as before.

Day 23-24:

Each 5 mL culture of the selected cells was diluted with 7.5 mL of MSP Medium supplemented with 50 mg/liter of kanamycin or 2 ng/mL chlorsulfuron in order to achieve a cell density of 2 X $10^{+3}$ protoplast equivalents/mL. This density-adjusted culture was mixed with 12.5 mL of a 1:1 mixture of 2X MSP Medium and 1.0% (w/v) type VII agarose solution (50°C) supplemented with 50 mg/liter of kanamycin or 2 ng/mL chlorsulfuron. Five mL aliquots of the mixed cultures were quickly dispensed with a 25 mL wide-mouth serological pipette into fresh petri dishes. The final plating density was 1 X $10^3$ protoplast equivalents/mL, and the osmoticum of the culture was 0.1 M. The plates were solidified as described above. The embedded cells were scored for growth on a colony counter approximately 10 days later.

Day 25+:

Ten to twelve individual transformed calluses/colonies were picked and transferred with a No. 11 scalpel to a petri dish containing MSR medium with or without the appropriate selective agent for plant regeneration. The calluses were

cultured at 27°C, with a photo period of 16 hours of light (5000-8000 lux) followed by 8 hours of darkness. Shoots appeared after 2-3 weeks and continued to be produced for several months. Shoots of 2-3 mm length were excited with a sharp surgical blade and transferred for rooting to OMS Medium in Magenta boxes.

After root formation (1 to 4 weeks), plants were transferred to soil for regeneration by the methods of R. S. Chaleff and M. F. Parsons, Proc. Natl. Acad. Sci.-U.S.A. 75:5104 (1978), and B. Tisserat in Plant Cell Culture: A Practical Approach, Ed. Dixon, R. A., IRL Press, Oxford (1985).

Results of Co-cultivation:

The results of the co-cultivation experiments show that the nucleic acid fragment of the invention -- but not the tobacco SURA gene for the herbicide-sensitive form of ALS -- confers herbicide resistance when introduced into herbicide-sensitive tobacco cells (Tables 4 and 5, below). Since the nucleic acid fragment of the invention can confer herbicide resistance at a similar frequency when introduced in either orientation with respect to the vector, it is believed to contain the regulatory sequences both 5′ and 3′ to the coding sequence which are required for the expression of the herbicide-resistant ALS gene.

The level of herbicide resistance conferred by the nucleic acid fragment of the invention was determined by plating one hundred colonies each of pAGS152 transformed N. tabacum cells resistant to chlorsulfuron at 2 ppb and non-co-cultured wild type N. tabacum cells on different concentrations of chlorsulfuron. The number of colonies actively growing on different concentrations of chlorsulfuron after one month was scored (Table 6, below). While wild type colonies are sensitive to chlorsulfuron at 2 ppb, colonies derived from co-cultivation with A. tumefaciens containing pAGS152 could tolerate up to 2000 ppb. This level of resistance of the transformants is comparable to that of the Hra herbicide-resistant mutant tobacco from which the nucleic acid fragment of the invention was isolated, and it is about ten fold higher than that of S4 herbicide-resistant mutant tobacco (parent of Hra).

Table 4

| Transfer of DNA from Phage Clone 1 to Sensitive N. tabacum Cells | | |
|---|---|---|
| Number of colony forming units derived from $10^5$ protoplast equivalents one month after co-cultivation | | |
| | N.t.[1] | N.t./P145[2] |
| no selection | $3.5 \times 10^4$ | $3.6 \times 10^4$ |
| Kanamycin 50 μg/mL | 0 | $5.9 \times 10^2$ |
| Chlorsulfuron 2 ng/mL | 0 | 0 |

1. Non-cocultured (control) plant cells.
2. Plant cells co-cultured with A. tumefaciens harboring pAGS145, kanamycin resistance vector containing the tobacco gene for herbicide-sensitive ALS from phage clone 1.

Table 5

| Transfer of DNA from Phage Clone 3 to Sensitive N. tabacum Cells | | | |
|---|---|---|---|
| Number of colony forming units derived from $10^5$ protoplast equivalents one month after co-cultivation | | | |
| | N.t.[1] | N.t./P112[2] | N.t./p152[3] |
| no selection | $2.0 \times 10^4$ | $2.0 \times 10^4$ | $1.6 \times 10^4$ |
| Kanamycin 50 μg/mL | 0 | $2.5 \times 10^3$ | $6.2 \times 10^2$ |
| Chlorsulfuron 2 ng/mL | 0 | 0 | $6.5 \times 10^2$ |

1. Non-cocultured (control) plant cells.
2. Plant cells co-cultured with A. tumefaciens harboring pAGS112, kanamycin resistance vector.
3. Plant cells co-cultured with A. tumefaciens harboring pAGS152, kanamycin resistance vector containing phage clone 3.

Table 6

| Level of Chlorsulfuron Resistance in Cells of N.tabacum cv. W38 Transformed with Mutant ALS Gene | | |
|---|---|---|
| Number of Colonies Actively Growing After One Month on Selective Media[1] | | |
| chlorsulfuron (ppb) | N. tabacum[2] | N. tabacum/[3] mutant ALS gene |
| 0 | 100 | 100 |
| 20 | 0 | 100 |
| 50 | 0 | 100 |
| 200 | 0 | 100 |
| 500 | 0 | 100 |
| 2000 | 0 | 99 |
| 20000 | N.D.[4] | 6 |
| 50000 | N.D.[4] | 0 |

1. One hundred colonies plated at each chlorsulfuron level.
2. Colonies derived from non-cocultured (control) plant cells.
3. Colonies derived from co-cultivation with A. tumefaciens harboring pAGS152 and initially selected for chlorsulfuron resistance at 2ppb.
4. Not determined.

N. tabacum Culture Media

| $K_3$ Medium | | | |
|---|---|---|---|
| Ingredient | Stock | [Final] | Amount/Liter |
| $K_3$ Major salts | 10X | | 100 mL |
| $CaCl_2 \cdot 2H_2O$ | 100X | | 10 mL |
| Fe EDTA | 100X | | 10 mL |
| B5 vitamins | 100X | | 10 mL |
| MS minors I | 1000X | | 1 mL |
| MS minors II | 1000X | | 1 mL |
| glucose or | --- | 0.4M | 72.08 gm |
| sucrose | | 0.4M | 136.8 |

$K_3$/S (1) - sucrose, phytohormone regime 1 (elevated)
$K_3$/G (1) - glucose, phytohormone regime 1 (elevated)
$K_3$/G (2) - glucose, phytohormone regime 2 (reduced)

| | |
|---|---|
| 1 - NAA 3.0 mg/liter<br>BAP 1.0 mg/liter | 2 - NAA 0.1 mg/liter<br>BAP 0.1 mg/liter |
| bring pH to 5.7, filter sterilize, and store at 5°. | |

| C-Medium | | | |
|---|---|---|---|
| Ingredient | Stock | [Final] | Amount/liter |
| C-Media majors | 10X | | 100 mL |
| Fe EDTA | 100X | | 10 mL |
| B5 vitamins | 100X | | 10 mL |
| MS minors I | 1000X | | 1 mL |
| MS minors II | 1000X | | 1 mL |
| Mannitol | | 0.2M | 36.44 gm |
| Sucrose | | 0.1M | 34.2 gm |
| Mes buffer | | 3.0mM | 590 mg |

(continued)

| C-Medium | | | |
|---|---|---|---|
| Ingredient | Stock | [Final] | Amount/liter |
| NAA | 1 mg/mL | 0.1 mg/liter | 100 ul |
| BAP | 1 mg/mL | 0.1 mg/liter | 100 ul |
| | bring pH to 5.7, filter sterilize. and store at 5°. | | |

| MSP-Medium (for cell proliferation) | | | |
|---|---|---|---|
| Ingredient | Stock | [Final] | Amount/liter |
| MS majors | 10X | | 100 mL |
| Fe EDTA | 100X | | 10 mL |
| B5 vitamins | 100X | | 10 mL |
| MS minors I | 1000X | | 1 mL |
| MS minors II | 1000X | | 1 mL |
| Sucrose | | 0.1 M | 34.2 gm |
| Mes buffer | | 3.0 mM | 590 mg |
| NAA | 1 mg/mL | 0.1 mg/liter | 100 ul |
| BAP | 1 mg/mL | 0.1 mg/liter | 100 ul |
| | bring pH to 5.7, filter sterilize, and store at 5°. | | |

| MSR-Medium (for plant regeneration) | | | |
|---|---|---|---|
| Ingredient | Stock | [Final] | Amount/liter |
| MS major | 10X | | 100 mL |
| Fe EDTA | 100X | | 10 mL |
| B5 vitamins | 100X | | 10 mL |
| MS minors I | 1000X | | 1 mL |
| MS minors II | 1000X | | 1 mL |
| Sucrose | | 0.1 M | 34.2 gm |
| Mes buffer | | 3.0 mM | 590 mg |
| NAA | 1 mg/mL | 0.1 mg/liter | 100 ul |
| BAP | 1 mg/mL | 1.0 mg/liter | 1.0 mL |
| | | bring pH to 5.7. ---> add agar | |
| Agar (T.C.) autoclave | | 0.8% (w/v) | 8.0 gm |
| Kanamycin Sulfate or | 50 mg/mL | 50 ug/mL | 1.0 mL |
| Chlorsulfuron | 1 mg/mL in 5 mM KOH | as desired | - |

Dispense 25 mL/100x25 mm petri dish and, if required, aseptically add selective agents when media has cooled to 50°.

| OMS-Medium (for plant maintenance) | | | |
|---|---|---|---|
| Ingredient | Stock | [Final] | Amount/liter |
| MS majors | 10X | | 100 mL |
| Fe EDTA | 100X | | 10 mL |
| MS minors I | 1000X | | 1 mL |
| MS minors II | 1000X | | 1 mL |
| B5 vitamins | 100X | | 10 mL |
| Sucrose | | 3.0% w/v | 30 gm |

(continued)

| OMS-Medium (for plant maintenance) | | | |
|---|---|---|---|
| Ingredient | Stock | [Final] | Amount/liter |
| Mes buffer | | 3.0 mM | 590 mg |
| | pH 5.7. ---> add agar | | |
| Agar (T.C.) | | 0.8% (w/v) | 8.0 gm |
| autoclave, dispense 50 mL/3" x 4" Magenta Box | | | |

| Minimal A Medium | | | |
|---|---|---|---|
| Ingredient | Stock | [Final] | Amount/liter |
| $K_2HPO_4$ | | | 10.5 g |
| $KH_2PO_4$ | | | 4.5 g |
| $(NH)_2SO_4$ | | | 1.0 g |
| Sodium citrate $2H_2O$ | | | 0.5 g |
| | autoclave in 990 mL | | |
| $MgSO_4$ $7H_2O$ | 1M | 1mm | 1.0 mL add sterile |
| Glucose | 20% | | 10.0 mL add sterile |

To solidify media: autoclave agar (15 gm/liter) Difco Bacto. in separate 500 mL volume. Then mix salts and agar before dispensing.

| Stock | Ingredient | [Final] | Amount/Liter |
|---|---|---|---|
| MS major salts (10x) | $NH_4NO_3$ | 20.6 mM | 16.5 g |
| | $KNO_3$ | 18.8 mM | 19.0 g |
| | $MgSO_4$ $7H_2O$ | 1.5 mM | 3.7 g |
| | $KH_2PO_4$ | 1.25 mM | 1.7 g |
| | $CaCl_2$ $2H_2O$ | 3.0 mM | 4.4 g |
| C-Medium major salts (10X) | $NH_4NO_3$ | 5.0 mM | 4.0 g |
| | $KNO_3$ | 15.0 mM | 15.2 g |
| | $MgSO_4$ $7H_2O$ | 3.0 mM | 7.4 g |
| | $K_2PO_4$ | 0.5 mM | 0.68 g |
| | $CaCl_2$ $2H_2O$ | 3.0 mM | 4.4 g |
| K Medium major salts (10X) | $KNO_3$ | | 25.0 g |
| | $(NH_4)_2SO_4$ | | 1.34 g |
| | $MgSO_4$ $7H_2O$ | | 2.5 g |
| | $KH_2PO_4$ | | 2.01 g |
| | $NH_4NO_3$ | | 2.5 g |
| $CaCl_2$ $2H_2O$ (100X) | $CaCl_2 \cdot 2H_2O$ | | 92.3 g |
| Fe-EDTA (100X) | $Na_2EDTA$ | | 3.73 g |
| | $FeSO_4 \cdot 7H_2O$ | | 2.78 g |
| (dissolve EDTA entirely before adding $FeSO_4$; pH to 3.0) | | | |
| Ms minor I (1000X) | $H_3BO_3$ | | 0.620 g |
| | $MnCl_2 \cdot 4H_2O$ | | 1.980 g |

(continued)

| Stock | Ingredient | [Final] | Amount/Liter |
|---|---|---|---|
| | $ZnSO_4 \cdot 7H_2O$ | | 0.920 g |
| MS minor II (1000X) | KI | | 83 mg |
| | $Na_2MoO_4 \cdot 2H_2O$ | | 25 mg |
| | $CuSO_4 \cdot 5H_2O$ | | 2.5 mg |
| | $CoCl_2 \cdot 6H_2O$ | | 2.16 mg |

| Stock | Ingredient | [Final] | Amount/Liter |
|---|---|---|---|
| B5 vitamins (100X) | nicotinic acid | | 0.1 g |
| | thiamin HCl | | 1.0 g |
| | pyridoxine HCl | | 0.1 g |
| | myo-inositol | | 10.0 g |
| NAA | Naphthelene acetic acid 1 mg/mL (dissolve in dilute KOH) | | 1.0 g |
| BAP | Benzylamino purine 1 mg/mL (dissolve in dilute HCl) | | 1.0 g |

| Supplemental Apparatus, Chemicals & Media | |
|---|---|
| MES buffer (2 [N-Morpholino] ethanesulfonic Acid) | Sigma No. M-8250 |
| Agarose Type VII Low Gelling Temperature (stock maintained molten at 50°) | Sigma No. A-4018 |
| Cellulysin™ | Calbiochem 219466 |
| Macerase™ | Calbiochem 441201 |
| Cefotaxime, sodium salt | Calbiochem 219380 |
| dilute w/g.d., sterile $H_2O$ | |
| store @ 5°, dark, < 10 day as 50 mg/mL stock | |
| Kanamycin Sulfate | Sigma No. K-4000 |
| dilute w/g.d., $H_2O$, filter sterile | |
| store @ -20°, dark as 50 gm/mL stock | |
| Chlorsulfuron | E. I. du Pont de Nemours and Company, Wilmington, Delaware 19898 |
| 100 mm x 20 mm tissue culture petri dish | Corning 25020 |
| Babcock bottle | Kimble |
| Centrifuge (Babcock compatible) | Damon/IEC Division HN-SII |
| Magenta Boxes 3" x 4" | Magenta Corp. 4149 W. Montrose Ave Chicago, IL 68641 |
| T.C. Agar | KC Biological CR-100 |

EXAMPLE II

Tobacco DNA from the C3 mutant was prepared and a genomic DNA library in bacteriophage vector EMBL3 was constructed as described in EXAMPLE I. Phage carrying ALS genes were identified, as described in EXAMPLE I, by hybridization to a $^{32}P$-labeled 5′ tobacco ALS gene fragment probe.

Six independent recombinant phage were isolated in a screen of 600,000 recombinants from the C3 library. Restriction endonuclease analysis of these isolated phage indicated that the DNA inserts of three phage could be aligned

with the SURA gene from the Hra library (phages 35, 36 and 38). The remaining three phage (phage 31, 34 and 37) had DNA inserts corresponding to the SURB gene. It was expected that the ALS gene carried on phages 35, 36 and 38 would be the SURA-C3 gene, encoding herbicide resistant ALS and the ALS gene carried on phages 31, 34 and 37 would be the SURB gene, encoding herbicide sensitive ALS.

DNA fragments from phages 31, 35 and 38 were subcloned into the pUC119 plasmid and subsequently into the pAGS135 binary vector essentially as described in EXAMPLE I. An approximately 8.3 kb Spe I restriction endonuclease fragment from phage 31, analogous to that present in pAGS148 (Fig. 2), but carrying the SURB gene encoding herbicide sensitive ALS, was subcloned in both possible orientations in the vector. An approximately 6.3 kb Spe I-Sal I restriction endonuclease fragment from phage 35 and an approximately 7.8 kb Spe I-Sal I fragment from phage 38 were subcloned yielding pALS35(ATCC #67424) and pALS38, respectively. The fragments included 2.5 kb in the 5′ direction (upstream) of the ALS coding region, 2.0 kb of ALS coding sequence, encoding herbicide resistant enzyme and 1.8 and 3.3 kb, respectively in the 3′ direction (downstream) from the ALS coding region. The latter two subcloned fragments contain a BamH I restriction endonuclease site. Partial BamH I digestions or pALS35 and pALS38 were employed for insertion of these plasmids into the BamH I site of the binary vector pAGS135. The ALS genes in the binary vector, designated p312, p313, p351 and p381 (Table 7) were moved into A. tumefaciens by tri-parental mating, as described in EXAMPLE I.

Introduction of the ALS genes into herbicide sensitive tobacco by co-cultivation of plant cells with A. tumefaciens carrying the ALS genes in the binary vector was performed as described in EXAMPLE I. The results of these co-cultivation experiments are shown in Table 7. The ALS gene isolated in phage 31, i.e. the SURB gene encoding herbicide sensitive ALS, yielded no herbicide resistant plant cells, as expected. The ALS gene isolated in phages 35 and 38, i.e. the SURA-C3 gene encoding herbicide resistant ALS did yield herbicide resistant plant cells. Herbicide resistant plant cells arose at a lower frequency than kanamycin resistant plant cells and at a lower frequency than was observed when the SURB-Hra gene was used. This may reflect either the lesser resistance to the herbicide of the ALS enzyme encoded by the SURA-C3 gene compared to that encoded by the SURB-Hra gene, or the lower expression of the SURA-C3 gene compared to the SURB-Hra gene, or both.

Table 7

| ALS Reintroduction Exp. 3 | | | | | | |
|---|---|---|---|---|---|---|
| Transfer of DNA from phage clones 31, 35 & 36 to Sensitive N.tabacum Cells | | | | | | |
| Number of Colony Forming Units derived from $10^5$ Protoplast Equivalents One Month after Co-cultivation | | | | | | |
| | N.t.[1] | N.t./p152[2] | N.t./P312[3] | N.t./p313[4] | N.t./p351[5] | N.t./p381[6] |
| no selection | $2.1x10^4$ | $1.5x10^4$ | $1.1x10^4$ | $1.9x10^4$ | $1.5x10^4$ | $1.4x10^4$ |
| Kanamycin 50 ug/ml | 0 | 88 | 65 | 48 | 139 | 87 |
| Chlorsulfuron 2 ng/ml | 0 | 83 | 0 | 0 | 32 | 18 |

1. Non co-cultured plant cells.
2. Plant cells co-cultured with A. tumefaciens harboring pAGS152, S4/Hra subclone.
3. Plant cells co-cultured with A. tumefaciens harboring pAGS312, C3 subclone, ϕ31 (orientation 1).
4. Plant cells co-cultured with A. tumefaciens harboring pAGS313, C3 subclone, ϕ31 (orientation 2).
5. Plant cells co-cultured with A. tumefaciens harboring pAGS351, C3 subclone, ϕ35.
6. Plant cells co-cultured with A. tumefaciens harboring pAGS381, C3 subclone, ϕ38.

## EXAMPLE III

Mutations were made in the wild-type SURA gene of tobacco in vitro in order to make it encode a herbicide resistant ALS. Restriction endonuclease fragments containing part of the SURA gene were subcloned into M13 phage vectors or plasmid vectors containing an M13 origin of replication to allow production of single-stranded DNA. The specific DNA fragment subcloned depended upon the region to be mutagenized in vitro and the availability of restriction endonuclease sites.

Oligonucleotides 16-17 bases in length, which hybridized to the single-stranded DNA from the SURA ALS gene with single base mismatches, were synthesized. These mismatches were designed to convert amino acid codons found in the wild-type ALS gene to the codons found in ALS genes which encode ALS enzymes resistant to sulfonylurea herbicides. These oligonucleotides include 5′ GTTCAATTGGAGGATC 3′, to change trp 591 to leu, 5′ GTCAAGTGGCACGTAGG 3′, to change pro 197 to ala, and 5′ GTCAAGTGTCACGTAGG 3′, to change pro 197 to ser, 5′ ATGTACCTGAGGATATT 3′ to change lys 256 to glu, 5′ GAGGTTTGTTGATAGAG 3′ to change asp 384 to val, 5′AGGTTT-

GAGGATAGAGT 3′ to change asp 384 to glu, 5′ TACTGATGATTTTCAGG 3′ to change ala 205 to asp and 5′ CAG-GTGGCCCTTCCATG 3′ to change ala 122 to pro.

The oligonucleotides were hybridized to single-stranded DNA and used as primers for synthesis of a complementary strand in a reaction catalyzed by the Klenow fragment of DNA polymerase I, following the procedures of Carter et al. (Oligonucleotide site-directed mutagenesis in M13, Anglian Biotechnology Limited, England, 1985). The resulting DNA was transformed into competent E. coli mutL cells (Kramer et al., 1984, Cell 38, 879). Individual plaques or colonies were purified, depending on whether M13 phage vectors (M13mp18/19) or M13 replication origin plasmid vectors (pTZ18R/19R) were used. Mini-preps of single-stranded DNA were made and used in DNA sequencing reactions to identify clones that carried the mutated bases.

These in vitro constructed site-specific mutations can be incorporated singly or in combination into the wild-type SURA gene which includes the 5′ and 3′ regulatory sequences needed to provide expression of the gene in plant cells (see EXAMPLE II). This is accomplished by substituting restriction endonuclease fragments carrying the mutations into a plasmid carrying the SURA gene from which the analogous fragment has been removed. The choice of the restriction fragment to substitute depends upon the position of the mutation in the gene and the availability of restriction endonuclease sites. The introduction of the mutated SURA genes into plant cells is then accomplished as described in EXAMPLES I and II. Any of the DNA fragments containing mutations which result in production of herbicide resistant ALS, as disclosed in the description of the invention, can be produced essentially by this method. Furthermore, the mutations need not be made exclusively in the SURA gene. Analogous mutations can be made in the SURB gene or any other plant gene encoding ALS for which DNA sequence information is available.

EXAMPLE IV

DNA was prepared from Beta vulgaris cv. Sennika (sugarbeet) and a genomic DNA library in bacteriophage lambda vector EMBL3 was constructed as described in EXAMPLE I. Recombinant phage (300,000) were screened by hybridization to a $^{32}$P-labeled 5′ ALS tobacco gene fragment probe as described in EXAMPLE I. The filters were washed at 42°C (0.1XSSC) and 20 individual clones were isolated. On the second round of purification the recombinant phage were hybridized to both the 3′ tobacco ALS gene probe and the 5′ probe. In addition, the filters which had been hybridized to the 5′ probe were washed at 55°C. Only one clone, ϕ21, hybridized to both 5′ and 3′ probes and also remained hybridized after the 55°C wash. Minilysate DNA preparations were made from the 20 clones and digested with EcoR I and Nco I. The different isolates had different restriction endonuclease digestion patterns and again only ϕ21 hybridized to both probes and remained hybridized after a 55°C wash. One phage, ϕ41, also had a hybridizing band remaining after a 55°C wash but it did not hybridize to the 3′ probe. Figure 7 shows the restriction endonuclease map of the phage ϕ21, together with subclones which have been constructed from it. The ALS coding region was localized to a 3.0 kb BamH I - Hind III fragment by hybridization with 5′ and 3′ probes from the N. tabacum gene. Both DNA strands of this fragment have been sequenced. The BamH I - Hind III fragment was subcloned into pUC119 of Bluescript vectors; then Exonuclease III or Bal 31 deletions were generated. The dideoxy sequencing method was used. A comparison of the deduced amino acid sequence encoded by the sugarbeet gene with that of the tobacco gene(s) indicates no homology in the first 88 amino acids of the predicted protein (see Figure 8). This region may represent the chloroplast transit peptide. Thereafter the homology is approximately 90% with an insertion of 4 amino acids around residue 290 of tobacco ALS. Inspection of the amino acid residues which define the sites for herbicide resistance identified in tobacco and yeast indicate that these residue are conserved in sugarbeet ALS also. These data allow a straightforward approach to the construction of a gene encoding herbicide resistant sugarbeet ALS enzyme, by site-directed mutagenesis, as described in EXAMPLE III.

Three sites have been mutagenized in this sugarbeet gene. The codon GCA for ala at position 122 (numbering of amino acid residues from Figure 6) was changed to CCA for pro, the codon CCA for pro at position 197 was changed to GCA for ala and the codon TGG for trp at position 591 was changed to TTG for leu. The double mutation yielding pro to ala at 197 and trp to leu at 591, which mimics the tobacco SURB-Hra gene, was also made by combining the two single mutations.

In order to transform plants with these in vitro constructed mutations in the sugarbeet ALS gene, DNA fragments containing the mutations and extending from the BamH I site about 910 bp in the 5′ direction (upstream) of the coding region to the Pst I site about 1000 bp in the 3′ direction (downstream) of the coding region (see Figure 17) were cloned into plasmid vector pUC119. These were introduced into the binary vector pAGS140 for transformation into plant cells as described in EXAMPLE I. The binary vector pAGS140 is similar to pAGS135 (Figure 2) except that between the BamH I site and the right border of the T-DNA of the Ti-plasmid of Agrobacterium tumefaciens a gene which will confer resistance on plants to the antibiotic hygromycin was inserted.

Introduction of the ALS genes into herbicide sensitive tobacco and sugarbeet, by co-cultivation of the plant cells with A. tumefaciens carrying the ALS genes in the binary vector, was performed as described in EXAMPLE I and EXAMPLE II. Results of a co-cultivation experiment in tobacco are shown in Table 8. Herbicide resistant transformants

were obtained with three of the four mutant sugarbeet ALS genes. The frequency of obtaining herbicide resistant transformants was lower than that for kanamycin resistant transformants, and also lower than the frequency of herbicide resistant transformants obtained when the tobacco SURB-Hra gene was used. It is believed that this results from poor expression of the mutant sugarbeet ALS genes in tobacco. This may reflect either insufficient nucleotide regulatory sequences upstream or downstream of the mutant sugarbeet ALS genes in the DNA fragments used or poor utilization of sugarbeet nucleotide regulatory sequences in tobacco or both. It is expected that the mutant sugarbeet ALS gene which did not yield herbicide resistant transformants would have done so if more transformants had been obtained and tested.

Table 8

| Herbicide Resistance in Plant Cells Transformed with Site Specific Mutant Sugarbeet ALS Gene | | | | | |
|---|---|---|---|---|---|
| Gene Origin | Tobacco | Sugarbeet | Sugarbeet | Sugarbeet | Sugarbeet |
| Mutation | pro(197)-ala/trp (591)-leu | pro(197)-ala/trp(591)-leu | trp(591)-leu | pro(197)-ala | ala(122)-pro |
| Chlorsulfuron$^R$ | 698 | 91 | 2 | 35 | 0 |
| Kanamycin$^R$ | 1083 | 1362 | 1080 | 1073 | 415 |
| $Ch_1^R$/Kan$^R$ | .645 | .067 | 1.85x10$^{-3}$ | .033 | - |

Gene introductions were done by standard co-cultivation method. For each construction an aliquot of the co-cultured plant cells (2x10$^5$ starting plant cells) was scored for chlorsulfuron and another aliquot for kanamycin resistance. Selection was with chlorsulforon at 2ppb or kanamycin at 50ppm.

## EXAMPLE V

The SURB-Hra gene described in EXAMPLE I was transformed into tobacco cultivars by Agrobacterium tumefaciens infection of tobacco leaf disks and progeny of the transformants were analyzed to demonstrate expression of resistance at the whole plant level and inheritance of the herbicide resistance trait. Standard aseptic techniques for the manipulation of sterile media and axenic plant/bacterial cultures were followed, including the use of a laminar flow hood for all transfers. Potted tobacco plants for leaf disk infections were grown in a growth chamber maintained for a 12 hr, 24°C day, 12 hr, 20°C night cycle, with approximately 80% RH, under mixed cool white fluorescent and incandescent lights. Tobacco leaf disk infections were carried out essentially by the method of Horsch, R.B., Fry, J. E., Hoffmann, N. L., Eichholtz, D., Rogers, S.G., Fraley, R.T., (1985). Science 227: 1229-1231.

Young leaves, not fully expanded and approximately 4-6 inches in length, were harvested with a scalpel from approximately 4-6 week old tobacco plants (Nicotiana tabacum CV.NK326 or K14). The leaves were surface sterilized for 30 minutes by submerging them in approximately 500 ml of a 10% Chlorox, 0.1% SDS solution and then rinsed 3 times with sterile deionized water. Leaf disks, 6 mm in diameter, were prepared from whole leaves using a sterile paper punch.

Leaf disks were inoculated by submerging them for several minutes in 20 ml of a 1:10 dilution of an overnight Agrobacterium culture carrying the plasmid pAGS152. Agrobacterium cultures were started by inoculating 10 ml of Min A (EXAMPLE I) broth with a single bacterial colony removed form a Min A plus tetracycline (EXAMPLE VI) plate. The culture was grown for approximately 17-20 hours in 18 mm glass culture tubes in a New Brunswick platform shaker maintained at 28°C.

After inoculation, the leaf disks were placed in petri dishes containing CN agar medium (EXAMPLE VI). The dishes were sealed with parafilm and incubated under mixed fluorescent and "Gro and Sho" plant lights (General Electric) for 2-3 days in a culture room maintained at approximately 25°C.

To rid the leaf disks of Agrobacterium and to select for the growth of transformed tobacco cells, the leaf disks were transferred to fresh CN medium containing 500 mg/l cefotaxime and 100 mg/l kanamycin. Cefotaxime was kept as a frozen 100 mg/ml stock solution and added aseptically (filter sterilized through a 0.45 μm filter) to the media after autoclaving. A fresh kanamycin stock (50 mg/ml) was made for each use and was filter sterilized into the autoclaved media.

Leaf disks were incubated under the growth conditions described above for 3 weeks and then transferred to fresh media of the same composition.

Approximately 1-2 weeks late shoots developing on kanamycin-selected explants were excised with a sterile scalpel and planted in A medium containing 100 mg/l kanamycin. Root formation on selective and non-selective media was recorded within 3 weeks. Shoots which rooted in kanamycin were transferred to soil and grown in a growth chamber as described above. After 3 to 5 weeks, but before flowering had occurred, leaf tissue was excised and used for ALS

assays as described in Example VI. The results of these assays, which indicate that a herbicide resistant form of ALS is being produced, are shown in Table 9. The plants exhibiting herbicide resistant ALS activity were then moved to a greenhouse where they were grown to maturity. Individual flowers were bagged to permit self-fertilization without cross-pollination. Mature seeds were harvested and progeny tests were conducted to determine inheritance of the herbicide resistance trait. Seeds were surface sterilized as described above, dried and planted on SG medium (1/4 MS salts, 0.75% sucrose, 0.8% Agar) in the presence or absence of herbicide (DPX-F6025, Classic®). Sensitive seeds germinated, but did not develop further. Results of the progeny analysis are shown in Table 9. A segregration ratio of 3 resistant progeny to 1 sensitive indicated the presence of a single-site insertion of the SURB-Hra gene in the trasnformant which was stably inherited. This was seen in 15 of 17 transformants. Higher ratios of resistant to sensitive progeny indicated multiple insertions at unlinked positions in the genome. The 15/1 ratio indicates the presence of 2 unlinked SURB-Hra genes and 255/1 ratio indicates 4 unlinked SURB-Hra genes in the transformants K14 #40 and K14 #7, respectively.

Table 9

| | % Unhibited ALS Activity[1] | Progeny Resistant/Sensitive[2] | | Segregation Ratio Resistant/Sensitive |
|---|---|---|---|---|
| | | 100ppb | 1000bbp | |
| NK326(wT) | 7 | --- | --- | --- |
| nk326 #1 | 36 | 98/37 | 90/35 | 3/1 |
| NK326 #9c | 47 | 163/49 | 99/63 | 3/1 |
| NK326 #9d | 37 | 288/67 | 150/58 | 3/1 |
| NK326 #10 | 26 | 93/31 | 96/24 | 3/1 |
| NK326 #10c | 56 | 333/45 | 290/76 | 3/1 |
| K14 wT | 7 | --- | --- | --- |
| K14 #7 | 71 | 990/4 | 109/1 | 255/1 |
| K14 #11 | 52 | 208/85 | 127/76 | 3/1 |
| K14 #27 | 45 | 129/45 | 108/42 | 3/1 |
| K14 #29 | 30 | 192/46 | 163/67 | 3/1 |
| K24 #31 | 44 | 106/35 | 99/34 | 3/1 |
| K14 #32c | 32 | 140/65 | 63/86 | 3/1 |
| K14 #40 | 41 | 218/18 | 212/26 | 15/1 |
| K14 #41 | 40 | 255/35 | 296/74 | 3/1 |
| K14 #42 | 29 | 162/74 | 77/72 | 3/1 |
| K14 #53 | 37 | 130/59 | 149/139 | 3/1 |
| X14 #54 | 34 | 99/38 | 92/43 | 3/1 |
| K14 #54A | 28 | 137/55 | 100/72 | 3/1 |

[1]The ALS activity in each line is related to the activity in the absence of herbicide which is taken as 100 percent. The sulfonylurea herbiciee used was DPX-F6025 (Classic®) at a concentration of 10ppb.

[2]Resistant progeny are able to growth at the indicated concentrations of herbicide DPX-F6025 (Classic®).

EXAMPLE VI

To transform herbicide sensitive tomato to resistance the SURB-Hra gene from tobacco, carried on the binary vector pAGS152 in A. tumefaciens strain LBA4404, was used (see EXAMPLE I).

Standard aseptic techniques for the manipulation of sterile media and axenic plant/bacterial cultures were followed, including the use of a laminar flow hood for all transfers.

Seeds of tomato (Lycopersicon esculentum var. Herbst Red Cherry) was surface sterilized for 30 minutes in a 10% Chlorox, 0.1% SDS solution and rinsed 3 times with sterile deionized water. The seeds were planted in Magenta boxes (Magenta Corp.) containing 100 ml of OMS agar medium and germinated under mixed fluorescent and "Gro and Sho" plant lights (General Electric) in a culture room maintained at approximately 25°C. Cotyledons from 10-15 day old seedlings were used for Agrobacterium inoculation.

Cotyledons were wounded by removing approximately 2 mm of tissue from each end of the cotyledon with a sterile scalpel. Wounded cotyledons were planted in petri dishes on CTM agar medium either with or without 75μM acetosyringone (Aldrich Chemical).

In preparation for the cotyledon inoculation, a single bacterial colony from a Min A + tetracycline (1 μg/ml) agar

plate was inoculated into a flask containing 30 ml of Min A broth (EXAMPLE I) and grown for 2 days at 28°C in a New Brunswick platform shaker. On the morning of the cotyledon inoculation, the bacterial culture was diluted with sterile Min A broth to an OD of 0.1 at 650 nM and allowed to multiply to an OD of 0.2 under the growth conditions previously described. This culture was then used undiluted for the inoculation.

CTM agar plates containing the colyledon explants were flooded with 5 ml of the bacterial solution for approximately 5 minutes, before removal of the solution. The plates were then secured with Time Tape (Shamrock Scientific Specialty) on two sides of the dish and incubated for 2 days under mixed fluorescent and "Gro and Sho" plant lights (General Electric) at approximately 25°C for two days.

To rid the plant cultures of Agrobacterium and to select for the growth of transformed tomato cells, the cotyledon explants were transferred to fresh CTM medium containing 500 mg/L cefotaxime and 50 mg/L kanamycin and incubated under the same culture conditions described above for approximately 3 weeks. The cotyledons were than transferred to fresh media of the same composition and selective agents as CTM but with 1/10 the zeatin concentration.

After approximately 2-4 weeks, shoots developing off of kanamycin-selected cotyledons were excised and planted in OMS media containing 500 mg/L cefotaxime and 100 mg/L kanamycin. Tomato shoots which rooted in kanamycin after about 2-3 weeks were transferred to soil in 8" pots and covered with plastic bags. The plants were grown under mixed fluorescent and incandescent lights for a 12 hr, 24°C day; 12 hr, 20°C night cycle, with approximately 80% relative humidity, for one week before removing the plastic bags. The plants were grown for another 2-4 weeks before performing ALS assays. An increase of uninhibited ALS activity in the presence of the sulfonylurea Classic® in leaf extracts from transformed plants was demonstrated in these experiments (Table 10).

Table 10

| ALS Activity of Wild-Type and Transformed Tomato | | | | |
|---|---|---|---|---|
| | Percent Uninhibited ALS Activity[1] | | | |
| | 0ppb | 10ppb | 100ppb | 1000ppb |
| Wild-type | 100 | 15 | 5 | 4 |
| Transformant #3 | 100 | 42 | 25 | 12 |
| Transformant #4a | 100 | 60 | 42 | 26 |
| Transformant #4b | 100 | 29 | 15 | 5 |
| Transformant #4c | 100 | 58 | 43 | 25 |
| Transformant #4d | 100 | 29 | 15 | 10 |

[1]The ALS activities in each line are relative to the activity in the absence of herbicide which is taken as 100 percent. The sulfonylurea compound used was DPX-F6025, the active ingredient in Classic® herbicide.

The assay for ALS activity in the absence or presence of herbicide from transformed or untransformed plants was conducted as follows:

1. Grind 2.5 grams of relatively young leaf tissue (4-6 inches in length) in a tissue homogenizer containing 10 ml extraction buffer (100 mM $KHPO_4$) pH 7.5, 0.5 mM $MgCl_2$, 10% glycerol, 1 mM pyruvate, to 0.5 mM TPP, 10 nM FAD) and 200 mg Polyclar AT (BDH Biochemicals). Keep on ice.
2. Homogenize extract for approximately 10 seconds in a polytron (Brinkman Instruments) on setting #7.
3. Centrifuge extract in a Sorvall SS-34 rotor, 20 min. 16K rpm, 4°C.
4. Equilibrate PD-10 (Pharmacia) columns by washing with column buffer (100 mM $KHPO_4$ pH 7.5, 0.5 mM $MgCl_2$, 10% glycerol, 1 mM pyruvate) 5 times.
5. To plant extract supernatant, add cold saturated $(NH4)_2SO_4$ to achieve a 50% cut. Incubate on ice for 30 minutes.
6. Centrifuge extract in SS-34 rotor, 20 minutes, 16K rpm, 4°C. Decant supernatant.
7. Resuspend pellet in 1 ml cold column buffer.
8. Load extract onto column and chase with a volume of column buffer to achieve total volume loaded equal to 2.5 ml. Let this run through column.
9. Elute proteins with 2X volume of extract loaded. Recover in 15 ml Falcon tube placed beneath column.
10. Set up reaction for each extract in microfuge tubes as follows: 350 μl reaction mix (200 mM pyruvate, 5 mM TPP, 0.9 mM FAD, 5 mM $KHPO_4$ pH 7.0), 50 μl of either 5 mM $KHPO_4$ or desired sulfonylurea concentration, and 100 μl plant extract.
11. Incubate reaction for 1 hour, 30°C.
12. To stop reaction, add 50 μl 6 M $H_2SO_4$ and incubate at 60°C for 10 minutes. Spin in microfuge 5 minutes.

13. Set up color development tubes as follows: 500 µl 0.5% creatin, reaction tube supernatant, 0.5 ml α-napthol solution (1.5 g α-napthol in 30 ml 2.5 N NaOH). Mix and heat at 60°C for 20 minutes.
14. Vortex each tube and load 100 µl of each sample into wells of microtiter plate. Read at OD 530.

| B | |
|---|---|
| Callus Induction Medium | |
| 1 package of MS salts (Gibco Murashige Organics Medium) with 3% sucrose<br>1 ml of 1 mg/ml NAA<br>0.2 ml of 1 mg/ml BAP<br>0.8% agar | per liter<br>pH 5.8 |

| CN | |
|---|---|
| Shoot Induction Medium | |
| 1 package of MS salts with 3% sucrose<br>1 ml of 1 mg/ml NAA<br>1 ml of 1 mg/ml BAP<br>0.8% agar | per liter<br>pH 5.8 |

| | |
|---|---|
| A | |
| Root Induction Medium | |
| 1 package of MS salts (without sucrose)<br>10 grams sucrose<br>0.8% agar | per liter<br>pH 5.8 |
| Agrobacterium R-Medium | |
| | Add 7.5 g agar to 440 ml $H_2O$, autoclave, and keep at 55°C. Add sterile stocks of: |
| 0.5 Ml | 1 M $MgSO_4$ |
| 0.5 ml | 1 M $CaCl_2$ |
| 10.0 ml | 20% sucrose |
| 5.0 ml | 100 mg/ml kanamycin |
| 50.0 ml | 10x salts ($Na_2HPO_4 \cdot 7H_2O$ 60 g/l; $KH_2PO_4$, 30 g/l; NaCl, 5 g/l; $NH_4Cl$, 10 g/l) |

CTM Medium

1 pkg MS salts
1 ml B5 vitamins (per 100 ml: Nicotinic Acid 100 mg. thiamine, hydrochloride 1000 mg, pyridoxine hydrochloride 100 mg, M-inositol 10,000 mg)
3 mM MES
3% glucose
0.7% agar
pH 5.7
Autoclave and add 1 ml 1 mg/ml zeatin stock

OMS Medium

1 pkg MS salts
1 ml B5 vitamins (see above)
3 mM MES
3% sucrose
0.8% agar
pH 5.7

Min A + Tetracycline (1 ug/ml) Medium

1. Add 7.5 g agar to 400 ml $H_2O$

2. Make stock:

| | |
|---|---|
| $K_2HPO_4$ | 5.25 g |
| $KH_2PO_4$ | 2.25 g |
| $(NH_4)_2\ SO_4$ | 0.5 g |
| Sodium Citrate $2H_2O$ | 0.25 g |
| | 100 ml |

3. Make $MgSO_4 \cdot 7H_2O$ stock = 20 g/100 ml, autoclaved

4. Make glucose stock = 20% solution, autoclaved

5. Make tetracycline stock = 1.0 mg/ml in ethanol/$H_2O$, 50% v/v filter sterilized

To make Min A medium + 1 µg/ml tetracyline:

Mix (1) and (2)
Add 0.5 ml of (3). 5 ml of (4), and 0.5 ml of (5)

| YEB Medium | |
|---|---|
| | per liter |
| Bacto Beef Extract | 5.0 g |
| Bacto Yeast Extract | 1.0 g |
| Peptone | 5.0 g |
| Sucrose | 5.0 g |
| $MgSO_4 \cdot 7H_2O$ | 0.5 g |
| Agar (optional) | 15.0 g |

Herbicide solutions: A 1 ppm stock solution of sulfonylurea herbicide can be made by dissolving 1 mg of herbicide in 100 ml of 0.01N $NH_4OH$, and then diluting 1:10 with 5 mM $KHPO_4$ pH 7.0. This

stock will suffice to assay herbicide concentrations of 100 ppb or lower. If higher concentrations are desired, dissolve 1 mg in 10 ml of 0.01N $NH_4OH$, etc.

Herbicide dilutions: In the standard array, 50 µl of herbicide is added to 450 µl assay mix and extract, for a 1:10 dilution of herbicide. So, for each concentration to be tested, a 10X solution in 5 mM $KHPO_4$ pH 7.0 should be diluted from the stock solution.

EXAMPLE VII

The tobacco SURB-Hra gene encoding herbicide resistant ALS was used to transform *Beta vulgaris* (sugarbeet) to herbicide resistance by the following *Agrobacterium tumefaciens* mediated transformation procedure.

In order to surface sterilize seeds, 50-100 seeds were placed in a 25 x 100mm sterile petri dish in laminar flow hood and 25-30 ml of 70% ethanol was added. Seeds were agitated by hand 1-2 min., the ethanol was decanted, and 25-30ml 20% Clorox (20 ml commercial bleach/80ml sterile $H_2O$/1 drop Tween 80) was added. The seeds were agitated on gyrotary shaker @ 40 rpm for 20 mins., and the bleach was decanted. The bleach sterilization was repeated 2 times for a total of 60 min., and the sterilized seeds were rinsed 3 times for 5 min. each with 25-30ml sterile $H_2O$.

To germinate the seeds, they were plated on 1-2$PG_O$ agar solidified medium 12 seed/50 ml media/15x 150mm petri dish, and cultured @ 24°C in darkness.

NOTE: 10-20% contamination of seed may be anticipated for a good, clean seed lot. For this reason seed is plated far apart on large plates and the germinations are monitored continuously and non-contaminated seed are transferred to fresh plates. If contamination is fungal, then transfers are conducted in a laminar flow cabinet with the fan off.

60-80% germination is expected for a good seed lot. Germination is not syncronous. An approximately 14 day period is required to obtain (a) all germinations and (b) sufficient elongation to provide many appropriate explants.

Prepare *Agrobacterium* overnight (O/N) suspension cultures as described in EXAMPLE I. Freshly plated Agrobacterium have shorter lag times than cultures stored for long periods at 5°C. It is important that log phase bacteria is used as inocula. Therefore, some trial should be conducted to assure an overnight culture which reaches log phase (OD 550 mm 0.4-0.8) before hypocotyl inoculation).

To prepare plant explants, hypocotyls were cut into approximately 0.5cm sections with a sharp surgical blade, and plated immediately onto agar solidified $PG_O$ $^{0.1}$0.1. Do not allow dessication or damage the wound by use of dull blade or by compressing with forceps.

To inoculate explants, they were dipped individually into a log phase suspension of the appropriate *Agrobacterium* strain. They were immersed briefly (1-3 sec.) and arranged in a grid pattern on a fresh plate: 25 explants/100mm plate of agar solidified $PG_O$ $^{0.1}$/0.1.

The explants were dried by leaving plates open in a laminar flow hood 10-30 min. This concentrates *Agrobacteria* onto the wound. It also many limit possible damage by water logging. It is important, though, not to dessicate tissue. Close observation of this step is required. The plates were then sealed with parafilm and cultured at 24°C for 3 days.

The explants were collected into liquid PG $^{0.1}$/0.1 containing cefotaxime 500µg/ml in a 25 x 100mm petri dish, and shaken gently on a gyrotary shaker at 40 rpm for 1 hr. The media was decanted, replaced with fresh counterselective media, and shaken gently for an additional 2 hrs. The explants were plated in grids, 25/100mm plate agar solidified PG $^{0.1}$/0.1 containing cefotaxime 500µg/ml and cultured at 24°C for 3 days.

Selection for transformed plant cells was applied as follows. Explants were transferred to fresh plates of agar solidified PG$^{0.1}$/0.1 containing vancomycin 100µg/ml or chlorsulfuron, 2ng/ml as selective agents. The number of resistant colonies was counted after 20-30 days. More than one may be observed at each wound. Transformants were excised as follows. Callus was removed from the wound/explant with a surgical blade and cultured independently on fresh selective media. Some *Agrobacterium* can escape from counter-selection. Additional washes in cefotaxime containing liquid media are possible, as is repeated transfer to cefotaxime containing agar solidified plates. Under the suggested protocol we observed approximately 15% explant contamination, which was an acceptable loss. The results of a transformation experiment using the sugarbeet line *Beta vulgaris* 87193 are shown in Table 11. The level of chlorsulfuron resistance in calli of *B. vulgaris* transformed with the SURB-Hra mutant ALS gene of tobacco, is compared to that of untransformed calli. These results demonstrate that the tobacco SURB-Hra gene, encoding herbicide-resistant ALS, can be expressed efficiently in sugarbeet.

Table 11

| Chlorsulfuron (ppb) | | | | | | | | |
|---|---|---|---|---|---|---|---|---|
| | 0 | 10 | 30 | 100 | 300 | 1000 | 3000 | 10000 |
| 87193/152[1] | 15/15 | 15/15 | 15/15 | 15/15 | 15/15 | 15/15 | 3/15 | 0/15 |
| 87193/0[2] | 15/15 | 0/15 | 0/15 | nd | nd | nd | nd | nd |

[1] Beta vulgaris line 87193 transformed with Agrobacterium tumefaciens LBA4404 carrying plasmid pAGS152.

[2] Untransformed Beta vulgaris line 87193.

| Media and Ammendments | | | | |
|---|---|---|---|---|
| 1/2 $PG_O$ | Ingredient | Stock | (Final) | Amt./Liter |
| | $PG_O$ Majors A | 10X | | 50ml |
| | $PG_O$ Majors B | 100X | | 5 |
| | FeEDTA | 100X | | 5 |
| | B5 vitamins | 100X | | 5 |
| | MS micronutrients | 1000X | | 1 |
| | Sucrose | | 1.5%w/v | 15gm |
| | Mes buffer | | 3mM | 590mg |
| | T.C. agar | | 0.8%w/v | 8gm |
| | pH5.7, autoclave sterile, dispense aspectically into 15x150mm petri plates | | | |
| PGO $^{0.1}/_{0.1}$ | Ingredient | Stock | (Final) | Amt./Liter |
| | $PG_O$ Majors A | 10X | | 100ml |
| | $PG_O$ Majors B | 100X | | 10 |
| | FeEDTA | 100X | | 10 |
| | B5 vitamins | 100X | | 10 |
| | MS micronutrients | 1000X | | 1 |
| | Sucrose | | 3.0%w/v | 30gm |
| | Mes buffer | | 3mM | 590mg |
| | 2,4-D | 1mg/ml | | 100ml |
| | BIAP | 1mg/ml | | 100µl |
| | pH5.7, autoclave sterile. | | | |
| $PG_O$ $^{0.1}$/0.1 | agar solidified, as above except with T.C. agar 0.8%w/v. Dispense 25ml/25X100 petri dish. | | | |

| Stock Solutions | | | | |
|---|---|---|---|---|
| Stock | Ingredient | MW | (Final) | Amount/Liter |
| $PG_O$ Majors A (10X) | | | | |
| | $KNO_3$ | 101.1 | 19.8mM | 20 |
| | $(NH_4)_2SO_4$ | 132.14 | 3 | 4 |
| | KCl | 74.55 | 8 | 6 |
| | $MgSO_4.7H_2O$ | 146.5 | 2 | 5 |
| | $CaCl_2.2H_2O$ | 147.0 | 2 | 3 |
| $PG_O$ Majors B (100X) | | | | |
| | $NaH_2PO_4$ | 120.0 | 2.1mM | 25 |

(continued)

| Stock Solutions | | | | |
|---|---|---|---|---|
| Stock | Ingredient | MW | (Final) | Amount/Liter |
| Fe EDTA (100X)M | | | | |
| | $FeSO_4\ 7H_2O$ | | 100µm | 2.78gm |
| | $Na_2EDTA$ | | 100µm | 3.72 |
| | Dissolve EDTA first. pH3.0. Store @ 4°C, dark | | | |
| B5 vitamins (100X) | | | | |
| | Nicotinic acid | | 1mg/lit | 0.1gm |
| | Thiamine HCl | | 10 | 1.0 |
| | Pyridoxine HCl | | 1 | 0.1 |
| | Myo-inositol | | 100 | 10 |
| MS micronutrients (1000X) | | | | |
| | $MnCl_2\ 4H_2O$ | 197.9 | 100µm | 19800mg |
| | $H_3BO_3$ | 01.8 | 100 | 6200 |
| | $ZnSO_4\ 7H_2O$ | 287.5 | 30 | 8625 |
| | KI | 166 | 5 | 830 |
| | $NaMoO_4\ 2H_2O$ | 206 | 1.2 | 250 |
| | $CuSO_4\ 5H_2O$ | 249.7 | 0.1 | 25 |
| | $CoCl_2\ 6H_2O$ | 237.9 | 0.1 | 25 |
| | Dissolve $MnCl_2\ 4H_2O$ in dil HCl | | | |
| | Dissolve one at a time and completely before adding next. | | | |
| | Boil, cool, pH 4.0. store dark at 4°C. | | | |

EXAMPLE VIII

The tobacco SURB-Hra gene encoding herbicide resistant ALS was used to transform Brassica napus cv. Olga by the following Agrobacterium tumefaciens mediated transformation procedure.

To surface sterilize seed, they were immersed for 1 min. in 70% ethanol, then 30-60 min. in Clorox/Tween (see EXAMPLE VII). The surface sterilized seeds were germinated on 1/2 MS, 1/2 PGO (SEE EXAMPLE VII), 24°C in the dark, At 5-10 days post germination, hypocotyls were divided into 0.5cm sections and placed on solid I medium containing Acetosyrigone 100 µm (IAS100).

Immediately the explants were dipped individually into a log phase suspension of LBA 4404 containing binary plasmid pAGS15.

The explants were plated back onto IAS100. The Agrobacterium droplet was carefully dried down onto the tissue by leaving the plate open in a laminar flow hood. Co-cultivation was conducted at 24°C in low light or darkness for 3 days.

After 3 days the explants were collected into liquid I medium containing cefotaxime 500mg/L in 100x25mm petri dishes, and shaken on a gyrotory shaker at 40rpm for 3 hrs.

The explants were plated on solid I medium containing cefotaxime 500mg/mL, and cultured for 3 days at 24°C in low light.

The explants were plated on solid I medium containing vancomycin 100 mg/L and kanamycin 100 mg/L.

After about 1 month transformed callus appeared as discreet nodules at the ends of explants.

As nodules appeared, they were excised with a sharp scalpel and placed on solid I medium containing kanamycin 100 mg/L.

When transformed callus reached a sufficient size (0.5cm diameter) it was transferred to KR medium containing kanamycin 100 mg/L. This material regenerates fastest if it is plated on fresh media every two weeks. Roots were regenerated on 1/2 MS containing IBA 2µm.

In one experiment, of 100 wound sites (either end of 0.5cm hypocotyl sector) 20 developed callus tissue which was resistant to kanamycin (100 mg/L). Five of the 20 transformed cell lines were subsequently induced to regenerate on kanamycin and somatic siblings for each regenerant genotype were produced by nodal multiplication. These plants were sprayed with various chlorsulfuron concentrations and the results are summarized in Table 12. Two of the five transformants are resistant to chlorsulfuron at levels which are about 10 times greater than that which is lethal to control (untransformed) plants.

Table 12

| | 0.3 ppm | 1 ppm | 3 ppm | 10 ppm |
|---|---|---|---|---|
| untransformed | ++ | + | - | - |
| $R_o$ #1 | ++ | ++ | ++ | + |
| $R_o$ #2 | ++ | + | - | - |
| $R_o$ #3 | ++ | + | + | - |
| $R_o$ #4 | ++ | + | - | - |
| $R_o$ #5 | +++ | ++ | ++ | + |

+++ normal growth, no axial induction
++ reduced growth, sublethal at apis. axial induction
+ reduced growth, lethal at apis, axial induction
- lethal

To further demonstrate the expression of the SURB-Hra gene in transformed Brassica napus, an ALS assay in the presence of the herbicide chlorsulfuron was performed as described in EXAMPLE VI. The ALS activities of the untransformed parent and transformant $R_o$ #5 (Table 12) were compared (Table 13). A consistent increase in the percent uninhibited ALS activity was observed in the transformant. Thus, the tobacco SURB-Hra gene, encoding herbicide-resistant ALS, can be expressed in Brassica napus, but that expression is not efficient. Addition of Brassica napus nucleotide regulatory sequences would be expected to increase the level of expression of herbicide resistant ALS and the level of tolerance to foliar application of the herbicide.

Table 13

| ALS Activity of Wild Type and Transformed Brassica napus | | | | | |
|---|---|---|---|---|---|
| | Percent Uninhibited ALS Activity[1] | | | | |
| | 0ppb | 1ppb | 10ppb | 100ppb | 1000ppb |
| Wild Type | 100.0 | 86.6 | 28.2 | 10.1 | 7.6 |
| Transformant $R_o$#5 | 100.0 | 88.1 | 36.6 | 20.1 | 14.5 |

[1] The ALS activities are relative to that in the absence of herbicide which is taken as 100 percent. The sulfonylurea compound used was chlorsulfuron (DPX-W4189), the active ingredient in Glean® herbicide.

| Brassica napus | | Culture Media | |
|---|---|---|---|
| I Media | | | |
| Ingredient | Stock | (Final) | Amount/Liter |
| MS Major Salts | 10X | | 100 ml |
| MS Micronutrients | 1000X | | 1 ml |
| Fe EDTA | 100X | | 10 ml |
| I Vitamins | 100X | | 10 ml |
| 2,4-D | 1mg/ml | | 0.2 ml |
| Kinetin | 1mg/ml | | 3 ml |
| Sucrose | | 3% w/v | 30 gm |
| Mannitol | | 1.8%w/v | 18.2 gm |
| T.C. agar | | 0.8%w/v | 8 gm |

(continued)

| Brassica napus | | Culture Media | |
|---|---|---|---|
| I Media | | | |
| Ingredient | Stock | (Final) | Amount/Liter |
| Mes Buffer | | 3 mM | 0.59 gm |
| | pH 5.7, autoclave sterile | | |
| KR Media | | | |
| Ingredient | Stock | (Final) | Amount/Liter |
| K3 Major Salts | 10X | | 100 ml |
| $CaCl_2$ $2H_2O$ | 100X | | 10 ml |
| MS Micronutrients | 1000X | | 1 ml |
| Fe EDTA | 100X | | 10 ml |
| B5 Vitamins | 100X | | 10 ml |
| Zeatin* | 1mg/ml | | 2 ml |
| IAA* | 1mg/ml | | 0.1 ml |
| Sucrose | | 1% w/v | 10 gm |
| Xylose | | 0.025%w/v | 0.25 gm |
| Agarose (Type 1, low E/Eo) | | 0.25% w/v | 2.5 gm |
| Mes Buffer | | 3mM | 0.59 gm |
| | pH 5.7, autoclave sterile | | |

*add these filter sterilized components aseptically

| Brassica napus | | Stock Solutions | | |
|---|---|---|---|---|
| Stock | Ingredient | (Stock) | (Final) | Amount/Liter |
| MS Major Salts | $NH_4NO_3$ | 10X | 20.5 mM | 16.5 gm |
| | $KNO_3$ | | 18.8 | 19.0 |
| | $MgSO_4$ $7H_2O$ | | 1.5 | 3.7 |
| | $KH_2PO_4$ | | 1.25 | 1.7 |
| | $CaCl_2$ $2H_2O$ | | 3.0 | 4.4 |
| K3 Major Salts | $KNO_3$ | 10X | 25.0 mM | 25.0 gm |
| | $(NH_4)_2SO_4$ | | 1.0 | 1.34 |
| | $MgSO_4$ 7 $H_2O$ | | 1.0 | 2.5 |
| | $KH_2PO_4$ | | 1.5 | 2.01 |
| | $NH_4NO_3$ | | 3.1 | 2.5 |
| $CaCl_2$ $2H_2O$ | $CaCl_2$ $2H_2O$ | 100X | 6.3 mM | 92.3 gm |
| MS Micronutrients | $MNCl_2$ $4H_2O$ | 1000X | 100 μm | 19800 mg |
| | $H_3BO_3$ | | 100 | 6200 |
| | $ZnSO_4$ $7H_2O$ | | 30 | 8625 |
| | KI | | 5 | 830 |
| | $NaMoO_4$ $2H_2O$ | | 1.2 | 250 |
| | $CuSO_4$ $5H_2O$ | | 0.1 | 25 |
| | $CoCl_2$ 6 $H_2O$ | | 0.1 | 25 |
| Fe EDTA | $Na_2$ EDTA | 100X | 100 μm | 3.73 gm |

(continued)

| Brassica napus | | Stock Solutions | | |
|---|---|---|---|---|
| Stock | Ingredient | (Stock) | (Final) | Amount/Liter |
| | $FeSO_4\ 7H_2O$ | | 100 | 2.78 |
| I Vitamins | Myo-Inositol | 100X | 100mg/l | 1000mg |
| | Thiamine | | 0.5 | 50 |
| | Glycine | | 2.0 | 200 |
| | Nicotinic acid | | 5.0 | 500 |
| | Pyrodoxine | | 0.5 | 50 |
| | Folic acid | | 0.5 | 50 |
| | Biotin | | 0.05 | 5 |

EXAMPLE IX

The tobacco SURB-Hra gene encoding herbicide resistant ALs was used to transform Cucumis melo cv. Amarillo Oro (melon) to herbicide resistance by the following Agrobacterium tumefaciens mediated transformation procedure. A reference to this procedure is Moreno, V., et al. Plant regeneration from calli of melon. Plant Cell Tissue Organ Culture, 5 (1985) 139-146.

Surface sterilization of seeds was greatly facilitated by first removing the seed coat. The seeds were then sterilized by rinsing in 70% ethanol for 2 min. then washing in Clorox/Tween (see EXAMPLE VII) for 20 mins. The sterile seeds were washed 3 times in the sterile distilled $H_2O$ and germinated on OMS at 24°C with a 16 hr. day length.

Cotyledons of 7-14 day old melon seedlings were cut into 5 mm slices with a fresh, sharp scalpel. These explants were dipped into a log phase Argobacterium culture prepared as described in EXAMPLE I, transferred to fresh co-cultivation plates and cultures at 24°C with 16 hr. days for 3 days.

The bacteria were killed by washing the explants for 3 hrs. with gentle agitation in washing media and cultured on fresh selection plates.

The explants were subcultured every 3-4 weeks, dissecting the more compact, darker green sectors away from the white fluffier callus.

When "morphogenic" callus (very dark green, compact, perhaps some recognizable leaves) was seen, it was transferred to regeneration media. The tissue can go directly to shoots instead of going through the morphogenic stage. Shoots were rooted in rooting media. Approximately 70% of the explants developed callus resistant to kanamycin at 100µm/l. Transformed callus was put on media containing increasing concentrations of chlorsulfuron and growth in the presence of herbicide was determined by weighing the callus after 30 days (Table 14). Some transformants grew as well at high concentrations of chlorsulfuron (1000ppb) as in its absence, e.g. Trans 1, Trans 2 and Trans 7. Thus the tobacco SURB-Hra gene can function to transform melon to high level herbicide resistance.

Table 14

| Chlorsulfuron ppb | Nontransformed | Trans 1 | Trans 2 | Trans 3 | Trans 4 | Trans 5 |
|---|---|---|---|---|---|---|
| 0 | 5.7 | 30.4 | 30.4 | 30.4 | 30.4 | 30.4 |
| 50 | 0 | 44.5 | 19.5 | 10 | 14 | 175 |
| 100 | 0 | 25.9 | 0 | 7.5 | 16.6 | 70 |
| 500 | 0 | 51.9 | 14.8 | 0 | 0 | 3.7 |
| 1000 | 0 | 46 | 26 | 11.7 | 10 | 5.7 |
| 2000 | 0 | 19.1 | 8 | 28.7 | 5 | 0 |
| 3000 | 0 | 15.2 | 18 | 0 | 0 | 0 |
| 4000 | 0 | 41.9 | 3.3 | 0 | 0 | 0 |
| 5000 | 0 | 14.4 | 0 | 3.6 | 0 | 0 |
| | | Trans 6 | Trans 7 | Trans 8 | Trans 9 | Trans 10 |
| | | 30.4 | 26.4 | 28.3 | 27.2 | 40 |
| | | 28.8 | 46.5 | 18.1 | 27.3 | 40 |
| | | 18.3 | 25.5 | 14.9 | 11.1 | 39 |

Table 14 (continued)

| Chlorsulfuron ppb | Nontransformed | Trans 1 | Trans 2 | Trans 3 | Trans 4 | Trans 5 |
|---|---|---|---|---|---|---|
| | | Trans 6 | Trans 7 | Trans 8 | Trans 9 | Trans 10 |
| | | 2 | 16.1 | 2 | 10.4 | 18.6 |
| | | 4.7 | 19.3 | 2.6 | 9 | 27.6 |
| | | 0 | 19.3 | 0 | 8.8 | 23.5 |
| | | 0 | 17.1 | 10 | 13.7 | 17.6 |
| | | 0 | 20.7 | 3.4 | 3 | 7.6 |
| | | 0 | 26.5 | 7.4 | 2.6 | 8.9 |
| Measurements indicate fold increase in weight of callus. | | | | | | |

Media

| OMS | |
|---|---|
| MS Salts and Fe EDTA | 1X |
| B5 Vitamins | 1X |
| Sucrose | 3% |
| MES | 3mM |
| pH 5.7 | |
| T.C. agar | 0.8% |
| Autoclave 20 min. | |

| Basic medium | |
|---|---|
| MS Salts and Fe EDTA | 1X |
| Myo inositol | 100mg/l |
| Thiamine | 1mg/l |
| Sucrose | 3% |
| MES | 3mM |
| pH 5.7 | |
| T.C. agar | 0.8% |
| Autoclave 20 min. | |

Co-cultivation Medium is Basic medium plus:

Acetosyringone 100 µm (acetosyringone is kept as a 100mM stock in DMSO)

| | |
|---|---|
| Kinetin | 6mg/l |
| IAA | 1.5 mg/l |

Washing Medium is Basic Medium without agar plus:

| | |
|---|---|
| Cefotaxime | 500 mg/l |
| Kinetin | 6 mg/l |
| IAA | 1.5 mg/l |

Selection Medium is Basic Medium plus:

| | |
|---|---|
| Kinetin | 6 mg/l |
| IAA | 1.5 mg/l |
| Vancomycin | 100 mg/l |

One of the following selective drugs depending upon Agrobacterium construction:

| | |
|---|---|
| Kanamycin | 100 mg/l |
| Hygromycin | 50 mg/l |
| Chlorsulfuron | 100 mg/l |

Regeneration Medium is Basic Medium plus:

| | |
|---|---|
| BAP | 0.1mg/l |
| Vancomycin | 100 mg/l |

Selective drugs as above

Rooting Medium is OMS plus:

| | |
|---|---|
| IBA | 2μm |
| Vancomycin | 100 mg/l |

Selective drugs as above

EXAMPLE X

The tobacco SURB Hra gene encoding herbicide resistant ALS was used to transform Medicago sativa cv. Rangelander (alfalfa) to herbicide resistance by the following Agrobacterium tumefaciens mediated transformation procedure. A reference to this procedure is Deak, M., Kiss, G., Koncz, C., and Dudits, D. Transformation of Medicago by Agrobacterium mediated gene transfer (preprint).

Plants were grown and subcultured in OMS. The materials used were petioles and stem segments (5mm in length) from plants about 2 months from the last subculture.

The petioles and stems of sterile plants were cut into 5mm lengths with a fresh, sharp scalpel. These explants were dipped into a log phase Agrobacterium culture prepared as described in EXAMPLE I, transferred to fresh co-cultivation plates, and cultured at 24°C with 16 hr. days for 3 days.

The bacteria were killed by washing the explants for 3 hrs. with gentle agitation in washing media, and cultured on fresh selection plates.

The explants were subcultured every 3-4 weeks. In about 1 month transformed callus growing out of the wounded ends of the explants was seen, dissected away from the explant and plated on fresh selection media. When callus became more organized, (areas are darker green and more compact) it was transferred to fresh maturation media.

When developed embryos appeared, they were transferred to gemination media. After germination, the small plants were grown on the same medium.

Less than 1% of explants developed callus resistant to kanamycin at 100mg/L. Kanamycin resistant sectors were found to be resistant to the herbicide chlorsulfuron at 50 ppb. Three shoots were produced from kanamycin resistant callus. Tissue from these transformants was assessed for herbicide resistant growth over a range of chlorsulfuron concentrations. One transformant was able to grow at 1000ppb chlorsulfuron; the other two were able to grow at 5000ppb. Thus, the tobacco SURB-Hra gene can function to transform alfalfa to high level herbicide resistance.

Media

| OMS | |
|---|---|
| MS Salts and Fe EDTA | 1X |
| B5 Vitamins | 1X |
| Sucrose | 3% |
| MES | 3mM |
| pH 5.7 | |
| T.C. agar | 0.8% |
| Autoclave 20 min | |

| Basic Medium | |
|---|---|
| MS Salts and Fe EDTA | 1X |
| UM Vitamins | 1X * |
| Sucrose | 3% |
| pH 5.7 | |
| T.C. agar | 0.8% |
| Autoclave 20 min. | |

*100X UM vitamins
(amounts given for 100ml of 100x stock)

| | |
|---|---|
| Thiamine HCl | 1g |
| Nicotinic acid | 0.5g |
| Pyridoxine HCl | 1g |
| Myo inositol | 10g |
| Glycine | 0.2g |

Co-Cultivation Medium
Basic Medium plus
Acetosyringone 100µm (Acetosyringone is kept as a stock of 100mM in DMSO)

| | |
|---|---|
| 2,4-D | 0.5mg/l |
| BAP | 0.2mg/l |

## Claims

**Claims for the following Contracting States : BE, CH, DE, FR, GB, GR, IT, LI, LU, NL, SE**

**1.** A nucleic acid fragment comprising a nucleotide sequence encoding acetolactate synthase, said nucleitide sequence corresponding at least in part to a plant acetolactate synthase gene, conferring increased sulfonylurea herbicide resistance on a plant in which said fragment is operative, said nucleotide sequence comprises at least one sub-sequence which encodes one of the substantially conserved amino acid sub-sequences designated A, B, C, D, E, F, and G in Figure 6, the nucleic acid fragment is further characterized in that at least one of the following conditions is met;

a) the nucleic acid fragment has a sequence which encodes an amino acid sub-sequence A wherein $\varepsilon_1$ is an amino acid other than alanine, and/or $\varepsilon_2$ is an amino acid other than glycine,
b) the nucleic acid fragment has a sequence which encodes an amino acid sub-sequence B wherein $\alpha_1$ is an amino acid other than proline,
c) the nucleic acid fragment has a sequence which encodes an amino acid sub-sequence C wherein $\delta_2$ is an amino acid other than alanine,
d) the nucleic acid fragment has a sequence which encodes an amino acid sub-sequence D wherein $\lambda_1$ is an amino acid other than lysine,
e) the nucleic acid fragment has a sequence which encodes an amino acid sub-sequence E wherein $\gamma_1$ is an amino acid other than aspartic acid,
f) the nucleic acid fragment has a sequence which encodes an amino acid sub-sequence F wherein $\beta_3$ is an amino acid other than tryptophan, and/or $\beta_8$ is an amino acid other than valine and/or $\beta_7$ is an amino acid other than phenylalanine, and
g) the nucleic acid fragment has a sequence which encodes an amino acid sub-sequence G wherein $\sigma_1$ is an amino acid other than methionine.

**2.** A nucleic acid fragment according to Claim 1, wherein the fragment has a sequence which encodes an amino acid

sub-sequence A having the formula

$$PG\varepsilon_2A$$

wherein:

P is proline,
G is glycine,
$\varepsilon_2$ is an amino acid other than glycine, and
A is alanine.

**3.** A nucleic acid fragment according to claim 1 or Claim 2, wherein $\varepsilon_2$ is an amino acid selected from the group consisting of serine, threonine or cysteine.

**4.** A nucleic acid fragment according to Claim 3, wherein $\varepsilon_2$ is serine.

**5.** A nucleic acid fragment according to Claim 1, wherein the fragment has a sequence which encodes an amino acid sub-sequence A having the formula

$$PGG\varepsilon_1$$

wherein:

P is proline,
G is glycine, and
$\varepsilon_1$ is an amino acid other than alanine or glycine

**6.** A nucleic acid fragment according to Claim 1, wherein the fragment has a sequence which encodes an amino acid sub-sequence B having the formula

$$GQV\alpha_1$$

wherein:

G is glycine,
Q is glutamine,
V is valine
$\alpha_1$ is an amino acid other than proline.

**7.** A nucleic acid fragment according to claim 1 or Claim 6 wherein $\alpha_1$ is an amino acid selected from the group consisting of alanine, glycine, arginine, lysine, histidine, serine, cysteine, threonine, glutamine and asparagine.

**8.** A nucleic acid fragment according to Claim 7, wherein $\alpha_1$ is alanine, arginine, serine or glutamine.

**9.** A nucleic acid fragment according to Claim 1 , wherein the fragments has a sequence which encodes an amino acid sub-sequence C having the formula

$$IG\delta_1D\delta_2FQE$$

wherein:

I is isoleucine.
G is glycine.
$\delta_1$ is selected from the group consisting of amino acids.

D is aspartic acid, and
$\delta_2$ is an amino acid other than alanine;
F is phenylalanine,
Q is glutamine, and
E is glutamic acid.

**10.** A nucleic acid fragment according to claim 1 Claim 9, wherein $\delta_2$ is an amino acid selected from the group consisting of threonine, serine, cysteine, tyrosine, aspartic acid, glutamic acid, tryptophan, histidine, phenylalanine, arginine, and lysine.

**11.** A nucleic acid fragment according to Claim 10, wherein $\delta_2$ is threonine, cysteine, aspartic acid, glutamic acid, tryptophan or arginine.

**12.** A nucleic acid fragment according to Claim 1 , wherein the fragment has a sequence which encodes an amino acid sub-sequence D having the formula

$$P\lambda_1 D$$

wherein:

P is proline;
$\lambda_1$ is an amino acid other than lysine; and
D is aspartic acid.

**13.** A nucleic acid fragment according to claim 1 or Claim 12, wherein $\lambda_1$ is an amino acid selected from the group consisting of glycine, alanine, leucine, isoleucine, valine, threonine, serine, cysteine, tyrosine, glutamic acid, aspartic acid, proline, asparagine, glutamine, tryptophan and phenylalanine.

**14.** A nucleic acid fragment according to Claim 13, wherein $\lambda_1$ is glycine, leucine, threonine, glutamic acid, aspartic acid, asparagine, tryptophan or proline.

**15.** A nucleic acid fragment according to Claim 1 , wherein the fragment has a sequence which encodes an amino acid sub-sequence G having the formula

$$MLG\sigma_1 HG$$

wherein

M is methionine,
L is leucine,
G is glycine
$\sigma_1$ is an amino acid other than methionine,
H is histidine, and
G is glycine.

**16.** A nucleic acid fragment according to claim 1 or Claim 15, wherein $\sigma_1$ is an amino acid selected from the group consisting of glutamic acid, aspartic acid, proline, valine, leucine, or isoleucine.

**17.** A nucleic acid fragment according to Claim 16, wherein $\sigma_1$ is proline, glutamic acid, or valine.

**18.** A nucleic acid fragment according to Claim 1 , wherein the fragment has a sequence which encodes an amino acid sub-sequence E having the formula

$$RFD\gamma_1 R$$

wherein:

R is arginine,
F is phenylalanine,
D is aspartic acid, and
$\gamma_1$ is an amino acid other than aspartic acid.

**19.** A nucleic acid fragment according to claim 1 or Claim 18. wherein $\gamma_1$ is an amino acid selected from the group consisting of glycine, alanine, valine, leucine, isoleucine, serine, threonine, cysteine, tyrosine, glutamic acid, proline, asparagine, glutamine, lysine, arginine, tryptophan, histidine, and phenylalanine.

**20.** A nucleic acid fragment according to Claim 19, wherein $\gamma_1$ is glycine, valine, serine, glutamic acid, proline, asparagine, lysine, cysteine or tryptophan.

**21.** A nucleic acid fragment according to Claim 1, wherein the fragment has a sequence which encodes an amino acid sub-sequence F having the formula

$$G\beta_1\beta_8\beta_2Q\beta_3\beta_4\beta_5\beta_6\beta_7$$

wherein:

| | |
|---|---|
| G | is glycine, |
| $\beta_1$, $\beta_2$, $\beta_3$, $\beta_4$, $\beta_5$, $\beta_6$ and $\beta_7$ | are selected from the group consisting of of amino acids, |
| Q | is glutamine, and |
| $\beta_8$ | is an amino acid other than valine. |

**22.** A nucleic acid fragment according to claim 1 or Claim 21, wherein $\beta_1$ is methionine, $\beta_3$ is tryptophan, $\beta_7$ is phenylalanine and $\beta_8$ is an amino acid other than valine.

**23.** A nucleic acid fragment according to any one of Claims 1, 21 and 22 wherein $\beta_8$ is an amino acid selected from the group consisting of alanine and glycine.

**24.** A nucleic acid fragment according to Claim 23 wherein $\beta_8$ is alanine.

**25.** A nucleic acid fragment according to Claim 1, wherein the fragment has a sequence which encodes an amino acid sub-sequence F having the formula

$$G\beta_1V\beta_2Q\beta_3\beta_4\beta_5\beta_6\beta_7$$

wherein:

| | |
|---|---|
| G | is glycine, |
| $\beta_1$, $\beta_2$, $\beta_4$, $\beta_5$, $\beta_6$ and $\beta_7$ | are selected from the group consisting of amino acids, |
| V | is valine, |
| Q | is glutamine, and |
| $\beta_3$ | is an amino acid other than tryptophan or proline. |

**26.** A nucleic acid fragment according to Claim 1, wherein the fragment has a sequence which encodes an amino acid sub-sequence F having the formula

$$G\beta_1V\beta_2Q\beta_3\beta_4\beta_5\beta_6\beta_7$$

wherein:

| | |
|---|---|
| G | is glycine, |

| | |
|---|---|
| $\beta_1$, $\beta_2$, $\beta_3$, $\beta_4$, $\beta_5$ and $\beta_6$ | are selected from the group consisting of amino acids, |
| V | is valine, |
| Q | is glutamine, and |
| $\beta_7$ | is an amino acid other than phenylalanine |

**27.** A nucleic acid fragment according to claim 1 or claim 26 wherein $\beta_7$ is an amino acid selected from the group consisting of leucine, valine and isoleucine.

**28.** A nucleic acid fragment according to claim 27, wherein $\beta_7$ is leucine.

**29.** A method for preparing a nucleic acid fragment comprising a nucleotide sequence encoding acetolactate synthase, said nucleic acid fragment conferring increased sulfonylurea herbicide resistance on a plant in which said fragment is operative which comprises:

a) isolating from bacteria or yeast a mutant nucleic acid fragment encoding acetolactate synthase which exhibits increased resistance to one or more sulfonylurea herbicides;
b) determining the site(s) of amino acid substitution in said acetolactate synthase which confer(s) resistance on said acetolactate synthase by comparison of the DNA sequence of said mutant nucleic acid fragment with the DNA sequence of a wild type nucleic acid fragment encoding acetolactate synthase which is sensitive to sulfonylurea herbicides; and
c) introducing a mutation in a nucleic acid fragment which encodes acetolactate synthase which has a sequence corresponding at least in part to a plant acetolactate synthase gene and which is operative in a plant, which mutation results in amino acid substitution at site(s) which correspond with said site(s) of amino acid substitution in said acetolactate synthase which exhibits increased resistance to sulfonylurea herbicides.

**30.** A method for preparing a nucleic acid fragment as defined in claim 1, which comprises:

a) selecting a sulfonylurea herbicide-resistant plant mutant; and
b) isolating the mutant acetolactate synthase gene from the mutant plant.

**31.** An acetolactate synthase protein encoded by a nucleic acid fragment as claimed in any one of claims 1 to 28.

**32.** A nucleic acid construct comprising a nucleic acid fragment as claimed in any one of claims 1 to 28.

**33.** A nucleic acid construct as claimed in claim 32, selected from the cloning vectors deposited at the ATCC, Rockville, Maryland, USA under accession numbers 40237, 67124, 67126 and 67424.

**34.** A nucleic acid construct as claimed in claim 32, wherein a nucleic acid fragment as claimed in any one of claims 1 to 28 is linked to a second nucleic acid fragment conferring a second trait such that when said nucleic acid construct is utilized to transform a plant, the expression of herbicide resistance by said plant upon application of sulfonylurea compounds can be utilized to detect the presence of said second nucleic acid fragment in said plant.

**35.** A microorganism containing a nucleic acid fragment or nucleic acid construct as claimed in any one of claims 1 to 28 or 32 to 34.

**36.** A plant cell transformed with a nucleic acid fragment or nucleic acid construct as claimed in any one of claims 1 to 28 or 32 to 34.

**37.** A plant tissue culture transformed with a nucleic acid fragment or nucleic acid construct as claimed in any one of claims 1 to 28 or 32 to 34.

**38.** A method for transforming a plant cell with a nucleic acid fragment as claimed in any one of claims 1 to 28 or prepared by a method as claimed in claims 29 or 30 comprising introducing a nucleic acid construct as claimed in any one of claims 32 to 34 into the plant cell.

**39.** A method for selecting a plant cell transformed with a nucleic acid fragment as claimed in any one of claims 1 to 28 or prepared by a method as claimed in claims 29 or 30 comprising

introducing a nucleic acid construct as claimed in any one of claims 32 to 34 into a plant cell whose growth is sensitive to inhibition by herbicides to which the ALS encoded by the nucleic acid fragment is resistant to form a transformed plant cell; and

selecting a transformed plant cell whose growth is resistant to inhibition by said herbicides at a selective concentration which inhibits the growth of untransformed cells.

**40.** A method for controlling the growth of undesired vegetation growing at a locus where a plant transformed with a nucleic acid fragment as claimed in any one of claims 1 to 28 or prepared by a method as claimed in claims 29 or 30 has been cultivated comprising applying to the locus an effective amount of herbicide to which said plant is resistant.

**41.** A method according to Claim 40 wherein the herbicide is a compound selected from the group consisting of

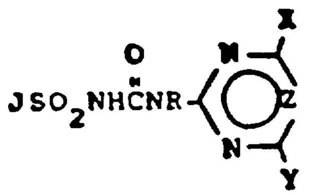

I

wherein

R is H or $CH_3$;

J is

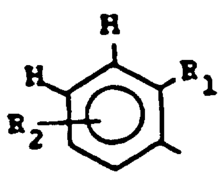 , 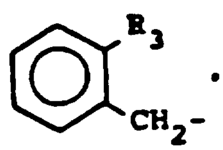 , 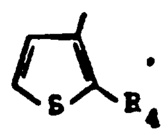

J-1 J-2 J-3

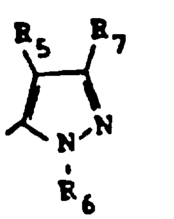 , 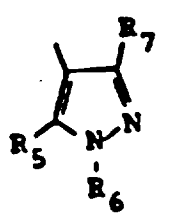 , 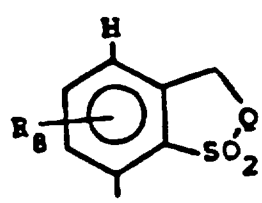

J-4 J-5 J-6

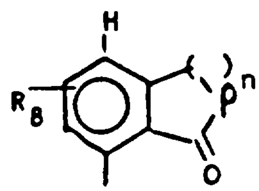 ; 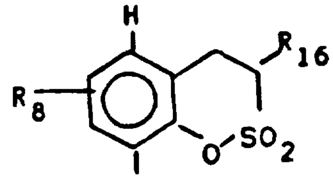 or 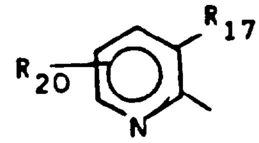 ;

J-7 J-8 J-9

$R_1$ is Cl, Br, $NO_2$, $C_1$-$C_4$ alkyl, $C_2$-$C_4$ alkenyl, $CF_3$, $C_1$-$C_4$ alkoxy, $C_1$-$C_4$ haloalkoxy, $C_3$-$C_4$ alkenyloxy, $C_2$-$C_4$ haloalkenyloxy, $C_3$-$C_4$ alkynyloxy, $CO_2R_9$, $CONR_{10}R_{11}$, $S(O)_mR_{12}$, $OSO_2R_{12}$, phenyl $SO_2N(OCH_3)CH_3$, $SO_2NR_{10}R_{11}$,

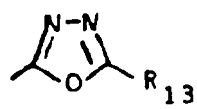 , 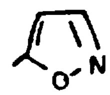 , 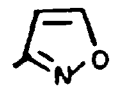 ,

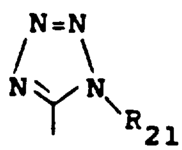 , 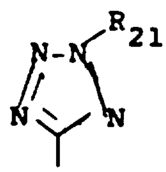 , 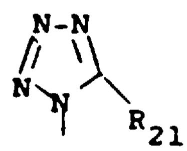 or 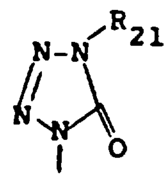

$R_2$ is H, Cl, Br, F, $CH_3$, $NO_2$, $SCH_3$, $OCF_2H$, $OCH_2CF_3$ or $OCH_3$;
$R_3$ is Cl, $NO_2$, $CO_2CH_3$, $CO_2C_2H_5$, $SO_2N(CH_3)_2$, $SO_2CH_3$ or $SO_2C_2H_5$;
$R_4$ is $C_1$-$C_3$ alkyl, Cl, Br, $NO_2$, $CO_2R_9$, $CON(CH_3)_2$, $SO_2N(CH_3)_2$, $SO_2N(OCH_3)CH_3$ or $S(O)_mR_{12}$;
$R_5$ is $C_1$-$C_3$ alkyl, $C_4$-$C_5$ cycloalkylcarbonyl, F, Cl, Br, $NO_2$, $CO_2R_{14}$, $SO_2N(CH_3)_2$, $SO_2R_{12}$ or phenyl;
$R_6$ is H, $C_1$-$C_3$ alkyl, or $CH_2CH{=}CH_2$;
$R_7$ is H, $CH_3$, $OCH_3$, Cl or Br;
$R_8$ is H, F, Cl, Br, $CH_3$, $OCH_3$, $CF_3$, $SCH_3$ or $OCF_2H$;
$R_9$ is $C_1$-$C_4$ alkyl, $C_3$-$C_4$ alkenyl or $CH_2CH_2Cl$;
$R_{10}$ is H or $C_1$-$C_3$ alkyl;
$R_{11}$ is H or $C_1$-$C_2$ alkyl;
$R_{12}$ is $C_1$-$C_3$ alkyl;
$R_{13}$ is H or $CH_3$;
$R_{14}$ is $C_1$-$C_3$ alkyl or $CH_2CH{=}CH_2$;
m is 0, 1 or 2;
n is 1 or 2;
Q is $CH_2$, $CHCH_3$ or $NR_{15}$;
$R_{15}$ is H or $C_1$-$C_4$ alkyl;
P is O or $CH_2$;
$R_{16}$ is H or $CH_3$;
$R_{17}$ is $C(O)NR_{18}R_{19}$;
$R_{18}$ is H or $CH_3$;
$R_{19}$ is $CH_3$;
$R_{20}$ is H, Cl, F, Br, $CH_3$, $CF_3$, $OCH_3$ or $OCF_2H$;
$R_{21}$ is H or $CH_3$;
X is $CH_3$, $OCH_3$, $OC_2H_5$ or $NHCH_3$;
Y is $CH_3$, $C_2H_5$, $OCH_3$, $OC_2H_5$, $OCF_2H$, $OCH_2CF_3$, Cl, $CH_2OCH_3$ or cyclopropyl;
Z is CH or N;

and their agriculturally suitable salts; provided that

a) when Y is Cl, then Z is CH and X is $OCH_3$;
b) when Y is $OCF_2H$, then Z is CH;
c) when J is J-1 and $R_1$ is $OSO_2R_{12}$ or phenyl, then Y is other than $OCF_2H$;
d) when J is J-2, then Y is other than $OCF_2H$ or $OCH_2CF_3$; and
e) when J is J-3 and $R_4$ is $S(O)_mR_{12}$, then Y is other than $OCH_2CF_3$.

**42.** A method according to Claim 40 wherein the herbicide is a compound selected from the group consisting of

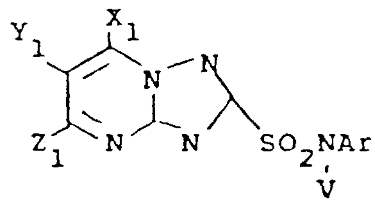

wherein

Ar is

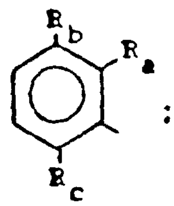

| | |
|---|---|
| $R_a$ | is $C_1$-$C_4$ alkyl, F, Cl, Br, I, $NO_2$, $S(O)_pR_d$, $COOR_e$ or $CF_3$; |
| $R_b$ | is H, F, Cl, Br, I, $C_1$-$C_4$ alkyl or $COOR_e$; |
| $R_c$ | is H, $C_1$-$C_4$ alkyl, F, Cl, Br, I, $CH_2OR_d$, phenyl, $NO_2$ or $COOR_e$: |
| $R_d$ | is $C_1$-$C_4$ alkyl: |
| $R_e$ | is an alkyl, alkenyl, alkynyl group, each having up to 4 carbon atoms or 2-ethoxyethyl; |
| V | is H, $C_1$-$C_3$ alkyl, allyl, propargyl, benzyl or ($C_1$-$C_3$ alkyl)carbonyl; |
| $X_1$, $Y_1$, and $Z_1$, | are independently H, F, Cl, Br, I, $C_1$-$C_4$ alkyl, $C_1$-$C_2$ alkylthio or $C_1$-$C_4$ alkoxyl; and p is 0, 1 or 2. |

**43.** A method according to Claim 40. wherein the herbicide is a compound selected from the group consisting of

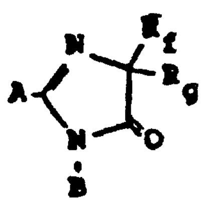

III

wherein

A is

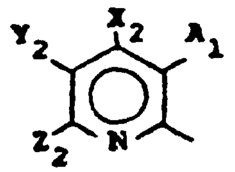
,
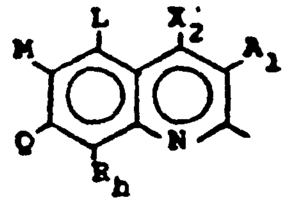

or

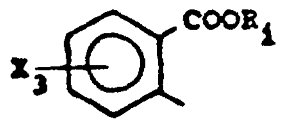
;

| | |
|---|---|
| $R_f$ | is $C_1$-$C_4$ alkyl; |
| $R_g$ | is $C_1$-$C_4$ alkyl or $C_3$-$C_6$ cycloalkyl; |
| $A_1$ | is $COOR_i$, $CH_2OH$ or CHO; |
| $R_i$ | is H; $C_1$-$C_{12}$ alkyl optionally substituted by $C_1$-$C_3$ alkyl, $C_3$-$C_6$ cycloalkyl or phenyl; $C_3$-$C_5$ alkenyl optionally substituted by phenyl or 1-2 $C_1$-$C_3$ alkyl, F, Cl, Br or I; or $C_3$-$C_5$ alkynyl optionally substituted by phenyl or 1-2 $C_1$-$C_3$ alkyl, F, Cl, Br or I; |
| B | is H; C(O)$C_1$-$C_6$ alkyl or C(O)phenyl optionally substituted by Cl, $NO_2$ or $OCH_3$; |
| $X_2$ | is H, F, Cl, Br, I, OH or $CH_3$; |
| $Y_2$ and $Z_2$ | are independently H, $C_1$-$C_6$ alkyl, $C_1$-$C_6$ alkoxy, F, Cl, Br, I, Phenyl, $NO_2$, CN, $CF_3$ or $SO_2CH_3$; |
| $X_3$ | is H, $C_1$-$C_3$ alkyl, F, Cl, Br, I or $NO_2$; and |
| L, M, Q and $R_h$ | are independently H, F, Cl, Br, I, $CH_3$, $OCH_3$, $NO_2$, $CF_3$, CN, $N(CH_3)_2$, $NH_2$, $SCH_3$ or $SO_2CH_3$ provided that only one of M or Q may be a substituent other than H, F, Cl, Br, I, $CH_3$ or $OCH_3$. |

**44.** A method for preparing a plant containing a nucleic acid fragment as defined in any one of claims 1 to 28 or prepared by a method as claimed in claim 29 or claim 30 which comprises transforming a plant cell with a nucleic acid fragment or nucleic acid construct as defined in any one of claims 1 to 28 or 32 to 34 or prepared by a method as claimed in claim 29 or claim 30, regenerating transformed plants from the transformed plant cell and optionally biologically replicating the transformed plant.

**45.** A method as claimed in claim 44 wherein the plant is a soybean, maize, sugarbeet, sunflower, tobacco, potato, tomato, alfalfa, melon, cotton, lettuce, wheat or rice plant or a Brassica species.

**46.** A method for preparing seed containing a nucleic acid fragment as defined in any one of claims 1 to 28 or prepared by a method as claimed in claim 29 or claim 30 which comprises transforming a plant cell with a nucleic acid fragment or nucleic acid construct as defined in any one of claims 1 to 28 or 32 to 34 or prepared by a method as claimed in claim 29 or claim 30, regenerating transformed plants from the transformed plant cell, optionally biologically replicating the transformed plants and obtaining seed from the transformed plants.

**47.** A plant containing a nucleic acid fragment or nucleic acid construct as claimed in any one of claims 1 to 28 or 32 to 34.

**48.** A plant as claimed in claim 47 which is a soybean, maize, sugarbeet, sunflower, tobacco, potato, tomato, alfalfa, melon, cotton, lettuce, wheat or rice plant or a Brassica species.

**49.** Seed containing a nucleic acid fragment or nucleic acid construct as claimed in any one of claims 1 to 28 or 32 to 34 obtained by growing a plant as claimed in claim 47 or claim 48.

**Claims for the following Contracting States : AT, ES**

**1.** A method of preparing a nucleic acid fragment comprising a nucleotide sequence encoding acetolactase synthase,

said nucleic acid fragment conferring increased sulfonylurea herbicide resistance on a plant in which said fragment is operative, wherein a nucleotide sequence which encodes acetolactate synthase which has a sequence corresponding at least in part to a plant acetolactate synthase gene and is operative in a plant, and which comprises at least one sub-sequence which encodes one of the substantially conserved amino acid sub-sequences designated A,B,C,D,E,F and G in Figure 6, is mutated such that at least one of the following conditions is met:

a) the nucleic acid fragment has a sequence which encodes an amino acid sub-sequence A wherein $\varepsilon_1$ is an amino acid other than alanine, and/or $\varepsilon_2$ is an amino acid other than glycine,
b) the nucleic acid fragment has a sequence which encodes an amino acid sub-sequence B wherein $\alpha_1$ is an amino acid other than proline,
c) the nucleic acid fragment has a sequence which encodes an amino acid sub-sequence C wherein $\delta_2$ is an amino acid other than alanine,
d) the nucleic acid fragment has a sequence which encodes an amino acid sub-sequence D wherein $\lambda_1$ is an amino acid other than lysine,
e) the nucleic acid fragment has a sequence which encodes an amino acid sub-sequence E wherein $\gamma_1$ is an amino acid other than aspartic acid,
f) the nucleic acid fragment has a sequence which encodes an amino acid sub-sequence F wherein $\beta_3$ is an amino acid other than tryptophan, and/or $\beta_8$ is an amino acid other than valine and/or $\beta_7$ is an amino acid other than phenylalanine, and
g) the nucleic acid fragment has a sequence which encodes an amino acid sub-sequence G wherein $\sigma_1$ is an amino acid other than methionine.

**2.** A method according to Claim 1. wherein the fragment has a sequence which encodes an amino acid sub-sequence A having the formula

$$PG\varepsilon_2A$$

wherein:

P is proline,
G is glycine,
$\varepsilon_2$ is an amino acid other than glycine, and
A is alanine.

**3.** A method according to claim 1 or Claim 2, wherein $\varepsilon_2$ is an amino acid selected from the group consisting of serine, threonine or cysteine.

**4.** A method according to Claim 3, wherein $\varepsilon_2$ is serine.

**5.** A method according to Claim 1, wherein the fragment has a sequence which encodes an amino acid sub-sequence A having the formula

$$PGG\varepsilon_1$$

wherein:

P is proline,
G is glycine, and
$\varepsilon_1$ is an amino acid other than alanine or glycine.

**6.** A method according to Claim 1, wherein the fragment has a sequence which encodes an amino acid sub-sequence B having the formula

$$GQV\alpha_1$$

wherein:

G is glycine,
Q is glutamine,
V is valine
$\alpha_1$ is an amino acid other than proline.

**7.** A method according to claim 1 or Claim 6, wherein $\alpha_1$ is an amino acid selected from the group consisting of alanine, glycine, arginine, lysine, histidine, serine, cysteine. threonine, glutamine and asparagine.

**8.** A method according to Claim 7, wherein $\alpha_1$ is alanine, arginine, serine or glutamine.

**9.** A method according to Claim 1, wherein the fragment has a sequence which encodes an amino acid sub-sequence C having the formula

$$IG\delta_1D\delta_2FQE$$

wherein:

I is isoleucine,
G is glycine,
$\delta_1$ is selected from the group consisting of amino acids,
D is aspartic acid, and
$\delta_2$ is an amino acid other than alanine.
F is phenylalanine
Q is glutamine
E is glutamic acid

**10.** A method according to claim 1 or Claim 9 wherein $\delta_2$ is an amino acid selected from the group consisting of threonine, serine, cysteine, tyrosine, aspartic acid, glutamic acid, tryptophan, histidine, phenylalanine, arginine, and lysine.

**11.** A method according to Claim 10, wherein $\delta_2$ is threonine, cysteine, aspartic acid, glutamic acid, tryptophan or arginine.

**12.** A method according to Claim 1, wherein the fragment has a sequence which encodes an amino acid sub-sequence D having the formula

$$P\lambda_1D$$

wherein:

P is proline;
$\lambda_1$ is an amino acid other than lysine; and
D is aspartic acid.

**13.** A method according to claim 1 or Claim 12, wherein $\lambda_1$ is an amino acid selected from the group consisting of glycine, alanine, leucine, isoleucine, valine, threonine, serine, cysteine, tyrosine, glutamic acid, aspartic acid, proline, asparagine, glutamine, tryptophan and phenylalanine.

**14.** A method according to Claim 13. wherein $\lambda_1$ is glycine, leucine. threonine, glutamic acid, aspartic acid, asparagine, tryptophan or proline.

**15.** A method according to Claim 1, wherein the fragment has a sequence which encodes an amino acid sub-sequence G having the formula

$$MLG\sigma_1HG$$

wherein

- M is methionine,
- L is leucine,
- G is glycine
- $\sigma_1$ is an amino acid other than methionine,
- H is histidine, and
- G is glycine.

**16.** A method according to claim 1 or Claim 15, wherein $\sigma_1$ is an amino acid selected from the group consisting of glutamic acid, aspartic acid, proline, valine, leucine, or isoleucine.

**17.** A method according to Claim 16, wherein $\sigma_1$ is proline, glutamic acid. or valine.

**18.** A method according to Claim 1, wherein the fragment has a sequence which encodes an amino acid sub-sequence E having the formula

$$RFD\gamma_1R\ .$$

wherein:

- R is arginine,
- F is phenylalanine,
- D is aspartic acid, and
- $\gamma_1$ is an amino acid other than aspartic acid.

**19.** A method according to claim 1 or Claim 18, wherein $\gamma_1$ is an amino acid selected from the group consisting of glycine, alanine, valine. leucine, isoleucine, serine, threonine, cysteine, tyrosine, glutamic acid, proline, asparagine, glutamine, lysine, arginine, tryptophan, histidine, and phenylalanine.

**20.** A method according to Claim 19. wherein $\gamma_1$ is glycine, valine, serine. glutamic acid, proline, asparagine, lysine, cysteine or tryptophan.

**21.** A method according to Claim 1, wherein the fragment has a sequence which encodes an amino acid sub-sequence F having the formula

$$G\beta_1\beta_8\beta_2Q\beta_3\beta_4\beta_5\beta_6\beta_7$$

wherein:

| | |
|---|---|
| G | is glycine, |
| $\beta_1$, $\beta_2$, $\beta_3$, $\beta_4$, $\beta_5$, $\beta_6$ and $\beta_7$ | are selected from the group consisting of of amino acids, |
| Q | is glutamine, and |
| $\beta_8$ | is an amino acid other than valine. |

**22.** A method according to claim 1 or Claim 21, wherein $\beta_1$ is methionine, $\beta_3$ is tryptophan, $\beta_7$ is phenylalanine and $\beta_8$ is an amino acid other than valine.

**23.** A method according to any one of Claims 1, 21 and 22 wherein $\beta_8$ is an amino acid selected from the group consisting of alanine and glycine.

**24.** A method according to Claim 23 wherein $\beta_8$ is alanine.

**25.** A method according to Claim 1, wherein the fragment has a sequence which includes an amino acid sub-sequence F having the formula

$$G\beta_1V\beta_2Q\beta_3\beta_4\beta_5\beta_6\beta_7$$

wherein:

| | |
|---|---|
| G | is glycine, |
| $\beta_1$, $\beta_2$, $\beta_4$, $\beta_5$, $\beta_6$ and $\beta_7$ | are selected from the group consisting of amino acids, |
| V | is valine, |
| Q | is glutamine, and |
| $\beta_3$ | is an amino acid other than tryptophan or proline. |

**26.** A method according to Claim 1, wherein the fragment has a sequence which includes an amino acid sub-sequence F having the formula

$$G\beta_1V\beta_2Q\beta_3\beta_4\beta_5\beta_6\beta_7$$

wherein:

| | |
|---|---|
| G | is glycine, |
| $\beta_1$, $\beta_2$, $\beta_3$, $\beta_4$, $\beta_5$ and $\beta_6$ | are selected from the group consisting of amino acids, |
| V | is valine, |
| Q | is glutamine, and |
| $\beta_7$ | is an amino acid other than phenylalanine. |

**27.** A method according to claim 1 or claim 26 wherein $\beta_7$ is an amino acid selected from the group consisting of leucine, valine and isoleucine.

**28.** A method according to claim 27 wherein $\beta_7$ is leucine.

**29.** A method for preparing a nucleic acid fragment comprising a nucleotide sequence encoding acetolactate synthase, said nucleic acid fragment conferring increased sulfonylurea herbicide resistance on a plant in which said fragment is operative which comprises:

a) isolating from bacteria or yeast a mutant nucleic acid fragment encoding acetolactate synthase which exhibits increased resistance to one or more sulfonylurea herbicides;
b) determining the site(s) of amino acid substitution in said acetolactate synthase which confer(s) resistance on said acetolactate synthase by comparison of the DNA sequence of said mutant nucleic acid fragment with the DNA sequence of a wild type nucleic acid fragment encoding acetolactate synthase which is sensitive to sulfonylurea herbicides; and
c) introducing a mutation into a nucleic acid fragment which encodes acetolactate synthase which has a sequence corresponding at least in part to a plant acetolactate synthase gene and which is operative in a plant, which mutation results in amino acid substitution at site(s) which correspond with said site(s) of amino acid substitution in said acetolactate synthase which exhibits increased resistance to sulfonylurea herbicides.

**30.** A method for preparing a nucleic acid fragment as defined in claim 1, which comprises:

a) selecting a sulfonylurea herbicide-resistant plant mutant; and
b) isolating the mutant acetolactate synthase gene from the mutant plant.

**31.** A method of preparing a nucleic acid construct comprising a nucleic acid fragment prepared by a method as claimed in any of claims 1 to 30 which comprises linking said nucleic acid fragment to a second nucleic acid fragment.

**32.** A method as claimed in claim 31 wherein said second nucleic acid fragment confers a second trait such that, when said nucleic acid construct is utilized to transform a plant, the expression of herbicide resistance by said plant upon

application of sulfonylurea compounds can be utilized to detect the presence of said second nucleic acid fragment in said plant.

**33.** A nucleic acid construct selected from the cloning vectors deposited at the ATCC, Rockville, Maryland, U.S.A. under accession numbers 40237, 67124, 67126 and 67424.

**34.** A method for preparing a plant acetolactate synthase with increased resistance to one or more sulfonylurea herbicides, which comprises expressing a plant acetolactate synthase-encoding sequence within a nucleic acid fragment or nucleic acid construct as defined in any one of claims 1 to 28 or 31 to 33 or prepared by a method as claimed in claim 29 or 30.

**35.** A microorganism containing a nucleic acid fragment or nucleic acid construct as defined in any one of claims 1 to 28 or 31 to 33.

**36.** A plant cell transformed with a nucleic acid fragment or nucleic acid construct as defined in any one of claims 1 to 28 or 31 to 33.

**37.** A plant tissue culture transformed with a nucleic acid fragment or nucleic acid construct as defined in any one of claims 1 to 28 or 31 to 33.

**38.** A method for transforming a plant cell with a nucleic acid fragment as defined in any one of claims 1 to 28 or prepared by a method as claimed in claim 29 or 30 comprising introducing a nucleic acid construct as defined in any one of claims 31 to 33 into the plant cell.

**39.** A method for selecting a plant cell transformed with a nucleic acid fragment as defined in any one of claims 1 to 28 or prepared by a method as claimed in claim 29 or 30 comprising

introducing a nucleic acid construct as defined in any one of claims 31 to 33 into a plant cell whose growth is sensitive to inhibition by herbicides to which the ALS encoded by the nucleic acid fragment is resistant to form a transformed plant cell; and

selecting a transformed plant cell whose growth is resistant to inhibition by said herbicides at a selective concentration which inhibits the growth of untransformed cells.

**40.** A method for controlling the growth of undesired vegetation growing at a locus where a plant transformed with a nucleic acid fragment as defined in any one of claims 1 to 28 or prepared by a method as claimed in claim 29 or 30 has been cultivated comprising applying to the locus an effective amount of herbicide to which said plant is resistant.

**41.** A method according to Claim 40 wherein the herbicide is a compound selected from the group consisting of

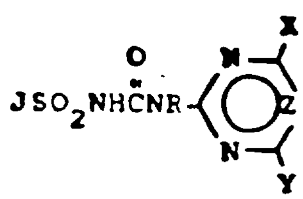

I

wherein

R is H or $CH_3$;

J is

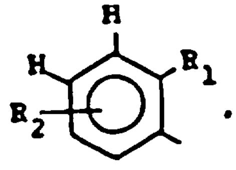 , 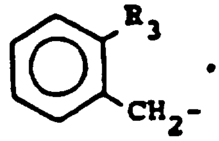 , 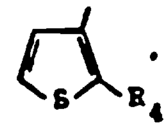 ,

J-1 J-2 J-3

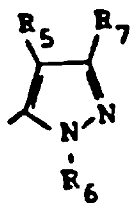 , 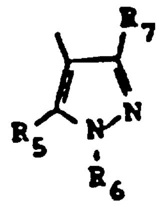 , 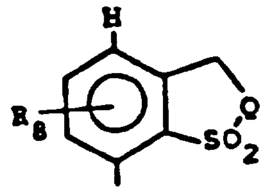

J-4 J-5 J-6

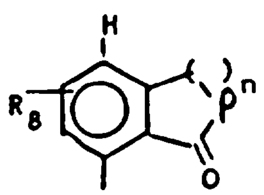 ; 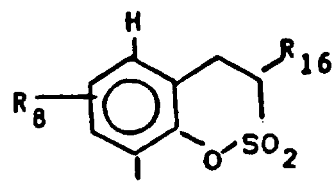 or 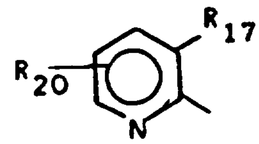 ;

J-7 J-8 J-9

$R_1$ is Cl, Br, $NO_2$, $C_1$-$C_4$ alkyl, $C_2$-$C_4$ alkenyl, $CF_3$, $C_1$-$C_4$ alkoxy, $C_1$-$C_4$ haloalkoxy, $C_3$-$C_4$ alkenyloxy, $C_2$-$C_4$ haloalkenyloxy, $C_3$-$C_4$ alkynyloxy, $CO_2R_9$, $CONR_{10}R_{11}$, $S(O)_mR_{12}$, $OSO_2R_{12}$, phenyl $SO_2N(OCH_3)CH_3$, $SO_2NR_{10}R_{11}$,

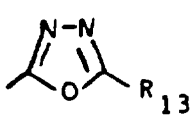 , 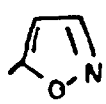 , 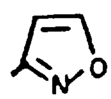 ,

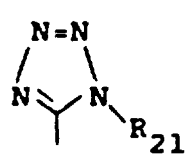 , 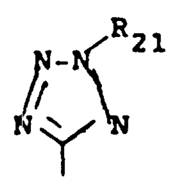 , 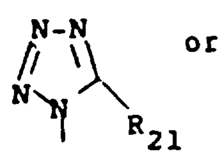 or 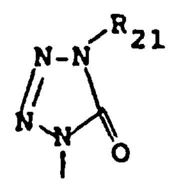

$R_2$ is H, Cl, Br, F, $CH_3$, $NO_2$, $SCH_3$, $OCF_2H$, $OCH_2CF_3$ or $OCH_3$;
$R_3$ is Cl, $NO_2$, $CO_2CH_3$, $CO_2C_2H_5$, $SO_2N(CH_3)_2$, $SO_2CH_3$ or $SO_2C_2H_5$;
$R_4$ is $C_1$-$C_3$ alkyl, Cl, Br, $NO_2$, $CO_2R_9$, $CON(CH_3)_2$, $SO_2N(CH_3)_2$, $SO_2N(OCH_3)CH_3$ or $S(O)_mR_{12}$;

$R_5$ is $C_1$-$C_3$ alkyl, $C_4$-$C_5$ cycloalkylcarbonyl, F, Cl, Br, $NO_2$, $CO_2R_{14}$, $SO_2N(CH_3)_2$ $SO_2R_{12}$ or phenyl;
$R_6$ is H, $C_1$-$C_3$ alkyl, or $CH_2CH{=}CH_2$;
$R_7$ is H, $CH_3$, $OCH_3$, Cl or Br;
$R_8$ is H, F, Cl, Br, $CH_3$, $OCH_3$, $CF_3$, $SCH_3$ or $OCF_2H$;
$R_9$ is $C_1$-$C_4$ alkyl, $C_3$-$C_4$ alkenyl or $CH_2CH_2Cl$;
$R_{10}$ is H or $C_1$-$C_3$ alkyl;
$R_{11}$ is H or $C_1$-$C_2$ alkyl;
$R_{12}$ is $C_1$-$C_3$ alkyl;
$R_{13}$ is H or $CH_3$;
$R_{14}$ is $C_1$-$C_3$ alkyl or $CH_2CH{=}CH_2$;
m is 0, 1 or 2;
n is 1 or 2;
Q is $CH_2$, $CHCH_3$ or $NR_{15}$;
$R_{15}$ is H or $C_1$-$C_4$ alkyl;
P is O or $CH_2$;
$R_{16}$ is H or $CH_3$;
$R_{17}$ is $C(O)NR_{18}R_{19}$;
$R_{18}$ is H or $CH_3$;
$R_{19}$ is $CH_3$;
$R_{20}$ is H, Cl, F, Br, $CH_3$, $CF_3$, $OCH_3$ or $OCF_2H$;
$R_{21}$ is H or $CH_3$;
X is $CH_3$, $OCH_3$, $OC_2H_5$ or $NHCH_3$;
Y is $CH_3$, $C_2H_5$, $OCH_3$, $OC_2H_5$, $OCF_2H$, $OCH_2CF_3$, Cl, $CH_2OCH_3$ or cyclopropyl;
Z is CH or N;

and their agriculturally suitable salts; provided that

a) when Y is Cl, then Z is CH and X is $OCH_3$;
b) when Y is $OCF_2H$, then Z is CH;
c) when J is J-1 and $R_1$ is $OSO_2R_{12}$ or phenyl, then Y is other than $OCF_2H$;
d) when J is J-2, then Y is other than $OCF_2H$ or $OCH_2CF_3$; and
e) when J is J-3 and $R_4$ is $S(O)_mR_{12}$, then Y is other than $OCH_2CF_3$,

**42.** A method according to Claim 40 wherein the herbicide is a compound selected from the group consisting of

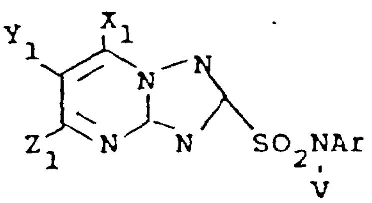

wherein

Ar is

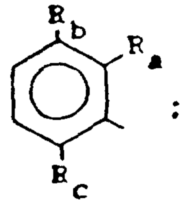

$R_a$ is $C_1$-$C_4$ alkyl, F, Cl, Br, I, $NO_2$, $S(O)_pR_d$, $COOR_e$ or $CF_3$;

$R_b$ is H, F, Cl, Br, I, $C_1$-$C_4$ alkyl or $COOR_e$;
$R_c$ is H, $C_1$-$C_4$ alkyl, F, Cl, Br, I, $CH_2OR_d$, phenyl, $NO_2$ or $COOR_e$;
$R_d$ is $C_1$-$C_4$ alkyl;
$R_e$ is an alkyl, alkenyl, alkynyl group, each having up to 4 carbon atoms or 2-ethoxyethyl;
V is H, $C_1$-$C_3$ alkyl, allyl, propargyl, benzyl or ($C_1$-$C_3$ alkyl)carbonyl;
$X_1$, $Y_1$, and $Z_1$, are independently H, F, Cl, Br, I, $C_1$-$C_4$ alkyl, $C_1$-$C_2$ alkylthio or $C_1$-$C_4$ alkoxy; and
p is 0, 1 or 2.

**43.** A method according to Claim 40 wherein the herbicide is a compound selected from the group consisting of

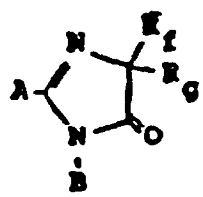

III

wherein

A is

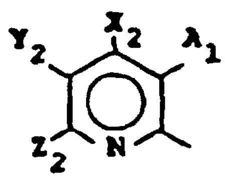

.

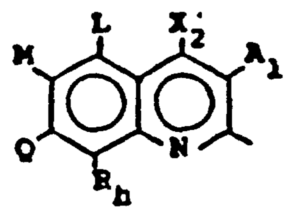

or

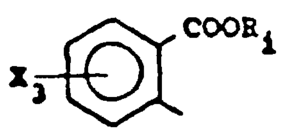

;

$R_f$ is $C_1$-$C_4$ alkyl;
$R_g$ is $C_1$-$C_4$ alkyl or $C_3$-$C_6$ cycloalkyl;
$A_1$ is $COOR_i$, $CH_2OH$ or CHO;
$R_i$ is H; $C_1$-$C_{12}$ alkyl optionally substituted by $C_1$-$C_3$ alkyl, $C_3$-$C_6$ cycloalkyl or phenyl; $C_3$-$C_5$ alkenyl optionally substituted by phenyl or 1-2 $C_1$-$C_3$ alkyl, F, Cl, Br or I; or $C_3$-$C_5$ alkynyl optionally substituted by phenyl or 1-2 $C_1$-$C_3$ alkyl, F, Cl, Br or I;
B is H; $C(O)C_1$-$C_6$ alkyl or C(O)phenyl optionally substituted by Cl, $NO_2$ or $OCH_3$;
$X_2$ is H, F, Cl, Br, I, OH or $CH_3$;
$Y_2$ and $Z_2$ are independently H, $C_1$-$C_6$ alkyl, $C_1$-$C_6$ alkoxy, F, Cl, Br, I, phenyl, $NO_2$, CN, $CF_3$ or $SO_2CH_3$;
$X_3$ is H, $C_1$-$C_3$ alkyl, F, Cl, Br, I or $NO_2$; and
L, M, Q and $R_h$ are independently H, F, Cl, Br, I, $CH_3$, $OCH_3$, $NO_2$, $CF_3$, CN, $N(CH_3)_2$, $NH_2$, $SCH_3$ or $SO_2CH_3$ provided that only one of M or Q may be a substituent other than H, F, Cl, Br, I, $CH_3$ or $OCH_3$.

**44.** A method for preparing a plant containing a nucleic acid fragment as defined in any one of claims 1 to 28 or prepared by a method as claimed in claim 29 or claim 30 which comprises transforming a plant cell with a nucleic acid fragment or nucleic acid construct as defined in any one of claims 1 to 28 or 31 to 33 or prepared by a method

as claimed in claim 29 or claim 30, regenerating transformed plants from the transformed plant cell and optionally biologically replicating the transformed plants.

**45.** A method as claimed in claim 44 wherein the plant is a soybean, maize, sugarbeet, sunflower, tobacco, potato, tomato, alfalfa, melon, cotton, lettuce, wheat or rice plant or a Brassica species.

**46.** A method for preparing seed containing a nucleic acid fragment as defined in any one of claims 1 to 28 or prepared by a method as claimed in claim 29 or claim 30 which comprises transforming a plant cell with a nucleic acid fragment or nucleic acid construct as defined in any one of claims 1 to 28 or 31 to 33 or prepared by a method as claimed in claim 29 or claim 30, regenerating transformed plants from the transformed plant cell, optionally biologically replicating the transformed plants and obtaining seed from the transformed plants.

**47.** A plant containing a nucleic acid fragment or nucleic acid construct as defined in any one of claims 1 to 28 or 31 to 33.

**48.** A plant as claimed in claim 47 which is a soybean, maize, sugarbeet, sunflower, tobacco, potato, tomato, alfalfa, melon, cotton, lettuce, wheat or rice plant or a Brassica species.

**49.** Seed containing a nucleic acid fragment or nucleic acid construct as defined in any one of claims 1 to 28 or 31 to 33 obtained by growing a plant as claimed in claim 47 or claim 48.

**Patentansprüche**

**Patentansprüche für folgende Vertragsstaaten : DE, GB, FR, IT, NL, SE, CH, LI, BE, LU, GR**

**1.** Nukleinsäurefragment, umfassend eine für Acetolaktatsynthase kodierende Nukleotidsequenz, wobei die Nukleotidsequenz mindestens teilweise einem Pflanzen-Acetolaktatsynthase-Gen entspricht, das einer Pflanze, in der das Fragment funktionsfähig ist, erhöhte Sulfonylharnstoff-Herbizidresistenz verleiht, wobei diese Nukleotidsequenz mindestens eine Subsequenz umfaßt, die für eine der im wesentlichen konservierten Aminosäure-Subsequenzen mit den Bezeichnungen A, B, C, D, E, F und G in Fig. 6 kodiert, welches Nukleinsäurefragment weiter dadurch gekennzeichnet ist, daß mindestens eine der folgenden Bedingungen erfüllt ist;

a) das Nukleinsäurefragment weist eine Sequenz auf, die für eine Aminosäure-Subsequenz A kodiert, worin $\varepsilon_1$ eine andere Aminosäure als Alanin ist und/oder $\varepsilon_2$ eine andere Aminosäure als Glycin ist,
b) das Nukleinsäurefragment weist eine Sequenz auf, die für eine Aminosäure-Subsequenz B kodiert, worin $\alpha_1$ eine andere Aminosäure als Prolin ist,
c) das Nukleinsäurefragment weist eine Sequenz auf, die für eine Aminosäure-Subsequenz C kodiert, worin $\delta_2$ eine andere Aminosäure als Alanin ist,
d) das Nukleinsäurefragment weist eine Sequenz auf, die für eine Aminosäure-Subsequenz D kodiert, worin $\lambda_1$ eine andere Aminosäure als Lysin ist,
e) das Nukleinsäurefragment weist eine Sequenz auf, die für eine Aminosäure-Subsequenz E kodiert, worin $\gamma_1$ eine andere Aminosäure als Asparaginsäure ist,
f) das Nukleinsäurefragment weist eine Sequenz auf, die für eine Aminosäure-Subsequenz F kodiert, worin $\beta_3$ eine andere Aminosäure als Tryptophan ist und/oder $\beta_8$ eine andere Aminosäure als Valin ist und/oder $\beta_7$ eine andere Aminosäure als Phenylalanin ist, und
g) das Nukleinsäurefragment weist eine Sequenz auf, die für eine Aminosäure-Subsequenz G kodiert, worin $\sigma_1$ eine andere Aminosäure als Methionin ist.

**2.** Nukleinsäurefragment nach Anspruch 1, worin das Fragment eine Sequenz aufweist, die für eine Aminosäure-Subsequenz A mit der Formel

$$PG\varepsilon_2A$$

kodiert, worin

P Prolin ist,
G Glycin ist,
$\varepsilon_2$ eine andere Aminosäure als Glycin ist, und
A Alanin ist.

3. Nukleinsäurefragment nach Anspruch 1 oder Anspruch 2, worin $\varepsilon_2$ eine Aminosäure ist, die aus der Gruppe aus Serin, Threonin oder Cystein ausgewählt ist.

4. Nukleinsäurefragment nach Anspruch 3, worin $\varepsilon_2$ Serin ist.

5. Nukleinsäurefragment nach Anspruch 1, worin das Fragment eine Sequenz aufweist, die für eine Aminosäure-Subsequenz A mit der Formel

$$PGG\varepsilon_1$$

kodiert, worin

P Prolin ist,
G Glycin ist, und
$\varepsilon_1$ eine andere Aminosäure als Alanin oder Glycin ist.

6. Nukleinsäurefragment nach Anspruch 1, worin das Fragment eine Sequenz aufweist, die für eine Aminosäure-Subsequenz B mit der Formel

$$GQV\alpha_1$$

kodiert, worin

G Glycin ist,
Q Glutamin ist
V Valin ist,
$\alpha_1$ eine andere Aminosäure als Prolin ist.

7. Nukleinsäurefragment nach Anspruch 1 oder Anspruch 6, worin $\alpha_1$ eine Aminosäure ist, die aus der Gruppe aus Alanin, Glycin, Arginin, Lysin, Histidin, Serin, Cystein, Threonin, Glutamin und Asparagin ausgewählt ist.

8. Nukleinsäurefragment nach Anspruch 7, worin $\alpha_1$ Alanin, Arginin, Serin oder Glutamin ist.

9. Nukleinsäurefragment nach Anspruch 1, worin das Fragment eine Sequenz aufweist, die für eine Aminosäure-Subsequenz C mit der Formel

$$IG\delta_1D\delta_2FQE$$

kodiert, worin

I Isoleucin ist,
G Glycin ist,
$\delta_1$ aus der Gruppe aus Aminosäuren ausgewählt ist,
D Asparaginsäure ist, und
$\delta_2$ eine andere Aminosäure als Alanin ist,
F Phenylalanin ist,
Q Glutamin ist, und
E Glutaminsäure ist.

10. Nukleinsäurefragment nach Anspruch 1 oder Anspruch 9, worin $\delta_2$ eine Aminosäure ist, die aus der Gruppe aus

Threonin, Serin, Cystein, Tyrosin, Asparaginsäure, Glutaminsäure, Tryptophan, Histidin, Phenylalanin, Arginin und Lysin ausgewählt ist.

**11.** Nukleinsäurefragment nach Anspruch 10, worin $\delta_2$ Threonin, Cystein, Asparaginsäure, Glutaminsäure, Tryptophan oder Arginin ist.

**12.** Nukleinsäurefragment nach Anspruch 1, worin das Fragment eine Sequenz aufweist, die für eine Aminosäure-Subsequenz D mit der Formel

$$P\lambda_1 D$$

kodiert, worin

P Prolin ist;
$\lambda_1$ eine andere Aminosäure als Lysin ist; und
D Asparaginsäure ist.

**13.** Nukleinsäurefragment nach Anspruch 1 oder Anspruch 12, worin $\lambda_1$ eine Aminosäure ist, die aus der Gruppe aus Glycin, Alanin, Leucin, Isoleucin, Valin, Threonin, Serin, Cystein, Tyrosin, Glutaminsäure, Asparaginsäure, Prolin, Asparagin, Glutamin, Tryptophan und Phenylalanin ausgewählt ist.

**14.** Nukleinsäurefragment nach Anspruch 13, worin $\lambda_1$ Glycin, Leucin, Threonin, Glutaminsäure, Asparaginsäure, Asparagin, Tryptophan oder Prolin ist.

**15.** Nukleinsäurefragment nach Anspruch 1, worin das Fragment eine Sequenz aufweist, die für eine Aminosäure-Subsequenz G mit der Formel

$$MLG\sigma_1 HG$$

kodiert, worin

M Methionin ist,
L Leucin ist,
G Glycin ist,
$\sigma_1$ eine andere Aminosäure als Methionin ist,
H Histidin ist, und
G Glycin ist.

**16.** Nukleinsäurefragment nach Anspruch 1 oder Anspruch 15, worin $\sigma_1$ eine Aminosäure ist, die aus der Gruppe aus Glutaminsäure, Asparaginsäure, Prolin, Valin, Leucin oder Isoleucin ausgewählt ist.

**17.** Nukleinsäurefragment nach Anspruch 16, worin $\sigma_1$ Prolin, Glutaminsäure oder Valin ist.

**18.** Nukleinsäurefragment nach Anspruch 1, worin das Fragment eine Sequenz aufweist, die für eine Aminosäure-Subsequenz E mit der Formel

$$RFD\gamma_1 R$$

kodiert, worin

R Arginin ist,
F Phenylalanin ist,
D Asparaginsäure ist, und
$\gamma_1$ eine andere Aminosäure als Asparaginsäure ist.

**19.** Nukleinsäurefragment nach Anspruch 1 oder Anspruch 18, worin $\gamma_1$ eine Aminosäure ist, die aus der Gruppe aus Glycin, Alanin, Valin, Leucin, Isoleucin, Serin, Threonin, Cystein, Tyrosin, Glutaminsäure, Prolin, Asparagin, Glutamin, Lysin, Arginin, Tryptophan, Histidin und Phenylalanin ausgewählt ist.

**20.** Nukleinsäurefragment nach Anspruch 19, worin $\gamma_1$ Glycin, Valin, Serin, Glutaminsäure, Prolin, Asparagin, Lysin, Cystein oder Tryptophan ist.

**21.** Nukleinsäurefragment nach Anspruch 1, worin das Fragment eine Sequenz aufweist, die für eine Aminosäure-Subsequenz F mit der Formel

$$G\beta_1\beta_8\beta_2Q\beta_3\beta_4\beta_5\beta_6\beta_7$$

kodiert, worin

| | |
|---|---|
| G | Glycin ist, |
| $\beta_1$, $\beta_2$, $\beta_3$, $\beta_4$, $\beta_5$, $\beta_6$ und $\beta_7$ | aus der Gruppe aus Aminosäuren ausgewählt sind, |
| Q | Glutamin ist, und |
| $\beta_8$ | eine andere Aminosäure als Valin ist. |

**22.** Nukleinsäurefragment nach Anspruch 1 oder Anspruch 21, worin $\beta_1$ Methionin ist, $\beta_3$ Tryptophan ist, $\beta_7$ Phenylalanin ist und $\beta_8$ eine andere Aminosäure als Valin ist.

**23.** Nukleinsäurefragment nach irgendeinem der Ansprüche 1, 21 und 22, worin $\beta_8$ eine Aminosäure ist, die aus der Gruppe aus Alanin und Glycin ausgewählt ist.

**24.** Nukleinsäurefragment nach Anspruch 23, worin $\beta_8$ Alanin ist.

**25.** Nukleinsäurefragment nach Anspruch 1, worin das Fragment eine Sequenz aufweist, die für eine Aminosäure-Subsequenz F mit der Formel

$$G\beta_1V\beta_2Q\beta_3\beta_4\beta_5\beta_6\beta_7$$

kodiert, worin

| | |
|---|---|
| G | Glycin ist, |
| $\beta_1$, $\beta_2$, $\beta_4$, $\beta_5$, $\beta_6$ und $\beta_7$ | aus der Gruppe aus Aminosäuren ausgewählt sind, |
| V | Valin ist, |
| Q | Glutamin ist, und |
| $\beta_3$ | eine andere Aminosäure als Tryptophan oder Prolin ist. |

**26.** Nukleinsäurefragment nach Anspruch 1, worin das Fragment eine Sequenz aufweist, die für eine Aminosäure-Subsequenz F mit der Formel

$$G\beta_1V\beta_2Q\beta_3\beta_4\beta_5\beta_6\beta_7$$

kodiert, worin

| | |
|---|---|
| G | Glycin ist, |
| $\beta_1$, $\beta_2$, $\beta_3$, $\beta_4$, $\beta_5$ und $\beta_6$ | aus der Gruppe aus Aminosäuren ausgewählt sind, |
| V | Valin ist, |
| Q | Glutamin ist, und |
| $\beta_7$ | eine andere Aminosäure als Phenylalanin ist. |

**27.** Nukleinsäurefragment nach Anspruch 1 oder Anspruch 26, worin $\beta_7$ eine Aminosäure ist, die aus der Gruppe aus Leucin, Valin und Isoleucin ausgewählt ist.

**28.** Nukleinsäurefragment nach Anspruch 27, worin $\beta_7$ Leucin ist.

**29.** Verfahren zur Herstellung eines Nukleinsäurefragments, das eine für Acetolaktatsynthase kodierende Nukleinsäuresequenz umfaßt, wobei das Nukleinsäurefragment einer Pflanze, in der das Fragment funktionsfähig ist, erhöhte Sulfonylharnstoff-Herbizidresistenz verleiht, welches umfaßt:

a) Isolierung eines Mutanten-Nukleinsäurefragments, das für eine Acetolaktatsynthase kodiert, die erhöhte Resistenz gegenüber einem oder mehreren Sulfonylharnstoff-Herbiziden aufweist, aus Bakterien oder Hefe;
b) Bestimmung der Aminosäuresubstitutionsstelle(n) in der Acetolaktatsynthase, welche der Acetolaktatsynthase Resistenz verleiht bzw. verleihen, durch Vergleich der DNA-Sequenz des Mutanten-Nukleinsäurefragments mit der DNA-Sequenz eines Wildtyp-Nukleinsäurefragments, welches für eine Acetolaktatsynthase kodiert, die gegenüber Sulfonylharnstoff-Herbiziden sensitiv ist; und
c) Einführung einer Mutation in ein für Acetolaktatsynthase kodierendes Nukleinsäurefragment, das eine Sequenz aufweist, die mindestens teilweise einem Pflanzen-Acetolaktatsynthase-Gen entspricht, und das in einer Pflanze funktionsfähig ist, welche Mutation in einer Aminosäuresubstitution an (einer) Stelle(n) resultiert, die der bzw. den Aminosäuresubstitutionsstelle(n) in der Acetolaktatsynthase, die erhöhte Resistenz gegenüber Sulfonylharnstoff-Herbiziden aufweist, entspricht bzw. entsprechen.

**30.** Verfahren zur Herstellung eines Nukleinsäurefragments wie in Anspruch 1 definiert, welches umfaßt:

a) Auswahl einer gegen Sulfonylharnstoff-Herbizid resistenten Pflanzenmutante; und
b) Isolierung des Mutanten-Acetolaktatsynthase-Gens aus der Mutantenpflanze.

**31.** Acetolaktatsynthase-Protein, kodiert von einem Nukleinsäurefragment nach irgendeinem der Ansprüche 1 bis 28.

**32.** Nukleinsäurekonstrukt, umfassend ein Nukleinsäurefragment nach irgendeinem der Ansprüche 1 bis 28.

**33.** Nukleinsäurekonstrukt nach Anspruch 32, ausgewählt aus den bei der ATCC, Rockville, Maryland, USA unter den Hinterlegungsnummern 40237, 67124, 67126 und 67424 hinterlegten Klonierungsvektoren.

**34.** Nukleinsäurekonstrukt nach Anspruch 32, worin ein Nukleinsäurefragment nach irgendeinem der Ansprüche 1 bis 28 mit einem zweiten Nukleinsäurefragment, welches ein zweites Merkmal verleiht, verknüpft ist, so daß, wenn das Nukleinsäurekonstrukt zur Transformation einer Pflanze eingesetzt wird, die Expression von Herbizidresistenz durch die Pflanze nach Aufbringung von Sulfonylharnstoffverbindungen genutzt werden kann, um die Anwesenheit des zweiten Nukleinsäurefragments in der Pflanze nachzuweisen.

**35.** Mikroorganismus, der ein Nukleinsäurefragment oder Nukleinsäurekonstrukt nach irgendeinem der Ansprüche 1 bis 28 oder 32 bis 34 enthält.

**36.** Pflanzenzelle, die mit einem Nukleinsäurefragment oder Nukleinsäurekonstrukt nach irgendeinem der Ansprüche 1 bis 28 oder 32 bis 34 transformiert ist.

**37.** Pflanzengewebekultur, die mit einem Nukleinsäurefragment oder Nukleinsäurekonstrukt nach irgendeinem der Ansprüche 1 bis 28 oder 32 bis 34 transformiert ist.

**38.** Verfahren zur Transformation einer Pflanzenzelle mit einem wie in irgendeinem der Ansprüche 1 bis 28 beanspruchten oder durch ein Verfahren nach den Ansprüchen 29 oder 30 hergestellten Nukleinsäurefragment, welches umfaßt, daß ein Nukleinsäurekonstrukt nach irgendeinem der Ansprüche 32 bis 34 in die Pflanzenzelle eingeführt wird.

**39.** Verfahren zur Auswahl einer Pflanzenzelle, die mit einem wie in irgendeinem der Ansprüche 1 bis 28 beanspruchten oder durch ein Verfahren nach den Ansprüchen 29 oder 30 hergestellten Nukleinsäurefragment transformiert wurde, welches umfaßt die Einführung eines Nukleinsäurekonstrukts nach irgendeinem der Ansprüche 32 bis 34 in eine Pflanzenzelle, deren Wachstum sensitiv ist gegenüber einer Hemmmung durch Herbizide, gegen welche die von dem Nukleinsäurefragment kodierte ALS resistent ist, um eine transformierte Pflanzenzelle zu erzeugen; und die Auswahl einer transformierten Pflanzenzelle, deren Wachstum resistent ist gegenüber einer Hemmung durch die genannten Herbizide bei einer selektiven Konzentration, welche das Wachstum von nicht-transformierten Zellen hemmt.

**40.** Verfahren zur Kontrolle des Wachstums von unerwünschter Vegetation, die an einer Stelle wächst, an der eine Pflanze kultiviert wurde, die mit einem wie in irgendeinem der Ansprüche 1 bis 28 beanspruchten oder durch ein Verfahren nach den Ansprüchen 29 oder 30 hergestellten Nukleinsäurefragment transformiert worden ist, welches umfaßt, daß auf die Stelle eine effektive Menge eines Herbizids aufgebracht wird, gegen das die Pflanze resistent ist.

**41.** Verfahren nach Anspruch 40, worin das Herbizid eine Verbindung ist, welche ausgewählt ist aus der Gruppe aus

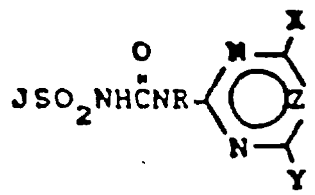

I

worin

R H oder $CH_3$ darstellt;

J

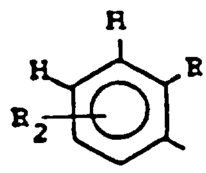 , 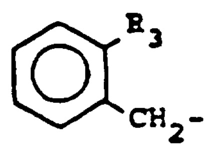 , 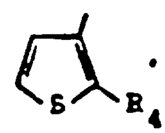 ,

J-1 J-2 J-3

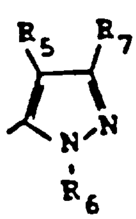 , 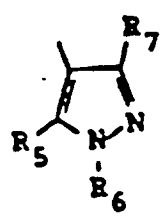 , 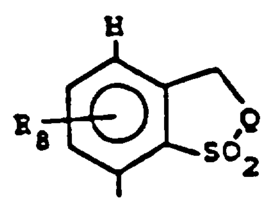

J-4 J-5 J-6

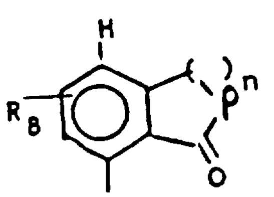 ; 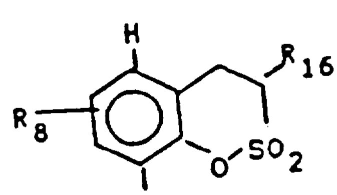 oder 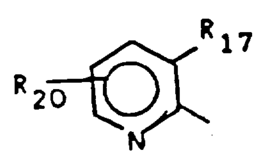

J-7 J-8 J-9

ist;

$R_1$ Cl, Br, $NO_2$, $C_1$-$C_4$-Alkyl, $C_2$-$C_4$-Alkenyl, $CF_3$, $C_1$-$C_4$-Alkoxy, $C_1$-$C_4$-Halogenalkoxy, $C_3$-$C_4$-Alkenyloxy, $C_2$-$C_4$-Halogenalkenyloxy, $C_3$-$C_4$-Alkinyloxy, $CO_2R_9$, $CONR_{10}R_{11}$, $S(O)_mR_{12}$, $OSO_2R_{12}$, Phenyl, $SO_2N(OCH_3)$

$CH_3$, $SO_2NR_{10}R_{11}$,

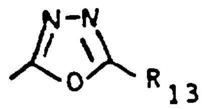 , 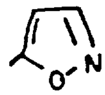 , 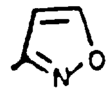 ,

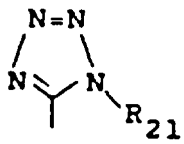 , 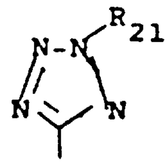 , 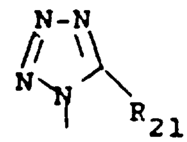 oder 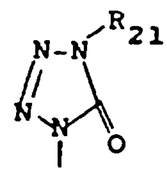

ist;

$R_2$ H, Cl, Br, F, $CH_3$, $NO_2$, $SCH_3$, $OCF_2H$, $OCH_2CF_3$ oder $OCH_3$ ist;
$R_3$ Cl, $NO_2$, $CO_2CH_3$, $CO_2C_2H_5$, $SO_2N(CH_3)_2$, $SO_2CH_3$ oder $SO_2C_2H_5$ ist;
$R_4$ $C_1$-$C_3$-Alkyl, Cl, Br, $NO_2$, $CO_2R_9$, $CON(CH_3)_2$, $SO_2N(CH_3)_2$, $SO_2N(OCH_3)CH_3$ oder $S(O)_mR_{12}$ ist;
$R_5$ $C_1$-$C_3$-Alkyl, $C_4$-$C_5$-Cycloalkylcarbonyl, F, Cl, Br, $NO_2$, $CO_2R_{14}$, $SO_2N(CH_3)_2$, $SO_2R_{12}$ oder Phenyl ist;
$R_6$ H, $C_1$-$C_3$-Alkyl oder $CH_2CH{=}CH_2$ ist;
$R_7$ H, $CH_3$, $OCH_3$, Cl oder Br ist;
$R_8$ H, F, Cl, Br, $CH_3$, $OCH_3$, $CF_3$, $SCH_3$ oder $OCF_2H$ ist;
$R_9$ $C_1$-$C_4$-Alkyl, $C_3$-$C_4$-Alkenyl oder $CH_2CH_2Cl$ ist;
$R_{10}$ H oder $C_1$-$C_3$-Alkyl ist;
$R_{11}$ H oder $C_1$-$C_2$-Alkyl ist;
$R_{12}$ $C_1$-$C_3$-Alkyl ist;
$R_{13}$ H oder $CH_3$ ist;
$R_{14}$ $C_1$-$C_3$-Alkyl oder $CH_2CH{=}CH_2$ ist;
m 0, 1 oder 2 ist;
n 1 oder 2 ist;
Q $CH_2$, $CHCH_3$ oder $NR_{15}$ ist;
$R_{15}$ H oder $C_1$-$C_4$-Alkyl ist;
P O oder $CH_2$ ist;
$R_{16}$ H oder $CH_3$ ist;
$R_{17}$ $C(O)NR_{18}R_{19}$ ist;
$R_{18}$ H oder $CH_3$ ist;
$R_{19}$ $CH_3$ ist;
$R_{20}$ H, Cl, F, Br, $CH_3$, $CF_3$, $OCH_3$ oder $OCF_2H$ ist;
$R_{21}$ H oder $CH_3$ ist;
X $CH_3$, $OCH_3$, $OC_2H_5$ oder $NHCH_3$ ist;
Y $CH_3$, $C_2H_5$, $OCH_3$, $OC_2H_5$, $OCF_2H$, $OCH_2CF_3$, Cl, $CH_2OCH_3$ oder Cyclopropyl ist;
Z CH oder N ist;

und deren landwirtschaftlich annehmbare Salze;
mit der Maßgabe, daß

a) wenn Y Cl ist, Z CH und X $OCH_3$ ist;
b) wenn Y $OCF_2H$ ist, Z CH ist;
c) wenn J J-1 ist und $R_1$ $OSO_2R_{12}$ oder Phenyl ist, Y nicht $OCF_2H$ ist;
d) wenn J J-2 ist, Y nicht $OCF_2H$ oder $OCH_2CF_3$ ist; und
e) wenn J J-3 ist und $R_4$ $S(O)_mR_{12}$ ist, Y nicht $OCH_2CF_3$ ist.

**42.** Verfahren nach Anspruch 40, worin das Herbizid eine Verbindung ist, die ausgewählt ist aus der Gruppe aus

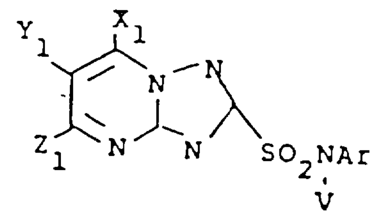

worin

Ar

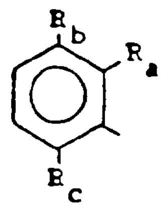

| | |
|---|---|
| | ist; |
| $R_a$ | $C_1$-$C_4$-Alkyl, F, Cl, Br, I, $NO_2$, $S(O)_pR_d$, $COOR_e$ oder $CF_3$ ist; |
| $R_b$ | H, F, Cl, Br, I, $C_1$-$C_4$-Alkyl oder $COOR_e$ ist; |
| $R_c$ | H, $C_1$-$C_4$-Alkyl, F, Cl, Br, I, $CH_2OR_d$, Phenyl, $NO_2$ oder $COOR_e$ ist; |
| $R_d$ | $C_1$-$C_4$-Alkyl ist; |
| $R_e$ | eine Alkyl-, Alkenyl-, Alkinylgruppe mit jeweils bis zu 4 Kohlenstoffatomen oder 2-Ethoxyethyl ist; |
| V | H, $C_1$-$C_3$-Alkyl, Allyl, Propargyl, Benzyl oder ($C_1$-$C_3$-Alkyl)carbonyl ist; |
| $X_1$, $Y_1$ und $Z_1$ | unabhängig H, F, Cl, Br, I, $C_1$-$C_4$-Alkyl, $C_1$-$C_2$-Alkylthio oder $C_1$-$C_4$-Alkoxy sind; und |
| p | 0, 1 oder 2 ist. |

**43.** Verfahren nach Anspruch 40, worin das Herbizid eine Verbindung ist, die ausgewählt ist aus der Gruppe aus

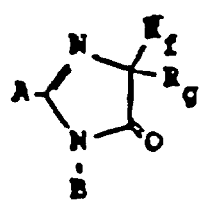

III

worin

A

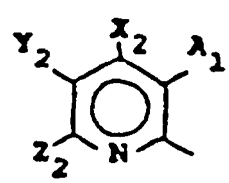

,

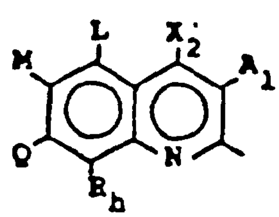

oder

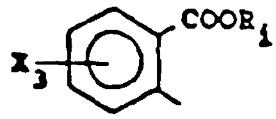

ist;

| | |
|---|---|
| $R_f$ | $C_1$-$C_4$-Alkyl ist; |
| $R_g$ | $C_1$-$C_4$-Alkyl oder $C_{3}$-$_6$ Cycloalkyl ist; |
| $A_1$ | $COOR_i$, $CH_2OH$ oder CHO ist; |
| $R_i$ | H; $C_1$-$C_{12}$-Alkyl, gegebenenfalls substituiert mit $C_1$-$C_3$-Alkyl, $C_3$-$C_6$-Cycloalkyl oder Phenyl; $C_3$-$C_5$-Alkenyl, gegebenenfalls substituiert mit Phenyl oder 1-2 $C_1$-$C_3$-Alkyl, F, Cl, Br oder oder $C_3$-$C_5$-Alkinyl, gegebenenfalls substituiert mit Phenyl oder 1-2 $C_1$-$C_3$-Alkyl, F, Cl, Br oder I, ist; |
| B | H; $C(O)C_1$-$C_6$-Alkyl oder C(O)Phenyl, gegebenenfalls mit Cl, $NO_2$ oder $OCH_3$ substituiert, ist; |
| $X_2$ | H, F, Cl, Br, I, OH oder $CH_3$ ist; |
| $Y_2$ und $Z_2$ | unabhängig H; $C_1$-$C_6$-Alkyl, $C_1$-$C_6$-Alkoxy, F, Cl, Br, I, Phenyl, $NO_2$, CN, $CF_3$ oder $SO_2CH_3$ sind; |
| $X_3$ | H, $C_1$-$C_3$-Alkyl, F, Cl, Br, I oder $NO_2$ ist; und |
| L, M, Q und $R_h$ | unabhängig H, F, Cl, Br, I, $CH_3$, $OCH_3$, $NO_2$, $CF_3$, CN, $N(CH_3)_2$, $NH_2$, $SCH_3$ oder $SO_2CH_3$ sind, mit der Maßgabe, daß nur eines von M oder Q ein anderer Substituent als H, F, Cl, Br, I, $CH_3$ oder $OCH_3$ sein kann. |

**44.** Verfahren zur Erzeugung einer Pflanze, die ein wie in irgendeinem der Ansprüche 1 bis 28 definiertes oder durch ein Verfahren nach Anspruch 29 oder Anspruch 30 hergestelltes Nukleinsäurefragment enthält, welches umfaßt die Transformation einer Pflanzenzelle mit einem Nukleinsäurefragment oder Nukleinsäurekonstrukt wie in irgendeinem der Ansprüche 1 bis 28 oder 32 bis 34 definiert oder durch ein Verfahren nach Anspruch 29 oder Anspruch 30 hergestellt, die Regeneration von transformierten Pflanzen aus der transformierten Pflanzenzelle und gegebenenfalls die biologische Replikation der transformierten Pflanze.

**45.** Verfahren nach Anspruch 44, worin die Pflanze eine Sojabohnen-, Mais-, Zuckerrüben-, Sonnenblumen-, Tabak-, Kartoffel-, Tomaten-, Alfalfa-, Melonen-, Baumwoll-, Salat-, Weizen- oder Reispflanze oder eine Brassica-Spezies ist.

**46.** Verfahren zur Erzeugung von Samen, der ein wie in irgendeinem der Ansprüche 1 bis 28 definiertes oder durch ein Verfahren nach Anspruch 29 oder Anspruch 30 hergestelltes Nukleinsäurefragment enthält, welches umfaßt die Transformation einer Pflanzenzelle mit einem Nukleinsäurefragment oder Nukleinsäurekonstrukt wie in irgendeinem der Ansprüche 1 bis 28 oder 32 bis 34 definiert oder durch ein Verfahren nach Anspruch 29 oder Anspruch 30 hergestellt, die Regeneration von transformierten Pflanzen aus der transformierten Pflanzenzelle, gegebenenfalls die biologische Replikation der transformierten Pflanzen und die Gewinnung von Samen aus den transformierten Pflanzen.

**47.** Pflanze, die ein Nukleinsäurefragment oder Nukleinsäurekonstrukt nach irgendeinem der Ansprüche 1 bis 28 oder 32 bis 34 enthält.

**48.** Pflanze nach Anspruch 47, die eine Sojabohnen-, Mais-, Zuckerrüben-, Sonnenblumen-, Tabak-, Kartoffel-, Tomaten-, Alfalfa-, Melonen-, Baumwoll-, Salat-, Weizen- oder Reispflanze oder eine Brassica-Spezies ist.

**49.** Samen, der ein Nukleinsäurefragment oder Nukleinsäurekonstrukt nach irgendeinem der Ansprüche 1 bis 28 oder 32 bis 34 enthält, erhalten durch Züchtung einer Pflanze nach Anspruch 47 oder Anspruch 48.

**Patentansprüche für folgende Vertragsstaaten : AT, ES**

**1.** Verfahren zur Herstellung eines Nukleinsäurefragments, das eine für Acetolaktatsynthase kodierende Nukleotidsequenz umfaßt, welches Nukleinsäurefragment einer Pflanze, in der das Fragment funktionsfähig ist, erhöhte Sulfonylharnstoff-Herbizidresistenz verleiht, worin eine Nukleotidsequenz, welche für Acetolaktatsynthase kodiert, welche eine Sequenz aufweist, die mindestens teilweise einem Pflanzen-Acetolaktatsynthase-Gen entspricht und in einer Pflanze funktionsfähig ist, und welche mindestens eine Subsequenz umfaßt, die für eine der im wesentlichen konservierten Aminosäure-Subsequenzen mit den Bezeichnungen A, B, C, D, E, F und G in Fig. 6 kodiert, so mutiert wird, daß mindestens eine der folgenden Bedingungen erfüllt ist;

a) das Nukleinsäurefragment weist eine Sequenz auf, die für eine Aminosäure-Subsequenz A kodiert, worin $\varepsilon_1$ eine andere Aminosäure als Alanin ist und/oder $\varepsilon_2$ eine andere Aminosäure als Glycin ist,
b) das Nukleinsäurefragment weist eine Sequenz auf, die für eine Aminosäure-Subsequenz B kodiert, worin $\alpha_1$ eine andere Aminosäure als Prolin ist,
c) das Nukleinsäurefragment weist eine Sequenz auf, die für eine Aminosäure-Subsequenz C kodiert, worin $\delta_2$ eine andere Aminosäure als Alanin ist,
d) das Nukleinsäurefragment weist eine Sequenz auf, die für eine Aminosäure-Subsequenz D kodiert, worin $\lambda_1$ eine andere Aminosäure als Lysin ist,
e) das Nukleinsäurefragment weist eine Sequenz auf, die für eine Aminosäure-Subsequenz E kodiert, worin $\gamma_1$ eine andere Aminosäure als Asparaginsäure ist,
f) das Nukleinsäurefragment weist eine Sequenz auf, die für eine Aminosäure-Subsequenz F kodiert, worin $\beta_3$ eine andere Aminosäure als Tryptophan ist und/oder $\beta_8$ eine andere Aminosäure als Valin ist und/oder $\beta_7$ eine andere Aminosäure als Phenylalanin ist, und
g) das Nukleinsäurefragment weist eine Sequenz auf, die für eine Aminosäure-Subsequenz G kodiert, worin $\sigma_1$ eine andere Aminosäure als Methionin ist.

**2.** Verfahren nach Anspruch 1, worin das Fragment eine Sequenz aufweist, die für eine Aminosäure-Subsequenz A mit der Formel

$$PG\varepsilon_2A$$

kodiert, worin

P Prolin ist,
G Glycin ist,
$\varepsilon_2$ eine andere Aminosäure als Glycin ist, und
A Alanin ist.

**3.** Verfahren nach Anspruch 1 oder Anspruch 2, worin $\varepsilon_2$ eine Aminosäure ist, die aus der Gruppe aus Serin, Threonin oder Cystein ausgewählt ist.

**4.** Verfahren nach Anspruch 3, worin $\varepsilon_2$ Serin ist.

**5.** Verfahren nach Anspruch 1, worin das Fragment eine Sequenz aufweist, die für eine Aminosäure-Subsequenz A mit der Formel

$$PGG\varepsilon_1$$

kodiert, worin

P Prolin ist,
G Glycin ist, und
$\varepsilon_1$ eine andere Aminosäure als Alanin oder Glycin ist.

**6.** Verfahren nach Anspruch 1, worin das Fragment eine Sequenz aufweist, die für eine Aminosäure-Subsequenz B mit der Formel

$$GQV\alpha_1$$

kodiert, worin

G Glycin ist,

Q Glutamin ist,
V Valin ist,
$\alpha_1$ eine andere Aminosäure als Prolin ist.

**7.** Verfahren nach Anspruch 1 oder Anspruch 6, worin $\alpha_1$ eine Aminosäure ist, die aus der Gruppe aus Alanin, Glycin, Arginin, Lysin, Histidin, Serin, Cystein, Threonin, Glutamin und Asparagin ausgewählt ist.

**8.** Verfahren nach Anspruch 7, worin $\alpha_1$ Alanin, Arginin, Serin oder Glutamin ist.

**9.** Verfahren nach Anspruch 1, worin das Fragment eine Sequenz aufweist, die für eine Aminosäure-Subsequenz C mit der Formel

$$IG\delta_1D\delta_2FQE$$

kodiert, worin

I Isoleucin ist,
G Glycin ist,
$\delta_1$ aus der Gruppe aus Aminosäuren ausgewählt ist,
D Asparaginsäure ist, und
$\delta_2$ eine andere Aminosäure als Alanin ist,
F Phenylalanin ist,
Q Glutamin ist, und
E Glutaminsäure ist.

**10.** Verfahren nach Anspruch 1 oder Anspruch 9, worin $\delta_2$ eine Aminosäure ist, die aus der Gruppe aus Threonin, Serin, Cystein, Tyrosin, Asparaginsäure, Glutaminsäure, Tryptophan, Histidin, Phenylalanin, Arginin und Lysin ausgewählt ist.

**11.** Verfahren nach Anspruch 10, worin $\delta_2$ Threonin, Cystein, Asparaginsäure, Glutaminsäure, Tryptophan oder Arginin ist.

**12.** Verfahren nach Anspruch 1, worin das Fragment eine Sequenz aufweist, die für eine Aminosäure-Subsequenz D mit der Formel

$$P\lambda_1D$$

kodiert, worin

P Prolin ist;
$\lambda_1$ eine andere Aminosäure als Lysin ist; und
D Asparaginsäure ist.

**13.** Verfahren nach Anspruch 1 oder Anspruch 12, worin $\lambda_1$ eine Aminosäure ist, die aus der Gruppe aus Glycin, Alanin, Leucin, Isoleucin, Valin, Threonin, Serin, Cystein, Tyrosin, Glutaminsäure, Asparaginsäure, Prolin, Asparagin, Glutamin, Tryptophan und Phenylalanin ausgewählt ist.

**14.** Verfahren nach Anspruch 13, worin $\lambda_1$ Glycin, Leucin, Threonin, Glutaminsäure, Asparaginsäure, Asparagin, Tryptophan oder Prolin ist.

**15.** Verfahren nach Anspruch 1, worin das Fragment eine Sequenz aufweist, die für eine Aminosäure-Subsequenz G mit der Formel

$$MLG\sigma_1HG$$

kodiert, worin

M Methionin ist,
L Leucin ist,
G Glycin ist,
$\sigma_1$ eine andere Aminosäure als Methionin ist,
H Histidin ist, und
G Glycin ist.

**16.** Verfahren nach Anspruch 1 oder Anspruch 15, worin $\sigma_1$ eine Aminosäure ist, die aus der Gruppe aus Glutaminsäure, Asparaginsäure, Prolin, Valin, Leucin oder Isoleucin ausgewählt ist.

**17.** Verfahren nach Anspruch 16, worin $\sigma_1$ Prolin, Glutaminsäure oder Valin ist.

**18.** Verfahren nach Anspruch 1, worin das Fragment eine Sequenz aufweist, die für eine Aminosäure-Subsequenz E mit der Formel

$$RFD\gamma_1R$$

kodiert, worin

R Arginin ist,
F Phenylalanin ist,
D Asparaginsäure ist, und
$\gamma_1$ eine andere Aminosäure als Asparaginsäure ist.

**19.** Verfahren nach Anspruch 1 oder Anspruch 18, worin $\gamma_1$ eine Aminosäure ist, die aus der Gruppe aus Glycin, Alanin, Valin, Leucin, Isoleucin, Serin, Threonin, Cystein, Tyrosin, Glutaminsäure, Prolin, Asparagin, Glutamin, Lysin, Arginin, Tryptophan, Histidin und Phenylalanin ausgewählt ist.

**20.** Verfahren nach Anspruch 19, worin $\gamma_1$ Glycin, Valin, Serin, Glutaminsäure, Prolin, Asparagin, Lysin, Cystein oder Tryptophan ist.

**21.** Verfahren nach Anspruch 1, worin das Fragment eine Sequenz aufweist, die für eine Aminosäure-Subsequenz F mit der Formel

$$G\beta_1\beta_8\beta_2Q\beta_3\beta_4\beta_5\beta_6\beta_7$$

kodiert, worin

| | |
|---|---|
| G | Glycin ist, |
| $\beta_1$, $\beta_2$, $\beta_3$, $\beta_4$, $\beta_5$, $\beta_6$ und $\beta_7$ | aus der Gruppe aus Aminosäuren ausgewählt sind, |
| Q | Glutamin ist, und |
| $\beta_8$ | eine andere Aminosäure als Valin ist. |

**22.** Verfahren nach Anspruch 1 oder Anspruch 21, worin $\beta_1$ Methionin ist, $\beta_3$ Tryptophan ist, $\beta_7$ Phenylalanin ist und $\beta_8$ eine andere Aminosäure als Valin ist.

**23.** Verfahren nach irgendeinem der Ansprüche 1, 21 und 22, worin $\beta_8$ eine Aminosäure ist, die aus der Gruppe aus Alanin und Glycin ausgewählt ist.

**24.** Verfahren nach Anspruch 23, worin $\beta_8$ Alanin ist.

**25.** Verfahren nach Anspruch 1, worin das Fragment eine Sequenz aufweist, die für eine Aminosäure-Subsequenz F mit der Formel

$$G\beta_1V\beta_2Q\beta_3\beta_4\beta_5\beta_6\beta_7$$

kodiert, worin

| | |
|---|---|
| G | Glycin ist, |
| $\beta_1$, $\beta_2$, $\beta_4$, $\beta_5$, $\beta_6$ und $\beta_7$ | aus der Gruppe aus Aminosäuren ausgewählt sind, |
| V | Valin ist, |
| Q | Glutamin ist, und |
| $\beta_3$ | eine andere Aminosäure als Tryptophan oder Prolin ist. |

**26.** Verfahren nach Anspruch 1, worin das Fragment eine Sequenz aufweist, die für eine Aminosäure-Subsequenz F mit der Formel

$$G\beta_1V\beta_2Q\beta_3\beta_4\beta_5\beta_6\beta_7$$

kodiert, worin

| | |
|---|---|
| G | Glycin ist, |
| $\beta_1$, $\beta_2$, $\beta_3$, $\beta_4$, $\beta_5$ und $\beta_6$ | aus der Gruppe aus Aminosäuren ausgewählt sind, |
| V | Valin ist, |
| Q | Glutamin ist, und |
| $\beta_7$ | eine andere Aminosäure als Phenylalanin ist. |

**27.** Verfahren nach Anspruch 1 oder Anspruch 26, worin $\beta_7$ eine Aminosäure ist, die aus der Gruppe aus Leucin, Valin und Isoleucin ausgewählt ist.

**28.** Verfahren nach Anspruch 27, worin $\beta_7$ Leucin ist.

**29.** Verfahren zur Herstellung eines Nukleinsäurefragments, das eine für Acetolaktatsynthase kodierende Nukleinsäuresequenz umfaßt, wobei das Nukleinsäurefragment einer Pflanze, in der das Fragment funktionsfähig ist, erhöhte Sulfonylharnstoff-Herbizidresistenz verleiht, welches umfaßt:

a) Isolierung eines Mutanten-Nukleinsäurefragments, das für eine Acetolaktatsynthase kodiert, die erhöhte Resistenz gegenüber einem oder mehreren Sulfonylharnstoff-Herbiziden aufweist, aus Bakterien oder Hefe;
b) Bestimmung der Aminosäuresubstitutionsstelle(n) in der Acetolaktatsynthase, welche der Acetolaktatsynthase Resistenz verleiht bzw. verleihen, durch Vergleich der DNA-Sequenz des Mutanten-Nukleinsäurefragments mit der DNA-Sequenz eines Wildtyp-Nukleinsäurefragments, welches für eine Acetolaktatsynthase kodiert, die gegenüber Sulfonylharnstoff-Herbiziden sensitiv ist; und
c) Einführung einer Mutation in ein für Acetolaktatsynthase kodierendes Nukleinsäurefragment, das eine Sequenz aufweist, die mindestens teilweise einem Pflanzen-Acetolaktatsynthase-Gen entspricht, und das in einer Pflanze funktionsfähig ist, welche Mutation in einer Aminosäuresubstitution an (einer) Stelle(n) resultiert, die der bzw. den Aminosäuresubstitutionsstelle(n) in der Acetolaktatsynthase, die erhöhte Resistenz gegenüber Sulfonylharnstoff-Herbiziden aufweist, entspricht bzw. entsprechen.

**30.** Verfahren zur Herstellung eines Nukleinsäurefragments wie in Anspruch 1 definiert, welches umfaßt:

a) Auswahl einer gegen Sulfonylharnstoff-Herbizid resistenten Pflanzenmutante; und
b) Isolierung des Mutanten-Acetolaktatsynthase-Gens aus der Mutantenpflanze.

**31.** Verfahren zur Herstellung eines Nukleinsäurekonstrukts, das ein durch ein Verfahren nach irgendeinem der Ansprüche 1 bis 30 hergestelltes Nukleinsäurefragment umfaßt, welches die Verknüpfung des Nukleinsäurefragments mit einem zweiten Nukleinsäurefragment umfaßt.

**32.** Verfahren nach Anspruch 31, worin das zweite Nukleinsäurefragment ein zweites Merkmal verleiht, so daß, wenn das Nukleinsäurekonstrukt zur Transformation einer Pflanze eingesetzt wird, die Expression von Herbizidresistenz durch die Pflanze nach Aufbringung von Sulfonylharnstoffverbindungen genutzt werden kann, um die Anwesenheit

des zweiten Nukleinsäurefragments in der Pflanze nachzuweisen.

**33.** Nukleinsäurekonstrukt, ausgewählt aus den bei der ATCC, Rockville, Maryland, USA unter den Hinterlegungsnummern 40237, 67124, 67126 und 67424 hinterlegten Klonierungsvektoren.

**34.** Verfahren zur Herstellung einer Pflanzen-Acetolaktatsynthase mit erhöhter Resistenz gegenüber einem oder mehreren Sulfonylharnstoff-Herbiziden, welches umfaßt die Expression einer für Pflanzen-Acetolaktatsynthase kodierenden Sequenz in einem Nukleinsäurefragment oder Nukleinsäurekonstrukt wie in irgendeinem der Ansprüche 1 bis 28 oder 31 bis 33 definiert oder durch ein Verfahren nach Anspruch 29 oder 30 hergestellt.

**35.** Mikroorganismus, enthaltend ein Nukleinsäurefragment oder Nukleinsäurekonstrukt wie in irgendeinem der Ansprüche 1 bis 28 oder 31 bis 33 definiert.

**36.** Pflanzenzelle, die mit einem Nukleinsäurefragment oder Nukleinsäurekonstrukt wie in irgendeinem der Ansprüche 1 bis 28 oder 31 bis 33 definiert transformiert ist.

**37.** Pflanzengewebekultur, die mit einem Nukleinsäurefragment oder Nukleinsäurekonstrukt wie in irgendeinem der Ansprüche 1 bis 28 oder 31 bis 33 definiert transformiert ist.

**38.** Verfahren zur Transformation einer Pflanzenzelle mit einem Nukleinsäurefragment wie in irgendeinem der Ansprüche 1 bis 28 definiert oder durch ein Verfahren nach Anspruch 29 oder 30 hergestellt, welches umfaßt, daß ein Nukleinsäurekonstrukt wie in irgendeinem der Ansprüche 31 bis 33 definiert in die Pflanzenzelle eingeführt wird.

**39.** Verfahren zur Auswahl einer Pflanzenzelle, die mit einem wie in irgendeinem der Ansprüche 1 bis 28 definierten oder durch ein Verfahren nach Anspruch 29 oder 30 hergestellten Nukleinsäurefragment transformiert wurde, welches umfaßt die Einführung eines Nukleinsäurekonstrukts wie in irgendeinem der Ansprüche 31 bis 33 definiert in eine Pflanzenzelle, deren Wachstum sensitiv ist gegenüber einer Hemmmung durch Herbizide, gegen welche die von dem Nukleinsäurefragment kodierte ALS resistent ist, um eine transformierte Pflanzenzelle zu erzeugen; und die Auswahl einer transformierten Pflanzenzelle, deren Wachstum resistent ist gegenüber einer Hemmung durch die genannten Herbizide bei einer selektiven Konzentration, welche das Wachstum von nicht-transformierten Zellen hemmt.

**40.** Verfahren zur Kontrolle des Wachstums von unerwünschter Vegetation, die an einer Stelle wächst, an der eine Pflanze kultiviert wurde, die mit einem wie in irgendeinem der Ansprüche 1 bis 28 definierten oder durch ein Verfahren nach Anspruch 29 oder 30 hergestellten Nukleinsäurefragment transformiert worden ist, welches umfaßt, daß auf die Stelle eine effektive Menge eines Herbizids aufgebracht wird, gegen das die Pflanze resistent ist.

**41.** Verfahren nach Anspruch 40, worin das Herbizid eine Verbindung ist, welche ausgewählt ist aus der Gruppe aus

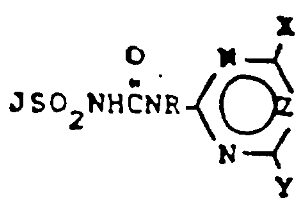

I

worin

R H oder $CH_3$ darstellt;
J

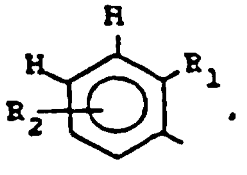 , 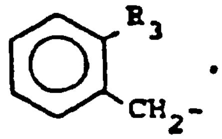 , 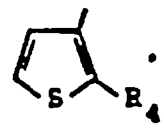 ,

J-1 J-2 J-3

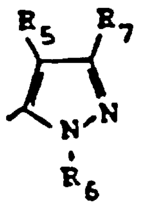 , 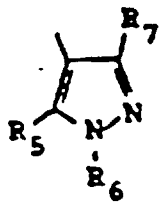 , 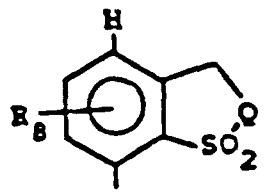

J-4 J-5 J-6

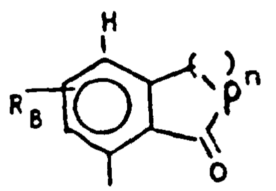 ; 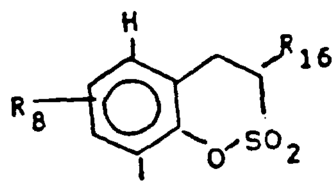 oder 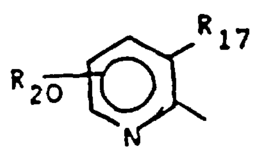

J-7 J-8 J-9

ist;

$R_1$ Cl, Br, $NO_2$, $C_1$-$C_4$-Alkyl, $C_2$-$C_4$-Alkenyl, $CF_3$, $C_1$-$C_4$-Alkoxy, $C_1$-$C_4$-Halogenalkoxy, $C_3$-$C_4$-Alkenyloxy, $C_2$-$C_4$-Halogenalkenyloxy, $C_3$-$C_4$-Alkinyloxy, $CO_2R_9$, $CONR_{10}R_{11}$, $S(O)_mR_{12}$, $OSO_2R_{12}$, Phenyl $SO_2N(OCH_3)CH_3$, $SO_2NR_{10}R_{11}$,

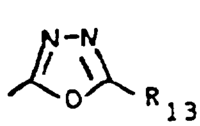 , 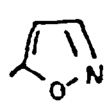 , 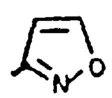 ,

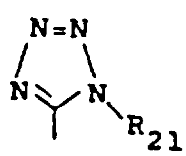 , 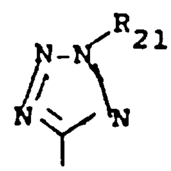 , 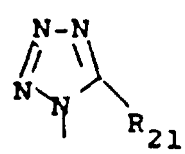 oder 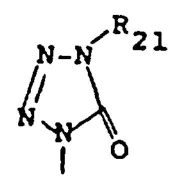

ist;

$R_2$ H, Cl, Br, F, $CH_3$, $NO_2$, $SCH_3$, $OCF_2H$, $OCH_2CF_3$ oder $OCH_3$ ist;

$R_3$ Cl, $NO_2$, $CO_2CH_3$, $CO_2C_2H_5$, $SO_2N(CH_3)_2$, $SO_2CH_3$ oder $SO_2C_2H_5$ ist;
$R_4$ $C_1$-$C_3$-Alkyl, Cl, Br, $NO_2$, $CO_2R_9$, $CON(CH_3)_2$, $SO_2N(CH_3)_2$, $SO_2N(OCH_3)CH_3$ oder $S(O)_mR_{12}$ ist;
$R_5$ $C_1$-$C_3$-Alkyl, $C_4$-$C_5$-Cycloalkylcarbonyl, F, Cl, Br, $NO_2$, $CO_2R_{14}$, $SO_2N(CH_3)_2$, $SO_2R_{12}$ oder Phenyl ist;
$R_6$ H, $C_1$-$C_3$-Alkyl oder $CH_2CH{=}CH_2$ ist;
$R_7$ H, $CH_3$, $OCH_3$, Cl oder Br ist;
$R_8$ H, F, Cl, Br, $CH_3$, $OCH_3$, $CF_3$, $SCH_3$ oder $OCF_2H$ ist;
$R_9$ $C_1$-$C_4$-Alkyl, $C_3$-$C_4$-Alkenyl oder $CH_2CH_2Cl$ ist;
$R_{10}$ H oder $C_1$-$C_3$-Alkyl ist;
$R_{11}$ H oder $C_1$-$C_2$-Alkyl ist;
$R_{12}$ $C_1$-$C_3$-Alkyl ist;
$R_{13}$ H oder $CH_3$ ist;
$R_{14}$ $C_1$-$C_3$-Alkyl oder $CH_2CH{=}CH_2$ ist;
m 0, 1 oder 2 ist;
n 1 oder 2 ist;
Q $CH_2$, $CHCH_3$ oder $NR_{15}$ ist;
$R_{15}$ H oder $C_1$-$C_4$-Alkyl ist;
P O oder $CH_2$ ist;
$R_{16}$ H oder $CH_3$ ist;
$R_{17}$ $C(O)NR_{18}R_{19}$ ist;
$R_{18}$ H oder $CH_3$ ist;
$R_{19}$ $CH_3$ ist;
$R_{20}$ H, Cl, F, Br, $CH_3$, $CF_3$, $OCH_3$ oder $OCF_2H$ ist;
$R_{21}$ H oder $CH_3$ ist;
X $CH_3$, $OCH_3$, $OC_2H_5$ oder $NHCH_3$ ist;
Y $CH_3$, $C_2H_5$, $OCH_3$, $OC_2H_5$, $OCF_2H$, $OCH_2CF_3$, Cl, $CH_2OCH_3$ oder Cyclopropyl ist;
Z CH oder N ist;

und deren landwirtschaftlich annehmbare Salze;
mit der Maßgabe, daß

a) wenn Y Cl ist, Z CH und X $OCH_3$ ist;
b) wenn Y $OCF_2H$ ist, Z CH ist;
c) wenn J J-1 ist und $R_1$ $OSO_2R_{12}$ oder Phenyl ist, Y nicht $OCF_2H$ ist;
d) wenn J J-2 ist, Y nicht $OCF_2H$ oder $OCH_2CF_3$ ist; und
e) wenn J J-3 ist und $R_4$ $S(O)_mR_{12}$ ist, Y nicht $OCH_2CF_3$ ist.

**42.** Verfahren nach Anspruch 40, worin das Herbizid eine Verbindung ist, die ausgewählt ist aus der Gruppe aus

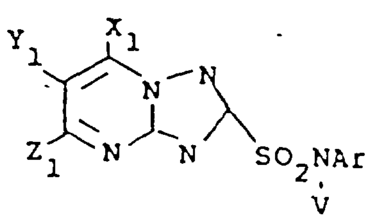

worin

Ar

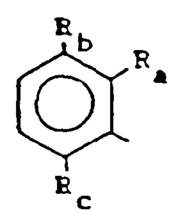

ist;

$R_a$ — $C_1$-$C_4$-Alkyl, F, Cl, Br, I, $NO_2$, $S(O)_pR_d$, $COOR_e$ oder $CF_3$ ist;
$R_b$ — H, F, Cl, Br, I, $C_1$-$C_4$-Alkyl oder $COOR_e$ ist;
$R_c$ — H, $C_1$-$C_4$-Alkyl, F, Cl, Br, I, $CH_2OR_d$, Phenyl, $NO_2$ oder $COOR_e$ ist;
$R_d$ — $C_1$-$C_4$-Alkyl ist;
$R_e$ — eine Alkyl-, Alkenyl-, Alkinylgruppe mit jeweils bis zu 4 Kohlenstoffatomen oder 2-Ethoxyethyl ist;
V — H, $C_1$-$C_3$-Alkyl, Allyl, Propargyl, Benzyl oder ($C_1$-$C_3$-Alkyl)carbonyl ist;
$X_1$, $Y_1$ und $Z_1$ — unabhängig H, F, Cl, Br, I, $C_1$-$C_4$-Alkyl, $C_1$-$C_2$-Alkylthio oder $C_1$-$C_4$-Alkoxy sind; und
p — 0, 1 oder 2 ist.

**43.** Verfahren nach Anspruch 40, worin das Herbizid eine Verbindung ist, die ausgewählt ist aus der Gruppe aus

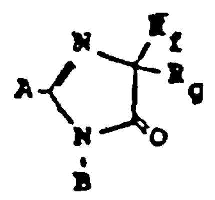

III

worin

A

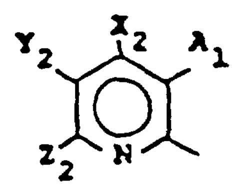

·

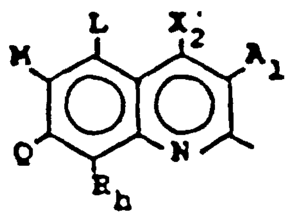

oder

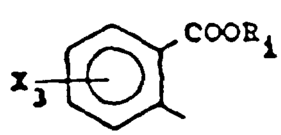

ist;

$R_f$ — $C_1$-$C_4$-Alkyl ist;
$R_g$ — $C_1$-$C_4$-Alkyl oder $C_3$-$C_6$-Cycloalkyl ist;
$A_1$ — $COOR_i$, $CH_2OH$ oder CHO ist;
$R_i$ — H; $C_1$-$C_{12}$-Alkyl, gegebenenfalls substituiert mit $C_1$-$C_3$-Alkyl, $C_3$-$C_6$-Cycloalkyl oder Phenyl; $C_3$-$C_5$-Alkenyl, gegebenenfalls substituiert mit Phenyl oder 1-2 $C_1$-$C_3$-Alkyl, F, Cl, Br oder I; oder $C_3$-$C_5$-Alkinyl, gegebenenfalls substituiert mit Phenyl oder 1-2 $C_1$-$C_3$-Alkyl, F, Cl, Br oder I, ist;
B — H; $C(O)C_1$-$C_6$-Alkyl oder C(O)Phenyl, gegebenenfalls mit Cl, $NO_2$ oder $OCH_3$ substituiert, ist;
$X_2$ — H, F, Cl, Br, I, OH oder $CH_3$ ist;
$Y_2$ und $Z_2$ — unabhängig H; $C_1$-$C_6$-Alkyl, $C_1$-$C_6$-Alkoxy, F, Cl, Br, I, Phenyl, $NO_2$, CN, $CF_3$ oder $SO_2CH_3$ sind;
$X_3$ — H, $C_1$-$C_3$-Alkyl, F, Cl, Br, I oder $NO_2$ ist; und
L, M, Q und $R_h$ — unabhängig H, F, Cl, Br, I, $CH_3$, $OCH_3$, $NO_2$, $CF_3$, CN, $N(CH_3)_2$, $NH_2$, $SCH_3$ oder $SO_2CH_3$ sind, mit der Maßgabe, daß nur eines von M oder Q ein anderer Substituent als H, F, Cl, Br, I, $CH_3$

oder $OCH_3$ sein kann.

**44.** Verfahren zur Erzeugung einer Pflanze, die ein wie in irgendeinem der Ansprüche 1 bis 28 definiertes oder durch ein Verfahren nach Anspruch 29 oder Anspruch 30 hergestelltes Nukleinsäurefragment enthält, welches umfaßt die Transformation einer Pflanzenzelle mit einem Nukleinsäurefragment oder Nukleinsäurekonstrukt wie in irgendeinem der Ansprüche 1 bis 28 oder 31 bis 33 definiert oder durch ein Verfahren nach Anspruch 29 oder Anspruch 30 hergestellt, die Regeneration von transformierten Pflanzen aus der transformierten Pflanzenzelle und gegebenenfalls die biologische Replikation der transformierten Pflanzen.

**45.** Verfahren nach Anspruch 44, worin die Pflanze eine Sojabohnen-, Mais-, Zuckerrüben-, Sonnenblumen-, Tabak-, Kartoffel-, Tomaten-, Alfalfa-, Melonen-, Baumwoll-, Salat-, Weizen- oder Reispflanze oder eine Brassica-Spezies ist.

**46.** Verfahren zur Erzeugung von Samen, der ein wie in irgendeinem der Ansprüche 1 bis 28 definiertes oder durch ein Verfahren nach Anspruch 29 oder Anspruch 30 hergestelltes Nukleinsäurefragment enthält, welches umfaßt die Transformation einer Pflanzenzelle mit einem Nukleinsäurefragment oder Nukleinsäurekonstrukt wie in irgendeinem der Ansprüche 1 bis 28 oder 31 bis 33 definiert oder durch ein Verfahren nach Anspruch 29 oder Anspruch 30 hergestellt, die Regeneration von transformierten Pflanzen aus der transformierten Pflanzenzelle, gegebenenfalls die biologische Replikation der transformierten Pflanzen und die Gewinnung von Samen aus den transformierten Pflanzen.

**47.** Pflanze, die ein wie in irgendeinem der Ansprüche 1 bis 28 oder 31 bis 33 definiertes Nukleinsäurefragment oder Nukleinsäurekonstrukt enthält.

**48.** Pflanze nach Anspruch 47, die eine Sojabohnen-, Mais-, Zuckerrüben-, Sonnenblumen-, Tabak-, Kartoffel-, Tomaten-, Alfalfa-, Melonen-, Baumwoll-, Salat-, Weizen- oder Reispflanze oder eine Brassica-Spezies ist.

**49.** Samen, der ein wie in irgendeinem der Ansprüche 1 bis 28 oder 31 bis 33 definiertes Nukleinsäurefragment oder Nukleinsäurekonstrukt enthält, erhalten durch Züchtung einer Pflanze nach Anspruch 47 oder Anspruch 48.

**Revendications**

**Revendications pour les Etats contractants suivants : DE, GB, FR, IT, NL, SE, CH, LI, BE, LU, GR**

**1.** Un fragment d'acide nucléique comprenant une séquence nucléotidique codant pour une acétolactate-synthase, ladite séquence nucléotidique correspondant, au moins en partie, à un gène d'acétolactate-synthase végétale conférant une résistance accrue aux herbicides du type sulfonylurée à une plante dans laquelle ledit fragment est fonctionnel, ladite séquence nucléotidique comprenant au moins une sous-séquence codant pour l'une des sous-séquences d'acides aminés sensiblement conservées désignées par A, B, C, D, E, F et G sur la Figure 6, le fragment d'acide nucléique étant en outre, caractérisé en ce qu'au moins l'une des conditions suivantes est satisfaite :

a) le fragment d'acide nucléique a une séquence qui code pour une sous-séquence d'acides aminés A dans laquelle $\varepsilon_1$ est un acide aminé autre l'alanine et/ou $\varepsilon_2$ est un acide aminé autre que la glycine,
b) le fragment d'acide nucléique a une séquence qui code pour une sous-séquence d'acides aminés B dans laquelle $\alpha_1$ est un acide aminé autre que la proline,
c) le fragment d'acide nucléique a une séquence qui code pour une sous-séquence d'acides aminés C dans laquelle $\delta_2$ est un acide aminé autre que l'alanine,
d) le fragment d'acide nucléique a une séquence qui code pour une sous-séquence d'acides aminés D dans laquelle $\lambda_1$ est un acide aminé autre que la lysine,
e) le fragment d'acide nucléique a une séquence qui code pour une sous-séquence d'acides aminés E dans laquelle $\gamma_1$ est un acide aminé autre que l'acide aspartique,
f) le fragment d'acide nucléique a une séquence qui code pour une sous-séquence d'acides aminés F dans laquelle $\beta_3$ est un acide aminé autre que le tryptophane et/ou $\beta_8$ est un acide aminé autre que la valine et/ou $\beta_7$ est un acide aminé autre que la phénylalanine, et
g) le fragment d'acide nucléique a une séquence qui code pour une sous-séquence d'acides aminés G dans

laquelle $\sigma_1$ est un acide aminé autre que la méthionine.

**2.** Un fragment d'acide nucléique selon la revendication 1, dans lequel le fragment a une séquence qui code pour une sous-séquence d'acides aminés A ayant la formule

$$PG\varepsilon_2A$$

où :

- P est la proline,
- G est la glycine,
- $\varepsilon_2$ est un acide aminé autre que la glycine, et
- A est l'alanine.

**3.** Un fragment d'acide nucléique selon la revendication 1 ou la revendication 2, dans lequel $\varepsilon_2$ est un acide aminé choisi dans le groupe formé par la sérine, la thréonine et la cystéine.

**4.** Un fragment d'acide nucléique selon la revendication 3, dans lequel $\varepsilon_2$ est la sérine.

**5.** Un fragment d'acide nucléique selon la revendication 1, dans lequel le fragment a une séquence qui code pour une sous-séquence d'acides aminés A ayant la formule

$$PGG\varepsilon_1$$

où :

- P est la proline,
- G est la glycine, et
- $\varepsilon_1$ est un acide aminé autre que l'alanine ou la glycine.

**6.** Un fragment d'acide nucléique selon la revendication 1, dans lequel le fragment a une séquence qui code pour une sous-séquence d'acides aminés B ayant la formule

$$GQV\alpha_1$$

où :

- G est la glycine,
- Q est la glutamine,
- V est la valine,
- $\alpha_1$ est un acide aminé autre que la proline.

**7.** Un fragment d'acide nucléique selon la revendication 1 ou la revendication 6, dans lequel $\alpha_1$ est un acide aminé choisi dans le groupe formé par l'alanine, la glycine, l'arginine, la lysine, l'histidine, la sérine, la cystéine, la thréonine, la glutamine et l'asparagine.

**8.** Un fragment d'acide nucléique selon la revendication 7, dans lequel $\alpha_1$ est l'alanine, l'arginine, la sérine ou la glutamine.

**9.** Un fragment d'acide nucléique selon la revendication 1, dans lequel le fragment a une séquence qui code pour une sous-séquence d'acides aminés C ayant la formule

$$IG\delta_1D\delta_2FQE$$

où :

- I est l'isoleucine,
- G est la glycine,
- $\delta_1$ est choisi dans le groupe des acides aminés,
- D est l'acide aspartique, et
- $\delta_2$ est un acide aminé autre que l'alanine.
- F est la phénylalanine
- Q est la glutamine, et
- E est l'acide glutamique

**10.** Un fragment d'acide nucléique selon la revendication 1 ou la revendication 9, dans lequel $\delta_2$ est un acide aminé choisi dans le groupe formé par la thréonine, la sérine, la cystéine, la tyrosine, l'acide aspartique, l'acide glutamique, le tryptophane, l'histidine, la phénylalanine, l'arginine et la lysine.

**11.** Un fragment d'acide nucléique selon la revendication 10, dans lequel $\delta_2$ est la thréonine, la cystéine, l'acide aspartique, l'acide glutamique, le tryptophane ou l'arginine.

**12.** Un fragment d'acide nucléique selon la revendication 1, dans lequel le fragment a une séquence qui code pour une sous-séquence d'acides aminés D ayant la formule

$$P\lambda_1D$$

où :

- P est la proline ;
- $\lambda_1$ est un acide aminé autre que la lysine ; et
- D est l'acide aspartique.

**13.** Un fragment d'acide nucléique selon la revendication 1 ou la revendication 12, dans lequel $\lambda_1$ est un acide-aminé choisi dans le groupe formé par la glycine, l'alanine, la leucine, l'isoleucine, la valine, la thréonine, la sérine, la cystéine, la tyrosine, l'acide glutamique, l'acide aspartique, la proline, l'asparagine, la glutamine, le tryptophane et la phénylalanine.

**14.** Un fragment d'acide nucléique selon la revendication 13, dans lequel $\lambda_1$ est la glycine, la leucine, la thréonine, l'acide glutamique, l'acide aspartique, l'asparagine, le tryptophane ou la proline.

**15.** Un fragment d'acide nucléique selon la revendication 1, dans lequel le fragment a une séquence qui code pour une sous-séquence d'acides aminés G ayant la formule

$$MLG\sigma_1HG$$

où :

- M est la méthionine,
- L est la leucine,
- G est la glycine,
- $\sigma_1$ est un acide aminé autre que la méthionine,
- H est l'histidine, et
- G est la glycine.

**16.** Un fragment d'acide nucléique selon la revendication 1 ou la revendication 15, dans lequel $\sigma_1$ est un acide aminé choisi dans le groupe formé par l'acide glutamique, l'acide aspartique, la proline, la valine, la leucine ou l'isoleucine.

**17.** Un fragment d'acide nucléique selon la revendication 16, dans lequel $\sigma_1$ est la proline, l'acide glutamique ou la valine.

**18.** Un fragment d'acide nucléique selon la revendication 1, dans lequel le fragment a une séquence qui code pour une sous-séquence d'acides aminés E ayant la formule

$$RFD\gamma_1R$$

où :

R est l'arginine,
F est la phénylalanine,
D est l'acide aspartique, et
$\gamma_1$ est un acide aminé autre que l'acide aspartique.

**19.** Un fragment d'acide nucléique selon la revendication 1 ou la revendication 18, dans lequel $\gamma_1$ est un acide aminé choisi dans le groupe formé par la glycine, l'alanine, la valine, la leucine, l'isoleucine, la sérine, la thréonine, la cystéine, la tyrosine, l'acide glutamique, la proline, l'asparagine, la glutamine, la lysine, l'arginine, le tryptophane, l'histidine et la phénylalanine.

**20.** Un fragment d'acide nucléique selon la revendication 19, dans lequel $\gamma_1$ est la glycine, la valine, la sérine, l'acide glutamique, la proline, l'asparagine, la lysine, la cystéine ou le tryptophane.

**21.** Un fragment d'acide nucléique selon la revendication 1, dans lequel le fragment a une séquence qui code pour une sous-séquence d'acides aminés F ayant la formule

$$G\beta_1\beta_8\beta_2Q\beta_3\beta_4\beta_5\beta_6\beta_7$$

où :

| | |
|---|---|
| G | est la glycine, |
| $\beta_1$, $\beta_2$, $\beta_3$, $\beta_4$, $\beta_5$, $\beta_6$ et $\beta_7$ | sont choisis dans le groupe des acides aminés, |
| Q | est la glutamine, et |
| $\beta_8$ | est un acide aminé autre que la valine. |

**22.** Un fragment d'acide nucléique selon la revendication 1 ou la revendication 21, dans lequel $\beta_1$ est la méthionine, $\beta_3$ est le tryptophane, $\beta_7$ est la phénylalanine et $\beta_8$ est un acide aminé autre que la valine.

**23.** Un fragment d'acide nucléique selon l'une quelconque des revendications 1, 21 et 22, dans lequel $\beta_8$ est un acide aminé choisi dans le groupe formé par l'alanine et la glycine.

**24.** Un fragment d'acide nucléique selon la revendication 23, dans lequel $\beta_8$ est l'alanine.

**25.** Un procédé selon la revendication 1, dans lequel le fragment a une séquence qui code pour une sous-séquence d'acides aminés F ayant la formule

$$G\beta_1V\beta_2Q\beta_3\beta_4\beta_5\beta_6\beta_7$$

où :

| | |
|---|---|
| G | est la glycine, |
| $\beta_1$, $\beta_2$, $\beta_4$, $\beta_5$, $\beta_6$ et $\beta_7$ | sont choisis dans le groupe des acides aminés, |
| V | est la valine, |
| Q | est la glutamine, et |
| $\beta_3$ | est un acide aminé autre que le tryptophane ou la proline. |

**26.** Un fragment d'acide nucléique selon la revendication 1, dans lequel le fragment a une séquence qui code pour une sous-séquence d'acides aminés F ayant la formule

$$G\beta_1V\beta_2Q\beta_3\beta_4\beta_5\beta_6\beta_7$$

où :

| | |
|---|---|
| G | est la glycine, |
| $\beta_1$, $\beta_2$, $\beta_3$, $\beta_4$, $\beta_5$ et $\beta_6$ | sont choisis dans le groupe des acides aminés, |
| V | est la valine, |
| Q | est la glutamine, et |
| $\beta_7$ | est un acide aminé autre que la phénylalanine. |

**27.** Un fragment d'acide nucléique selon la revendication 1 ou la revendication 26, dans lequel $\beta_7$ est un acide aminé choisi dans le groupe formé par la leucine, la valine et l'isoleucine.

**28.** Un fragment d'acide nucléique selon la revendication 27, dans lequel $\beta_7$ est la leucine.

**29.** Un procédé pour préparer un fragment d'acide nucléique comprenant une séquence nucléotidique codant pour une acétolactate-synthase, ledit fragment d'acide nucléique conférant une résistance accrue aux herbicides du type sulfonylurée à une plante dans laquelle ledit fragment est fonctionnel, qui consiste à :

a) isoler de bactéries ou levures un fragment d'acide nucléique mutant qui code pour une acétolactate-synthase manifestant une résistance accrue à un ou plusieurs herbicides du type sulfonylurée ;
b) déterminer le(s) ou site(s) de substitution d'acide(s) aminé(s) dans ladite acétolactate-synthase qui confère (nt) la résistance à ladite acétolactate-synthase en comparant la séquence d'ADN dudit fragment d'acide nucléique mutant avec la séquence d'ADN d'un fragment d'acide nucléique de type sauvage qui code pour une acétolactate-synthase sensible aux herbicides du type sulfonylurée ; et
c) introduire une mutation dans un fragment d'acide nucléique qui code pour une acétolactate-synthase qui a une séquence correspondant au moins en partie à un gène d'acétolactate-synthase végétale et qui est fonctionnel dans une plante, ladite mutation ayant pour résultat une substitution d'acide(s) aminé(s) au(x) site (s) qui correspond(ent) audit ou auxdits site(s) de substitution d'acide(s) aminé(s) dans ladite acétolactate-synthase manifestant une résistance accrue aux herbicides du type sulfonylurée.

**30.** Un procédé pour préparer un fragment d'acide nucléique tel que défini dans la revendication 1, qui consiste à :

a) sélectionner une plante mutante résistante aux herbicides du type sulfonylurée ; et
b) isoler le gène mutant d'acétolactate-synthase de la plante mutante.

**31.** Une protéine acétolactate-synthase codée par un fragment d'acide nucléique tel que revendiqué dans l'une quelconque des revendications 1 à 28.

**32.** Un produit de construction d'acide nucléique comprenant un fragment d'acide nucléique tel que revendiqué dans l'une quelconque des revendications 1 à 28.

**33.** Un produit de construction d'acide nucléique selon la revendication 32, choisi parmi les vecteurs de clonage déposés à ATCC, Rockville, Maryland, E.U.A., sous les numéros de dépôt 40237, 67124, 67126 et 67424.

**34.** Un produit de construction d'acide nucléique tel que revendiqué dans la revendication 32, dans lequel un fragment d'acide nucléique tel que revendiqué dans l'une quelconque des revendications 1 à 28 est lié à un second fragment d'acide nucléique confèrant un second caractère de telle sorte que, lorsque ledit produit de construction d'acide nucléique est utilisé pour transformer une plante, l'expression de résistance aux herbicides par ladite plante à l'application de sulfonylurées peut être utilisée pour détecter la présence dudit second fragment d'acide nucléique dans ladite plante.

**35.** Un microorganisme contenant un fragment d'acide nucléique ou un produit de construction d'acide nucléique tel que défini dans l'une quelconque des revendications 1 à 28 ou 32 à 34.

**36.** Une cellule végétale transformée par un fragment d'acide nucléique ou un produit de construction d'acide nucléique tel que défini dans l'une quelconque des revendications 1 à 28 ou 32 à 34.

**37.** Une culture tissulaire végétale transformée par un fragment d'acide nucléique ou un produit de construction d'acide nucléique tel que défini dans l'une quelconque des revendications 1 à 28 ou 32 à 34.

**38.** Un procédé pour transformer une cellule végétale par un fragment d'acide nucléique tel que défini dans l'une quelconque des revendications 1 à 28 ou préparé par un procédé tel que revendiqué dans la revendication 29 ou 30, consistant à introduire dans la cellule végétale un produit de construction d'acide nucléique tel que défini dans l'une quelconque des revendications 32 à 34.

**39.** Un procédé pour sélectionner une cellule végétale transformée par un fragment d'acide nucléique tel que défini dans l'une quelconque des revendications 1 à 28 ou préparé par un procédé tel que revendiqué dans la revendication 29 ou 30, consistant à

introduire un produit de construction d'acide nucléique tel que défini dans l'une quelconque des revendications 32 à 34 dans une cellule végétale dont la croissance est sensible à une inhibition par des herbicides auxquels l'ALS codée par le fragment d'acide nucléique est résistante, pour former une cellule végétale transformée ; et sélectionner une cellule végétale transformée dont la croissance est résistante à l'inhibition par lesdits herbicides à une concentration sélective qui inhibe la croissance des cellules non transformées.

**40.** Un procédé pour combattre la croissance de végétation indésirable poussant en un site où a été cultivée une plante transformée par un fragment d'acide nucléique tel que défini dans l'une quelconque des revendications 1 à 28 ou préparé par un procédé tel que revendiqué dans la revendication 29 ou 30, consistant à appliquer au site une quantité efficace d'herbicide auquel ladite plante est résistante.

**41.** Un procédé selon la revendication 40, dans lequel l'herbicide est un composé choisi dans le groupe formé par

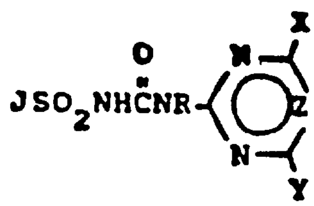

I

où

R est H ou $CH_3$ ;
J est

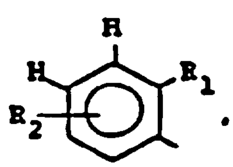

J-1

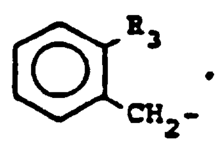

J-2

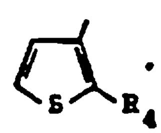

J-3

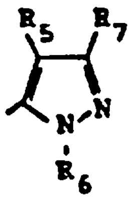 , 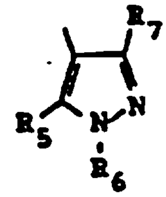 , 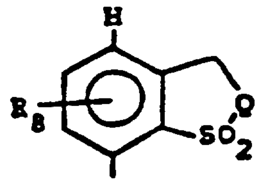

J-4 J-5 J-6

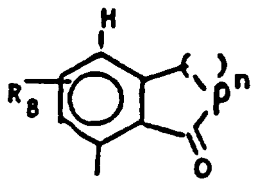 ; 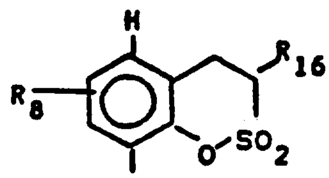 ou , 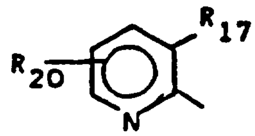 ;

J-7 J-8 J-9

$R_1$ est Cl, Br, $NO_2$, un groupe alkyle en $C_1$-$C_4$, alcényle en $C_2$-$C_4$, $CF_3$, alcoxy en $C_1$-$C_4$, halogénalcoxy en $C_1$-$C_4$, alcényloxy en $C_3$-$C_4$, halogénalcényloxy en $C_2$-$C_4$, alcynyloxy en $C_3$-$C_4$, $CO_2R_9$, $CONR_{10}R_{11}$, $S(O)_mR_{12}$, $OSO_2R_{12}$, phényle, $SO_2N(OCH_3)CH_3$, $SO_2N_{10}R_{11}$,

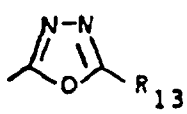 , 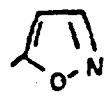 , 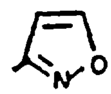 ,

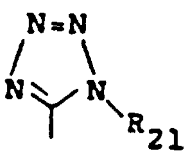 , 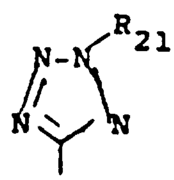 , 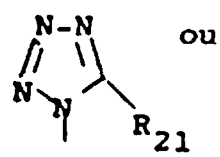 ou 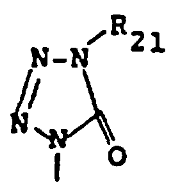

$R_2$ est H, Cl, Br, F, $CH_3$, $NO_2$, $SCH_3$, $OCF_2H$, $OCH_2CF_3$ ou $OCH_3$ ;
$R_3$ est Cl, $NO_2$, $CO_2CH_3$ , $CO_2C_2H_5$, $SO_2N(CH_3)_2$, $SO_2CH_3$ ou $SO_2C_2H_5$ ;
$R_4$ est un groupe alkyle en $C_1$-$C_3$, Cl, Br, $NO_2$, $CO_2R_9$, $CON(CH_3)_2$, $SO_2N(CH_3)_2$, $SO_2N(OCH_3)CH_3$ ou $S(O)_mR_{12}$ ;
$R_5$ est un groupe alkyle en $C_1$-$C_3$, cycloalkylcarbonyle en $C_4$-$C_5$, F, Cl, Br, $NO_2$, $CO_2R_{14}$, $SO_2N(CH_3)_2$, $SO_2R_{12}$ ou phényle ;
$R_6$ est H, un groupe alkyle en $C_1$-$C_3$ ou $CH_2CH{=}CH_2$ ;
$R_7$ est H, $CH_3$, $OCH_3$, Cl ou Br ;
$R_8$ est H, F, Cl, Br, $CH_3$, $OCH_3$, $CF_3$, $SCH_3$ ou $OCF_2H$ ;
$R_9$ est un groupe alkyle en $C_1$-$C_4$, alcényle en $C_3$-$C_4$ ou $CH_2CH_2Cl$ ;
$R_{10}$ est H ou un groupe alkyle en $C_1$-$C_3$ ;
$R_{11}$ est H ou un groupe alkyle en $C_1$-$C_2$ ;
$R_{12}$ est un groupe alkyle en $C_1$-$C_3$ ;
$R_{13}$ est H ou $CH_3$ ;
$R_{14}$ est un groupe alkyle en $C_1$-$C_3$ ou $CH_2CH{=}CH_2$ ; m est 0, 1 ou 2 ;
n est 1 ou 2 ;
Q est $CH_2$, $CHCH_3$ ou $NR_{15}$ ;

$R_{15}$ est H ou un groupe alkyle en $C_1$-$C_4$ ;
P est O ou $CH_2$ ;
$R_{16}$ est H ou $CH_3$ ;
$R_{17}$ est $C(O)NR_{18}R_{19}$ ;
$R_{18}$ est H ou $CH_3$ ;
$R_{19}$ est $CH_3$ ;
$R_{20}$ est H, Cl, F, Br, $CH_3$, $CF_3$, $OCH_3$ ou $OCF_2H$ ;
$R_{21}$ est H ou $CH_3$ ;
X est $CH_3$, $OCH_3$, $OC_2H_5$ ou $NHCH_3$ ;
Y est $CH_3$, $C_2H_5$, $OCH_3$, $OC_2H_5$, $OCF_2H$, $OCH_2CF_3$, Cl, $CH_2OCH_3$ ou un groupe cyclopropyle ;
Z est CH ou N ;

et leurs sels utilisables en agriculture ;
avec les conditions suivantes :

a) si Y est Cl, alors Z est CH et X est $OCH_3$ ;
b) si Y est $OCF_2H$, alors Z est CH ;
c) si J est J-1 et $R_1$ est $OSO_2R_{12}$ ou un groupe phényle, alors Y est autre chose que $OCF_2H$ ;
d) si J est J-2, alors Y est autre chose que $OCF_2H$ ou $OCH_2CF_3$ ; et
e) si J est J-3 et $R_4$ est $S(O)_mR_{12}$, alors Y est autre chose que $OCH_2CF_3$.

**42.** Un procédé selon la revendication 40, dans lequel l'herbicide est un composé choisi dans le groupe formé par

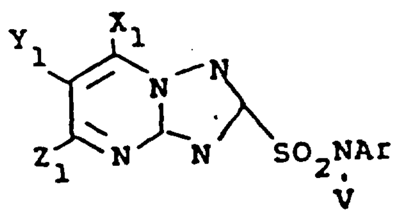

où

Ar est

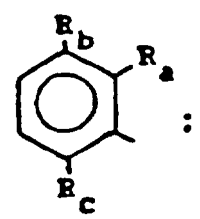

$R_a$ est un groupe alkyle en $C_1$-$C_4$, F, Cl, Br, I, $NO_2$, $S(O)_pR_d$, $COOR_e$ ou $CF_3$ ;
$R_b$ est H, F, Cl, Br, I, un groupe alkyle en $C_1$-$C_4$ ou $COOR_e$ ;
$R_c$ est H, un groupe alkyle en $C_1$-$C_4$, F, Cl, Br, I, $CH_2OR_d$, phényle, $NO_2$ ou $COOR_e$ ;
$R_d$ est un groupe alkyle en $C_1$-$C_4$ ;
$R_e$ est un groupe alkyle, alcényle, alcynyle, chacun ayant jusqu'à 4 atomes de carbone, ou 2-éthoxyéthyle ;
V est H, un groupe alkyle en $C_1$-$C_3$, allyle, propargyle, benzyle ou (alkyle en $C_1$-$C_3$)-carbonyle ;
$X_1$, $Y_1$ et $Z_1$ sont indépendamment H, F, Cl, Br, I, un groupe alkyle en $C_1$-$C_5$, alkylthio en $C_1$-$C_2$ ou alcoxy en $C_1$-$C_4$ ; et
p est 0, 1 ou 2.

**43.** Un procédé selon la revendication 40, dans lequel l'herbicide est un composé choisi dans le groupe formé par

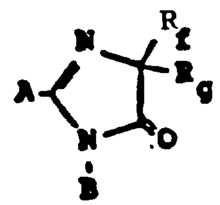

III

où

A est

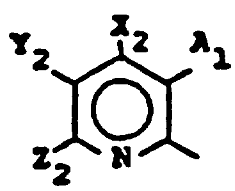

,

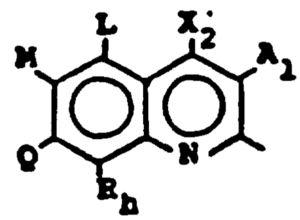

ou

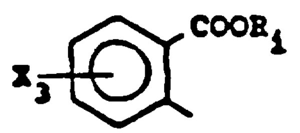

;

| | |
|---|---|
| $R_f$ | est un groupe alkyle en $C_1$-$C_4$ ; |
| $R_g$ | est un groupe alkyle en $C_1$-$C_4$ ou cycloalkyle en $C_3$-$C_6$ ; |
| $A_1$ | est $COOR_i$, $CH_2OH$ ou CHO ; |
| $R_i$ | est H ; un groupe alkyle en $C_1$-$C_{12}$ facultativement substitué par un groupe alkyle en $C_1$-$C_3$, cycloalkyle en $C_3$-$C_6$ ou phényle ; un groupe alcényle en $C_3$-$C_5$ facultativement substitué par un groupe phényle ou 1 ou 2 groupes alkyles en $C_1$-$C_3$, F, Cl, Br ou I ; ou un groupe alcynyle en $C_3$-$C_5$ facultativement substitué par un groupe phényle ou 1 ou 2 groupes alkyles en $C_1$-$C_3$, F, Cl, Br ou I ; |
| B | est H ; un groupe C(O)alkyle en $C_1$-$C_6$ ou C(O)-phényle facultativement substitué par Cl, $NO_2$ ou $OCH_3$ ; |
| $X_2$ | est H, F, Cl, Br, I, OH ou $CH_3$ ; |
| $Y_2$ et $Z_2$ | sont indépendamment H, un groupe alkyle en $C_1$-$C_6$, alcoxy en $C_1$-$C_6$, F, Cl, Br, I, phényle, $NO_2$, CN, $CF_3$ ou $SO_2CH_3$ ; |
| $X_3$ | est H, un groupe alkyle en $C_1$-$C_3$, F, Cl, Br, I ou $NO_2$ ; et |
| L, M, Q et $R_h$ | sont indépendamment H, F, Cl, Br, I, $CH_3$, $OCH_3$, $NO_2$, $CF_3$, CN, N $(CH_3)_2$, $NH_2$, $SCH_3$ ou $SO_2CH_3$, à condition qu'un seul de M et Q puisse être un substituant autre que H, F, Cl, Br, I, $CH_3$ ou $OCH_3$. |

**44.** Un procédé pour préparer une plante contenant un fragment d'acide nucléique tel que défini dans l'une quelconque des revendications 1 à 28 ou préparé par un procédé tel que revendiqué dans la revendication 29 ou la revendication 30, qui consiste à transformer une cellule végétale avec un fragment d'acide nucléique ou un produit de construction d'acide nucléique tel que défini dans l'une quelconque des revendications 1 à 28 ou 32 à 34, ou préparé par un procédé tel que revendiqué dans la revendication 29 ou la revendication 30, régénérer des plantes transformées à partir de la cellule végétale transformée et, facultativement, répliquer biologiquement les plantes transformées.

**45.** Un procédé tel que revendiqué dans la revendication 44, dans lequel la plante est du soja, du maïs, de la betterave à sucre, du tournesol, du tabac, de la pomme de terre, de la tomate, de la luzerne, du melon, du cotonnier, de la laitue, du blé ou du riz ou une espèce du genre *Brassica.*

**46.** Un procédé pour produire une graine contenant un fragment d'acide nucléique tel que défini dans l'une quelconque des revendications 1 à 28 ou préparé par un procédé tel que revendiqué dans la revendication 29 ou la revendication 30, qui consiste à transformer une cellule végétale avec un fragment d'acide nucléique ou un produit de construction d'acide nucléique tel que défini dans l'une quelconque des revendications 1 à 28 ou 32 à 34 ou préparé par un procédé tel que revendiqué dans la revendication 29 ou la revendication 30, régénérer des plantes transformées à partir de la cellule végétale transformée, facultativement répliquer biologiquement les plantes transformées et obtenir la graine à partir des plantes transformées.

**47.** Une plante contenant un fragment d'acide nucléique ou un produit de construction d'acide nucléique tel que défini dans l'une quelconque des revendications 1 à 28 ou 32 à 34.

**48.** Une plante telle que revendiquée dans la revendication 47, qui est du soja, du maïs, de la betterave à sucre, du tournesol, du tabac, de la pomme de terre, de la tomate, de la luzerne, du melon, du cotonnier, de la laitue, du blé ou du riz ou une espèce du genre *Brassica.*

**49.** Graine contenant un fragment d'acide nucléique ou un produit de construction d'acide nucléique tel que défini dans l'une quelconque des revendications 1 à 28 ou 32 à 34, obtenue par croissance d'une plante telle que revendiquée dans la revendication 47 ou la revendication 48.

**Revendications pour les Etats contractants suivants : AT, ES**

**1.** Un procédé de préparation d'un fragment d'acide nucléique comprenant une séquence nucléotidique codant pour une acétolactate-synthase, ledit fragment d'acide nucléique conférant une résistance accrue aux herbicides du type sulfonylurée à une plante dans laquelle ledit fragment est fonctionnel, dans lequel une séquence nucléotidique qui code pour une acétolactate-synthase qui a une séquence correspondant, au moins en partie, à un gène d'acétolactate-synthase végétale et est fonctionnelle dans une plante, et qui comprend au moins une sous-séquence codant pour l'une des sous-séquences d'acides aminés sensiblement conservées désignées par A, B, C, D, E, F et G sur la Figure 6, est amenée à muter de telle manière qu'au moins l'une des conditions suivantes soit satisfaite :

a) le fragment d'acide nucléique a une séquence qui code pour une sous-séquence d'acides aminés A dans laquelle $\varepsilon_1$ est un acide aminé autre l'alanine et/ou $\varepsilon_2$ est un acide aminé autre que la glycine,
b) le fragment d'acide nucléique a une séquence qui code pour une sous-séquence d'acides aminés B dans laquelle $\alpha_1$ est un acide aminé autre que la proline,
c) le fragment d'acide nucléique a une séquence qui code pour une sous-séquence d'acides aminés C dans laquelle $\delta_2$ est un acide aminé autre que l'alanine,
d) le fragment d'acide nucléique a une séquence qui code pour une sous-séquence d'acides aminés D dans laquelle $\lambda_1$ est un acide aminé autre que la lysine,
e) le fragment d'acide nucléique a une séquence qui code pour une sous-séquence d'acides aminés E dans laquelle $\gamma_1$ est un acide aminé autre que l'acide aspartique,
f) le fragment d'acide nucléique a une séquence qui code pour une sous-séquence d'acides aminés F dans laquelle $\beta_3$ est un acide aminé autre que le tryptophane et/ou $\beta_8$ est un acide aminé autre que la valine et/ou $\beta_7$ est un acide aminé autre que la phénylalanine, et
g) le fragment d'acide nucléique a une séquence qui code pour une sous-séquence d'acides aminés G dans laquelle $\sigma_1$ est un acide aminé autre que la méthionine.

**2.** Un procédé selon la revendication 1, dans lequel le fragment a une séquence qui code pour une sous-séquence d'acides aminés A ayant la formule

$$PG\varepsilon_2A$$

où :

P est la proline,
G est la glycine,
$\varepsilon_2$ est un acide aminé autre que la glycine, et
A est l'alanine.

**3.** Un procédé selon la revendication 1 ou la revendication 2, dans lequel $\varepsilon_2$ est un acide aminé choisi dans le groupe formé par la sérine, la thréonine et la cystéine.

**4.** Un procédé selon la revendication 3, dans lequel $\varepsilon_2$ est la sérine.

**5.** Un procédé selon la revendication 1, dans lequel le fragment a une séquence qui code pour une sous-séquence d'acides aminés A ayant la formule

$$PGG\varepsilon_1$$

où :

P est la proline,
G est la glycine, et
$\varepsilon_1$ est un acide aminé autre que l'alanine ou la glycine.

**6.** Un procédé selon la revendication 1, dans lequel le fragment a une séquence qui code pour une sous-séquence d'acides aminés B ayant la formule

$$GQV\alpha_1$$

où :

G est la glycine,
Q est la glutamine,
V est la valine,
$\alpha_1$ est un acide aminé autre que la proline.

**7.** Un procédé selon la revendication 1 ou la revendication 6, dans lequel $\alpha_1$ est un acide aminé choisi dans le groupe formé par l'alanine, la glycine, l'arginine, la lysine, l'histidine, la sérine, la cystéine, la thréonine, la glutamine et l'asparagine.

**8.** Un procédé selon la revendication 7, dans lequel $\alpha_1$ est l'alanine, l'arginine, la sérine ou la glutamine.

**9.** Un procédé selon la revendication 1, dans lequel le fragment a une séquence qui code pour une sous-séquence d'acides aminés C ayant la formule

$$IG\delta_1 D\delta_2 FQE$$

où :

I est l'isoleucine,
G est la glycine,
$\delta_1$ est choisi dans le groupe des acides aminés,
D est l'acide aspartique, et
$\delta_2$ est un acide aminé autre que l'alanine.
F est la phénylalanine
Q est la glutamine, et
E est l'acide glutamique

**10.** Un procédé selon la revendication 1 ou la revendication 9, dans lequel $\delta_2$ est un acide aminé choisi dans le groupe formé par la thréonine, la sérine, la cystéine, la tyrosine, l'acide aspartique, l'acide glutamique, le tryptophane, l'histidine, la phénylalanine, l'arginine et la lysine.

**11.** Un procédé selon la revendication 10, dans lequel $\delta_2$ est la thréonine, la cystéine, l'acide aspartique, l'acide glutamique, le tryptophane ou l'arginine.

**12.** Un procédé selon la revendication 1, dans lequel le fragment a une séquence qui code pour une sous-séquence d'acides aminés D ayant la formule

$$P\lambda_1 D$$

où :

P est la proline ;
$\lambda_1$ est un acide aminé autre que la lysine ; et
D est l'acide aspartique.

**13.** Un procédé selon la revendication 1 ou la revendication 12, dans lequel $\lambda_1$ est un acide aminé choisi dans le groupe formé par la glycine, l'alanine, la leucine, l'isoleucine, la valine, la thréonine, la sérine, la cystéine, la tyrosine, l'acide glutamique, l'acide aspartique, la proline, l'asparagine, la glutamine, le tryptophane et la phénylalanine.

**14.** Un procédé selon la revendication 13, dans lequel $\lambda_1$ est la glycine, la leucine, la thréonine, l'acide glutamique, l'acide aspartique, l'asparagine, le tryptophane ou la proline.

**15.** Un procédé selon la revendication 1, dans lequel le fragment a une séquence qui code pour une sous-séquence d'acides aminés G ayant la formule

$$MLG\sigma_1 HG$$

où :

M est la méthionine,
L est la leucine,
G est la glycine,
$\sigma_1$ est un acide aminé autre que la méthionine,
H est l'histidine, et
G est la glycine.

**16.** Un procédé selon la revendication 1 ou la revendication 15, dans lequel $\sigma_1$ est un acide aminé choisi dans le groupe formé par l'acide glutamique, l'acide aspartique, la proline, la valine, la leucine ou l'isoleucine.

**17.** Un procédé selon la revendication 16, dans lequel $\sigma_1$ est la proline, l'acide glutamique ou la valine.

**18.** Un procédé selon la revendication 1, dans lequel le fragment a une séquence qui code pour une sous-séquence d'acides aminés E ayant la formule

$$RFD\gamma_1 R$$

où :

R est l'arginine,
F est la phénylalanine,
D est l'acide aspartique, et
$\gamma_1$ est un acide aminé autre que l'acide aspartique.

**19.** Un procédé selon la revendication 1 ou la revendication 18, dans lequel $\gamma_1$ est un acide aminé choisi dans le groupe formé par la glycine, l'alanine, la valine, la leucine, l'isoleucine, la sérine, la thréonine, la cystéine, la tyrosine, l'acide glutamique, la proline, l'asparagine, la glutamine, la lysine, l'arginine, le tryptophane, l'histidine et la phénylalanine.

**20.** Un procédé selon la revendication 19, dans lequel $\gamma_1$ est la glycine, la valine, la sérine, l'acide glutamique, la proline, l'asparagine, la lysine, la cystéine ou le tryptophane.

**21.** Un procédé selon la revendication 1, dans lequel le fragment a une séquence qui code pour une sous-séquence d'acides aminés F ayant la formule

$$G\beta_1\beta_8\beta_2Q\beta_3\beta_4\beta_5\beta_6\beta_7$$

où :

| | |
|---|---|
| G | est la glycine, |
| $\beta_1$, $\beta_2$, $\beta_3$, $\beta_4$, $\beta_5$, $\beta_6$ et $\beta_7$ | sont choisis dans le groupe des acides aminés, |
| Q | est la glutamine, et |
| $\beta_8$ | est un acide aminé autre que la valine. |

**22.** Un procédé selon la revendication 1 ou la revendication 21, dans lequel $\beta_1$ est la méthionine, $\beta_3$ est le tryptophane, $\beta_7$ est la phénylalanine et $\beta_8$ est un acide aminé autre que la valine.

**23.** Un procédé selon l'une quelconque des revendications 1, 21 et 22, dans lequel $\beta_8$ est un acide aminé choisi dans le groupe formé par l'alanine et la glycine.

**24.** Un procédé selon la revendication 23, dans lequel $\beta_8$ est l'alanine.

**25.** Un procédé selon la revendication 1, dans lequel le fragment a une séquence qui code pour une sous-séquence d'acides aminés F ayant la formule

$$G\beta_1V\beta_2Q\beta_3\beta_4\beta_5\beta_6\beta_7$$

où :

| | |
|---|---|
| G | est la glycine, |
| $\beta_1$, $\beta_2$, $\beta_4$, $\beta_5$, $\beta_6$ et $\beta_7$ | sont choisis dans le groupe des acides aminés, |
| V | est la valine, |
| Q | est la glutamine, et |
| $\beta_3$ | est un acide aminé autre que le tryptophane ou la proline. |

**26.** Un procédé selon la revendication 1, dans lequel le fragment a une séquence qui code pour une sous-séquence d'acides aminés F ayant la formule

$$G\beta_1V\beta_2Q\beta_3\beta_4\beta_5\beta_6\beta_7$$

où :

| | |
|---|---|
| G | est la glycine, |
| $\beta_1$, $\beta_2$, $\beta_3$, $\beta_4$, $\beta_5$ et $\beta_6$ | sont choisis dans le groupe des acides aminés, |
| V | est la valine, |
| Q | est la glutamine, et |
| $\beta_7$ | est un acide aminé autre que la phénylalanine. |

**27.** Un procédé selon la revendication 1 ou la revendication 26, dans lequel $\beta_7$ est un acide aminé choisi dans le

groupe formé par la leucine, la valine et l'isoleucine.

**28.** Un procédé selon la revendication 27, dans lequel $\beta_7$ est la leucine.

**29.** Un procédé pour préparer un fragment d'acide nucléique comprenant une séquence nucléotidique codant pour une acétolactate-synthase, ledit fragment d'acide nucléique conférant une résistance accrue aux herbicides du type sulfonylurée à une plante dans laquelle ledit fragment est fonctionnel, qui consiste à :

a) isoler de bactéries ou levures un fragment d'acide nucléique mutant qui code pour une acétolactate-synthase manifestant une résistance accrue à un ou plusieurs herbicides du type sulfonylurée ;
b) déterminer le(s) ou site(s) de substitution d'acide(s) aminé(s) dans ladite acétolactate-synthase qui confère(nt) la résistance à ladite acétolactate-synthase en comparant la séquence d'ADN dudit fragment d'acide nucléique mutant avec la séquence d'ADN d'un fragment d'acide nucléique de type sauvage qui code pour une acétolactate-synthase sensible aux herbicides du type sulfonylurée ; et
c) introduire une mutation dans un fragment d'acide nucléique qui code pour une acétolactate-synthase qui a une séquence correspondant au moins en partie à un gène d'acétolactate-synthase végétale et qui est fonctionnel dans une plante, ladite mutation ayant pour résultat une substitution d'acide(s) aminé(s) au(x) site(s) qui correspond(ent) audit ou auxdits site(s) de substitution d'acide(s) aminé(s) dans ladite acétolactate-synthase manifestant une résistance accrue aux herbicides du type sulfonylurée.

**30.** Un procédé pour préparer un fragment d'acide nucléique tel que défini dans la revendication 1, qui consiste à :

a) sélectionner une plante mutante résistante aux herbicides du type sulfonylurée ; et
b) isoler le gène mutant d'acétolactate-synthase de la plante mutante.

**31.** Un procédé pour préparer un produit de construction d'acide nucléique comprenant un fragment d'acide nucléique préparé par un procédé tel que revendiqué dans l'une quelconque des revendications 1 à 30, qui consiste à lier ledit fragment d'acide nucléique à un second fragment d'acide nucléique.

**32.** Un procédé tel que revendiqué dans la revendication 31, dans lequel ledit second fragment d'acide nucléique confère un second caractère de telle sorte que, lorsque ledit produit de construction d'acide nucléique est utilisé pour transformer une plante, l'expression de résistance aux herbicides par ladite plante à l'application de sulfonylurées peut être utilisée pour détecter la présence dudit second fragment d'acide nucléique dans ladite plante.

**33.** Un produit de construction d'acide nucléique choisi parmi les vecteurs de clonage déposés à ATCC, Rockville, Maryland, E.U.A., sous les numéros de dépôt 40237, 67124, 67126 et 67424.

**34.** Un procédé pour préparer une acétolactate-synthase végétale ayant une résistance accrue à un ou plusieurs herbicides du type sulfonylurée, qui consiste à faire s'exprimer une séquence codant pour une acétolactate-synthase végétale présente dans un fragment d'acide nucléique ou un produit de construction d'acide nucléique tel que défini dans l'une quelconque des revendications 1 à 28 ou 31 à 33 ou préparé par un procédé tel que revendiqué dans la revendication 29 ou 30.

**35.** Un microorganisme contenant un fragment d'acide nucléique ou un produit de construction d'acide nucléique tel que défini dans l'une quelconque des revendications 1 à 28 ou 31 à 33.

**36.** Une cellule végétale transformée par un fragment d'acide nucléique ou un produit de construction d'acide nucléique tel que défini dans l'une quelconque des revendications 1 à 28 ou 31 à 33.

**37.** Une culture tissulaire végétale transformée par un fragment d'acide nucléique ou un produit de construction d'acide nucléique tel que défini dans l'une quelconque des revendications 1 à 28 ou 31 à 33.

**38.** Un procédé pour transformer une cellule végétale par un fragment d'acide nucléique tel que défini dans l'une quelconque des revendications 1 à 28 ou préparé par un procédé tel que revendiqué dans la revendication 29 ou 30, consistant à introduire dans la cellule végétale un produit de construction d'acide nucléique tel que défini dans l'une quelconque des revendications 31 à 33.

**39.** Un procédé pour sélectionner une cellule végétale transformée par un fragment d'acide nucléique tel que défini

dans l'une quelconque des revendications 1 à 28 ou préparé par un procédé tel que revendiqué dans la revendication 29 ou 30, consistant à

introduire un produit de construction d'acide nucléique tel que défini dans l'une quelconque des revendications 31 à 33 dans une cellule végétale dont la croissance est sensible à une inhibition par des herbicides auxquels l'ALS codée par le fragment d'acide nucléique est résistante, pour former une cellule végétale transformée ; et sélectionner une cellule végétale transformée dont la croissance est résistante à l'inhibition par lesdits herbicides à une concentration sélective qui inhibe la croissance des cellules non transformées.

**40.** Un procédé pour combattre la croissance de végétation indésirable poussant en un site où a été cultivée une plante transformée par un fragment d'acide nucléique tel que défini dans l'une quelconque des revendications 1 à 28 ou préparé par un procédé tel que revendiqué dans la revendication 29 ou 30, consistant à appliquer au site une quantité efficace d'herbicide auquel ladite plante est résistante.

**41.** Un procédé selon la revendication 40, dans lequel l'herbicide est un composé choisi dans le groupe formé par

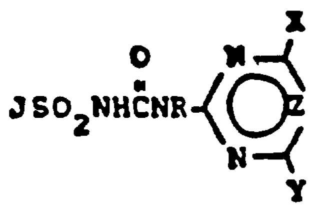

I

où

R est H ou $CH_3$ ;
J est

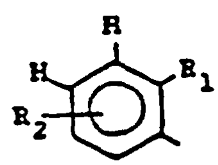 , 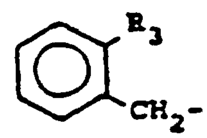 , 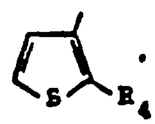 ,

J-1 J-2 J-3

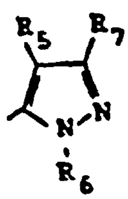 , 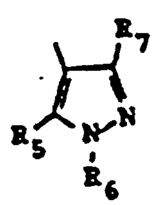 , 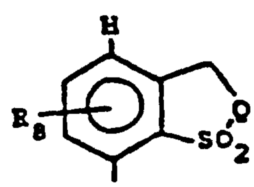

J-4 J-5 J-6

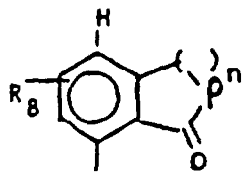 ; 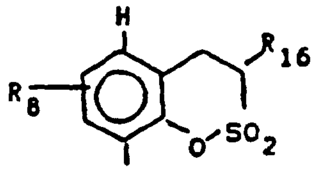 ou 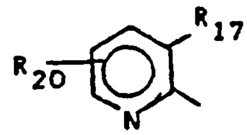 ;

J-7 J-8 J-9

$R_1$ est Cl, Br, $NO_2$, un groupe alkyle en $C_1$-$C_4$, alcényle en $C_2$-$C_4$, $CF_3$, alcoxy en $C_1$-$C_4$, halogénalcoxy en $C_1$-$C_4$, alcényloxy en $C_3$-$C_4$, halogénalcényloxy en $C_2$-$C_4$, alcynyloxy en $C_3$-$C_4$, $CO_2R_9$, $CONR_{10}R_{11}$, $S(O)_mR_{12}$, $OSO_2R_{12}$, phényle, $SO_2N(OCH_3)CH_3$, $SO_2NR_{10}R_{11}$,

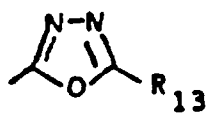 , 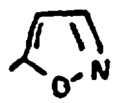 , 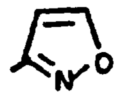 ,

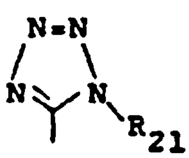 , 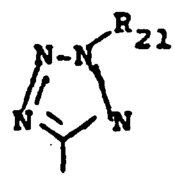 , 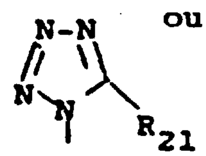 ou 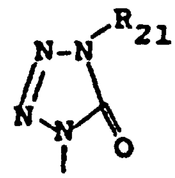

$R_2$ est H, Cl, Br, F, $CH_3$, $NO_2$, $SCH_3$, $OCF_2H$, $OCH_2CF_3$ ou $OCH_3$ ;
$R_3$ est Cl, $NO_2$, $CO_2CH_3$, $CO_2C_2H_5$, $SO_2N(CH_3)_2$, $SO_2CH_3$ ou $SO_2C_2H_5$ ;
$R_4$ est un groupe alkyle en $C_1$-$C_3$, Cl, Br, $NO_2$, $CO_2R_9$, $CON(CH_3)_2$, $SO_2N(CH_3)_2$, $SO_2N(OCH_3)CH_3$ ou $S(O)_mR_{12}$;
$R_5$ est un groupe alkyle en $C_1$-$C_3$, cycloalkylcarbonyle en $C_4$-$C_5$, F, Cl, Br, $NO_2$, $CO_2R_{14}$, $SO_2N(CH_3)_2$, $SO_2R_{12}$ ou phényle ;
$R_6$ est H, un groupe alkyle en $C_1$-$C_3$ ou $CH_2CH=CH_2$ ;
$R_7$ est H, $CH_3$, $OCH_3$, Cl ou Br ;
$R_8$ est H, F, Cl, Br, $CH_3$, $OCH_3$, $CF_3$, $SCH_3$ ou $OCF_2H$ ;
$R_9$ est un groupe alkyle en $C_1$-$C_4$, alcényle en $C_3$-$C_4$ ou $CH_2CH_2Cl$ ;
$R_{10}$ est H ou un groupe alkyle en $C_1$-$C_3$ ;
$R_{11}$ est H ou un groupe alkyle en $C_1$-$C_2$ ;
$R_{12}$ est un groupe alkyle en $C_1$-$C_3$ ;
$R_{13}$ est H ou $CH_3$ ;
$R_{14}$ est un groupe alkyle en $C_1$-$C_3$ ou $CH_2CH=CH_2$ ;
m est 0, 1 ou 2 ;
n est 1 ou 2 ;
Q est $CH_2$, $CHCH_3$ ou $NR_{15}$ ;
$R_{15}$ est H ou un groupe alkyle en $C_1$-$C_4$ ;
P est O ou $CH_2$ ;
$R_{16}$ est H ou $CH_3$ ;
$R_{17}$ est $C(O)NR_{18}R_{19}$ ;
$R_{18}$ est H ou $CH_3$ ;
$R_{19}$ est $CH_3$ ;
$R_{20}$ est H, Cl, F, Br, $CH_3$, $CF_3$, $OCH_3$ ou $OCF_2H$ ;
$R_{21}$ est H ou $CH_3$ ;
X est $CH_3$, $OCH_3$, $OC_2H_5$ ou $NHCH_3$ ;
Y est $CH_3$, $C_2H_5$, $OCH_3$, $OC_2H_5$, $OCF_2H$, $OCH_2CF_3$, Cl, $CH_2OCH_3$ ou un groupe cyclopropyle ;
Z est CH ou N ;

et leurs sels utilisables en agriculture ;
avec les conditions suivantes :

a) si Y est Cl, alors Z est CH et X est $OCH_3$ ;
b) si Y est $OCF_2H$, alors Z est CH ;
c) si J est J-1 et $R_1$ est $OSO_2R_{12}$ ou un groupe phényle, alors Y est autre chose que $OCF_2H$ ;
d) si J est J-2, alors Y est autre chose que $OCF_2H$ ou $OCH_2CF_3$ ; et
e) si J est J-3 et $R_4$ est $S(O)_mR_{12}$, alors Y est autre chose que $OCH_2CF_3$.

**42.** Un procédé selon la revendication 40, dans lequel l'herbicide est un composé choisi dans le groupe formé par

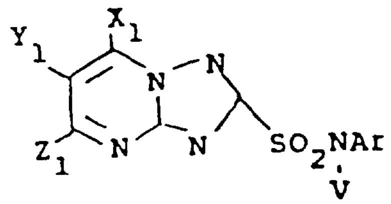

où

| | |
|---|---|
| Ar | est |

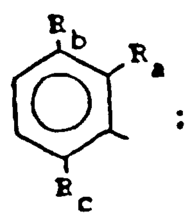

| | |
|---|---|
| $R_a$ | est un groupe alkyle en $C_1$-$C_4$, F, Cl, Br, I, $NO_2$, $S(O)_pR_d$, $COOR_e$ ou $CF_3$ ; |
| $R_b$ | est H, F, Cl, Br, I, un groupe alkyle en $C_1$-$C_4$ ou $COOR_e$ ; |
| $R_c$ | est H, un groupe alkyle en $C_1$-$C_4$, F, Cl, Br, I, $CH_2OR_d$, phényle, $NO_2$ ou $COOR_e$ ; |
| $R_d$ | est un groupe alkyle en $C_1$-$C_4$ ; |
| $R_e$ | est un groupe alkyle, alcényle, alcynyle, chacun ayant jusqu'à 4 atomes de carbone, ou 2-éthoxyéthyle ; |
| V | est H, un groupe alkyle en $C_1$-$C_3$, allyle, propargyle, benzyle ou (alkyle en $C_1$-$C_3$)carbonyle ; |
| $X_1$, $Y_1$ et $Z_1$ | sont indépendamment H, F, Cl, Br, I, un groupe alkyle en $C_1$-$C_4$, alkylthio en $C_1$-$C_2$ ou alcoxy en $C_1$-$C_4$ ; et |
| p | est 0, 1 ou 2. |

**43.** Un procédé selon la revendication 40, dans lequel l'herbicide est un composé choisi dans le groupe formé par

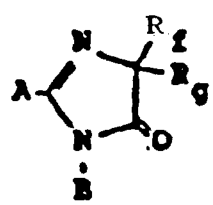

III

où

A est

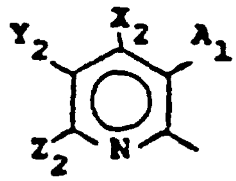

,

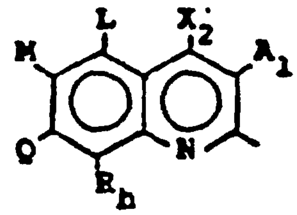

ou

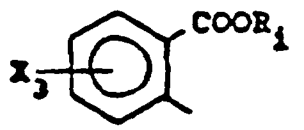

;

| | |
|---|---|
| $R_f$ | est un groupe alkyle en $C_1$-$C_4$ ; |
| $R_g$ | est un groupe alkyle en $C_1$-$C_4$ ou cycloalkyle en $C_3$-$C_6$ ; |
| $A_1$ | est $COOR_i$, $CH_2OH$ ou CHO ; |
| $R_i$ | est H ; un groupe alkyle en $C_1$-$C_{12}$ facultativement substitué par un groupe alkyle en $C_1$-$C_3$, cycloalkyle en $C_3$-$C_6$ ou phényle ; un groupe alcényle en $C_3$-$C_5$ facultativement substitué par un groupe phényle ou 1 ou 2 groupes alkyles en $C_1$-$C_3$, F, Cl, Br ou I ; ou un groupe alcynyle en $C_3$-$C_5$ facultativement substitué par un groupe phényle ou 1 ou 2 groupes alkyles en $C_1$-$C_3$, F, Cl, Br ou I ; |
| B | est H ; un groupe C(O)alkyle en $C_1$-$C_6$ ou C(O)phényle facultativement substitué par Cl, $NO_2$ ou $OCH_3$ ; |
| $X_2$ | est H, F, Cl, Br, I, OH ou $CH_3$ ; |
| $Y_2$ et $Z_2$ | sont indépendamment H, un groupe alkyle en $C_1$-$C_6$, alcoxy en $C_1$-$C_6$B F, Cl, Br, I, phényle, $NO_2$, CN, $CF_3$ ou $SO_2CH_3$ ; |
| $X_3$ | est H, un groupe alkyle en $C_1$-$C_3$, F, Cl, Br, I ou $NO_2$ ; et |
| L, M, Q et $R_h$ | sont indépendamment H, F, Cl, Br, I, $CH_3$, $OCH_3$, $NO_2$, $CF_3$, CN, $N(CH_3)_2$, $NH_2$, $SCH_3$ ou $SO_2CH_3$, à condition qu'un seul de M et Q puisse être un substituant autre que H, F, Cl, Br, I, $CH_3$ ou $OCH_3$. |

**44.** Un procédé pour préparer une plante contenant un fragment d'acide nucléique tel que défini dans l'une quelconque des revendications 1 à 28 ou préparé par un procédé tel que revendiqué dans la revendication 29 ou la revendication 30, qui consiste à transformer une cellule végétale avec un fragment d'acide nucléique ou un produit de construction d'acide nucléique tel que défini dans l'une quelconque des revendications 1 à 28 ou 31 à 33, ou préparé par un procédé tel que revendiqué dans la revendication 29 ou la revendication 30, régénérer des plantes transformées à partir de la cellule végétale transformée et, facultativement, répliquer biologiquement les plantes transformées.

**45.** Un procédé tel que revendiqué dans la revendication 44, dans lequel la plante est du soja, du maïs, de la betterave à sucre, du tournesol, du tabac, de la pomme de terre, de la tomate, de la luzerne, du melon, du cotonnier, de la laitue, du blé ou du riz ou une espèce du genre *Brassica.*

**46.** Un procédé pour produire une graine contenant un fragment d'acide nucléique tel que défini dans l'une quelconque des revendications 1 à 28 ou préparé par un procédé tel que revendiqué dans la revendication 29 ou la revendication 30, qui consiste à transformer une cellule végétale avec un fragment d'acide nucléique ou un produit de construction d'acide nucléique tel que défini dans l'une quelconque des revendications 1 à 28 ou 31 à 33 ou préparé par un procédé tel que revendiqué dans la revendication 29 ou la revendication 30, régénérer des plantes transformées à partir de la cellule végétale transformée, facultativement répliquer biologiquement les plantes transformées et obtenir la graine à partir des plantes transformées.

**47.** Une plante contenant un fragment d'acide nucléique ou un produit de construction d'acide nucléique tel que défini dans l'une quelconque des revendications 1 à 28 ou 31 à 33.

**48.** Une plante telle que revendiquée dans la revendication 47, qui est du soja, du maïs, de la betterave à sucre, du tournesol, du tabac, de la pomme de terre, de la tomate, de la luzerne, du melon, du cotonnier, de la laitue, du blé ou du riz ou une espèce du genre *Brassica.*

**49.** Graine contenant un fragment d'acide nucléique ou un produit de construction d'acide nucléique tel que défini dans l'une quelconque des revendications 1 à 28 ou 31 à 33, obtenue par croissance d'une plante telle que revendiquée dans la revendication 47 ou la revendication 48.

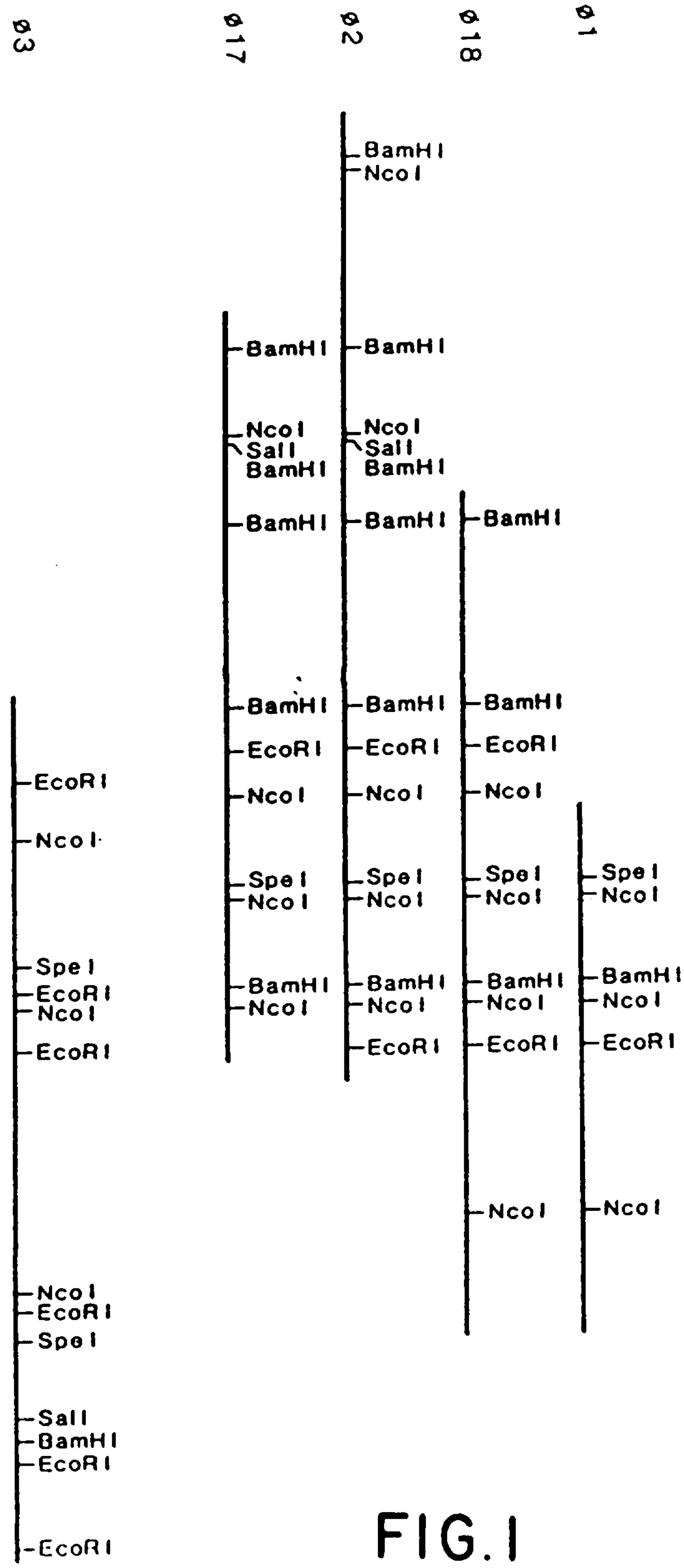
ø3
EcoR I
Nco I
Spe I
EcoR I
Nco I
EcoR I
Nco I
EcoR I
Spe I
Sal I
BamH I
EcoR I
EcoR I
ø17
BamH I
Nco I
Sal I
BamH I
BamH I
BamH I
EcoR I
Nco I
Spe I
Nco I
BamH I
Nco I
ø2
BamH I
Nco I
BamH I
Nco I
Sal I
BamH I
BamH I
BamH I
EcoR I
Nco I
Spe I
Nco I
BamH I
Nco I
EcoR I
ø18
BamH I
BamH I
EcoR I
Nco I
Spe I
Nco I
BamH I
Nco I
EcoR I
Nco I
ø1
Spe I
Nco I
BamH I
Nco I
EcoR I
Nco I

FIG.1

FIG.2

pAGS152

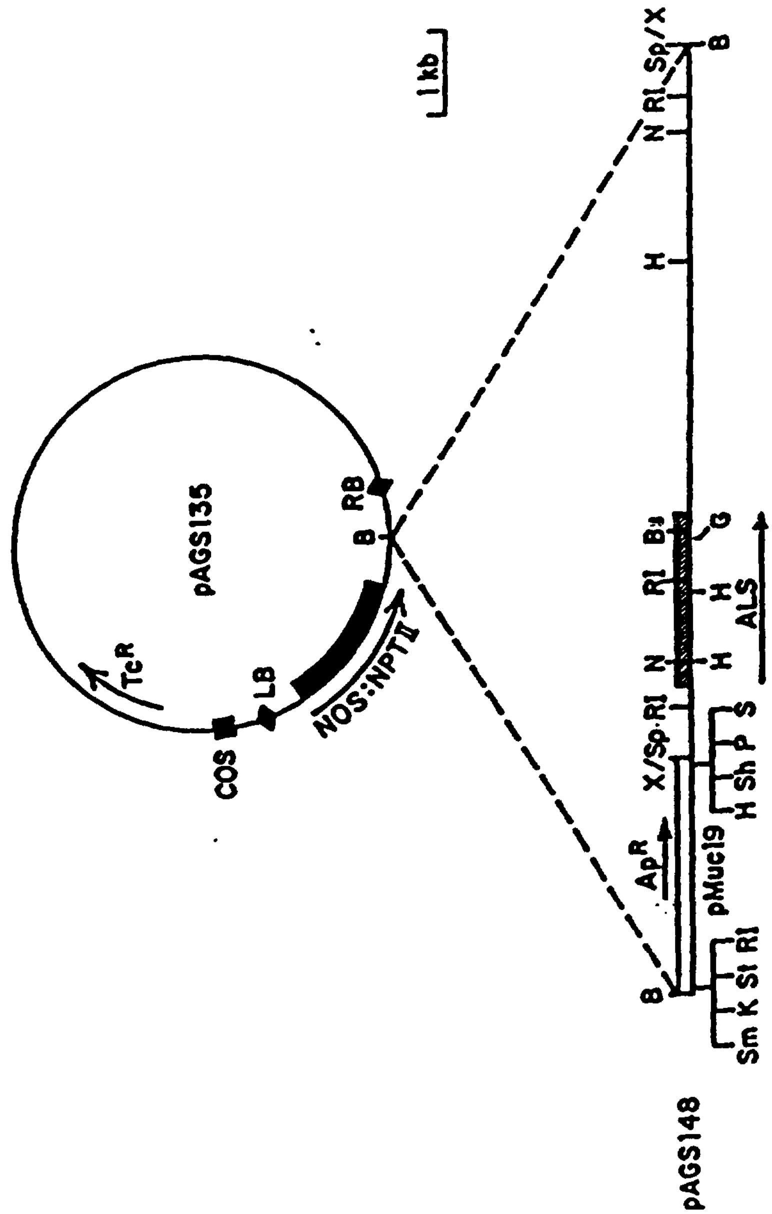

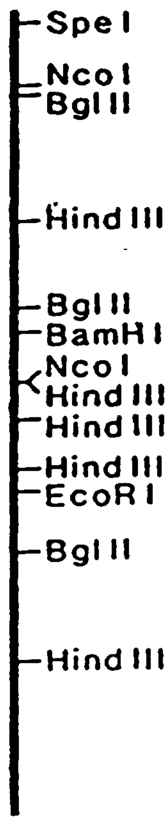
Spe I
Nco I
Bgl II
Hind III
Bgl II
BamH I
Nco I
Hind III
Hind III
Hind III
EcoR I
Bgl II
Hind III

FIG.3

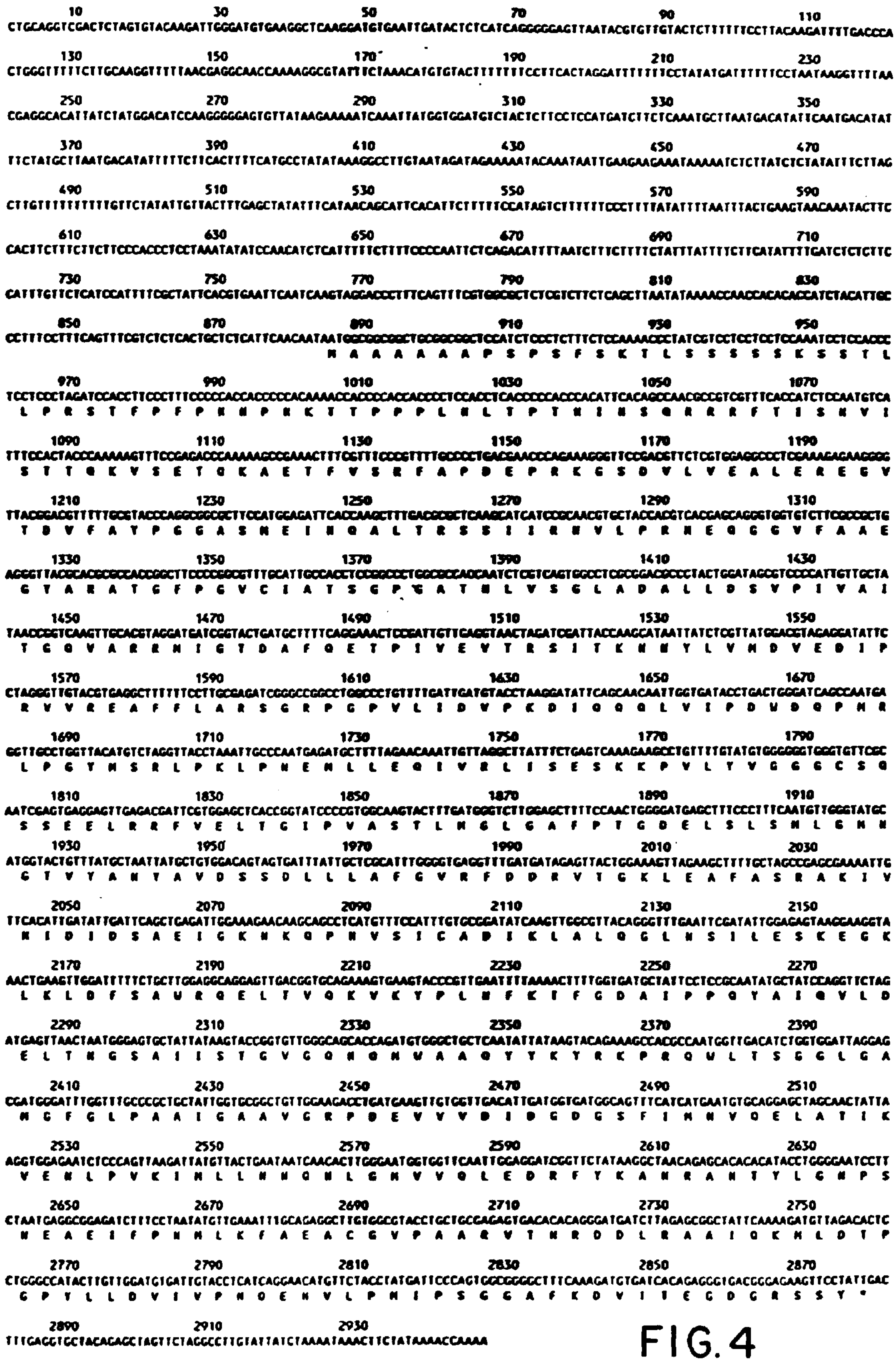

FIG. 4

10 30 50 70 90 110
GGATCCCTCTTTCATTTGTTCTCATCCATTTTTGCGATTCATGTGCATTTAATCAGTAGGACCCCTTTTTAGCTTAGTAGTGCTCTCATGTTCTCAACTTAATATTAAACCAACCACACT

130 150 170 190 210 230
CCATCTGCATTACCCTCCTTCCAGTTTCGTCTCTCCCTGCCCTCCCCTTCAACAATGGCGGCGGCGGCTCCATCTCCCTCTTCTTCCGCTTTCTCCAAAACCCTATCGCCTTCCTCCTCC
M A A A A P S P S S S A F S K T L S P S S S

250 270 290 310 330 350
ACATCCTCCACCCTCCTCCCTAGATCAACCTTCCCTTTCCCCCACCACCCCCACAAGACCACCCCCACCACCCCTCCACCTCACCCACACTCACATTCACATTCACAGCCAACGCCGTCGT
T S S T L L P R S T F P F P H H P H K T T P P P L H L T H T H I H I H S Q R R R

370 390 410 430 450 470
TTCACCATATCCAATGTCATTTCCACTAACCAAAAAGTTTCCCAGACCGAAAAAACCGAAACTTTCGTTTCCCGTTTTGCTCCTGACGAACCCAGAAAGGGTTCCGACGTTCTCGTGGAG
F T I S N V I S T N Q K V S Q T E K T E T F V S R F A P D E P R K G S D V L V E

490 510 530 550 570 590
GCTCTCGAAAGAGAAGGGGTTACGGACGTCTTTGCGTACCCAGGTGGCGCTTCCATGGAGATTCACCAAGCTTTGACCCGTTCAAGCATCATCCGCAACGTGCTGCCACGTCACGAGCAG
A L E R E G V T D V F A Y P G G A S M E I H Q A L T R S S I I R N V L P R H E Q

610 630 650 670 690 710
GGCGGTGTCTTCGCCGCTGAGGGTTACGCACGCGCCACCGGATTTCCCGGCGTTTGCATTGCCACCTCTGGCCCCGGCGCCACCAATCTCGTCAGCGGCCTCGCTGACGCGCTACTGGAT
G G V F A A E G Y A R A T G F P G V C I A T S G P G A T N L V S G L A D A L L D

730 750 770 790 810 830
AGCGTCCCCATTGTTGCTATAACAGGTCAAGTGCAACGTAGGATGATAGGTACTGATGCTTTTCAGGAAACTCCTATTGTTGAGGTAACTAGATCGATTACCAAGCATAATTATCTCGTT
S V P I V A I T G Q V Q R R M I G T D A F Q E T P I V E V T R S I T K H N Y L V

850 870 890 910 930 950
ATGGACGTAGAGGATATTCCTAGGGTTGTACGTGAAGCTTTTTTCCTCGCGAGATCGGGCCGGCCTGGCCCTATTTTGATTGATGTACCTAAGGATATTCAGCAACAATTGGTGATACCT
M D V E D I P R V V R E A F F L A R S G R P G P I L I D V P K D I Q Q Q L V I P

970 990 1010 1030 1050 1070
GACTGGGATCAGCCAATGAGGTTACCTGGTTACATGTCTAGGTTGCCTAAATTGCCCAATGAGATGCTTTTAGAACAAATTGTTAGGCTTATTTCTGAGTCAAAGAAGCCTGTTTTGTAT
D W D Q P M R L P G Y M S R L P K L P N E M L L E Q I V R L I S E S K K P V L Y

1090 1110 1130 1150 1170 1190
GTGGGGGGTGGGTGTTCGCAATCGAGTGAGGACTTGAGACGATTCGTGGAGCTCACGGGTATCCCCGTGGCAAGTACTTTGATGGGTCTTGGAGCTTTTCCAACTGGGGATGAGCTTTCC
V G G G C S Q S S E D L R R F V E L T G I P V A S T L M G L G A F P T G D E L S

1210 1230 1250 1270 1290 1310
CTTTCAATGTTGGGTATGCATGGTACTGTTTATGCTAATTATGCTGTGGACAGTAGTGATTTGTTGCTCGCATTTGGGGTGAGGTTTGATGATAGAGTTACTGGAAAGTTAGAAGCTTTT
L S M L G M H G T V Y A N Y A V D S S D L L L A F G V R F D D R V T G K L E A F

1330 1350 1370 1390 1410 1430
GCTAGCCGAGCAAAAATTGTTCACATTGATATTGATTCAGCTGAGATTGGAAAGAACAAGCAGCCTCATGTTTCCATTTGTGCAGATATCAAGTTGGCGTTACAGGGTTTGAATTCGATA
A S R A K I V H I D I D S A E I G K N K Q P H V S I C A D I K L A L Q G L N S I

1450 1470 1490 1510 1530 1550
CTGGAGAGTAAGGAAGGTAAACTGAAGTTGGATTTTTCTGCTTGGAGGCAGGAGTTGACGGAGCAGAAAGTGAAGCACCCATTGAACTTTAAAACTTTTGGTGATGCAATTCCTCCGCAA
L E S K E G K L K L D F S A W R Q E L T E Q K V K H P L N F K T F G D A I P P Q

1570 1590 1610 1630 1650 1670
TATGCTATCCAGGTTCTAGATGAGTTAACTAATGGGAATGCTATTATAAGTACTGGTGTGGGGCAACACCAGATGTGGGCTGCTCAATACTATAAGTACAGAAAGCCACGCCAATGGTTG
Y A I Q V L D E L T N G N A I I S T G V G Q H Q M W A A Q Y Y K Y R K P R Q W L

1690 1710 1730 1750 1770 1790
ACATCTGGTGGATTAGGAGCAATGGGATTTGGTTTGCCCGCTGCTATTGGTGCGGCTGTTGGAAGACCGGATGAAGTTGTGGTTGACATTGATGGTGATGGCAGTTTCATCATGAATGTG
T S G G L G A M G F G L P A A I G A A V G R P D E V V V D I D G D G S F I M N V

1810 1830 1850 1870 1890 1910
CAGGAGCTTGCAACAATTAAGGTGGAGAATCTCCCAGTTAAGATTATGTTACTGAATAATCAACACTTGGGAATGGTGGTTCAATGGGAGGATCGGTTCTATAAGGCTAACAGAGCACAC
Q E L A T I K V E N L P V K I M L L N N Q H L G M V V Q W E D R F Y K A N R A H

1930 1950 1970 1990 2010 2030
ACATACCTGGGGAATCCTTCTAATGAGGCGGAGATCTTTCCTAATATGCTGAAATTTGCAGAGGCTTGTGGCGTACCTGCTGCAAGAGTGACACATAGGGATGATCTTAGAGCTGCCATT
T Y L G N P S N E A E I F P N M L K F A E A C G V P A A R V T H R D D L R A A I

2050 2070 2090 2110 2130 2150
CAGAAGATGTTAGACACTCCTGGGCCATACTTGTTGGATGTGATTGTACCTCATCAGGAACATGTTTTACCTATGATTCCCAGTGGCGGAGCTTTCAAAGATGTGATCACAGAGGGTGAC
Q K M L D T P G P Y L L D V I V P H Q E H V L P M I P S G G A F K D V I T E G D

2170 2190 2210 2230 2250 2270
GGGAGAAGTTCCTATTGAGTTTGAGAAGCTACAGAGCTAGTTCTAGGCCTTGTATTATCTAAAATAAACTTCTATTAAGCCAAACATGTTCTGTCTATTAGTTTGTTGTTAGTTTTTGCT
G R S S Y *

2290 2310 2330 2350 2370 2390
GTGGCTTTGCTCGTTGTCACTGTTGTACTATTAAGTAGTTGATATTTATGTTTGCTTTAAGTTTTGCATCATCTCCCTTTGGTTTTGAATGTGAAGGATTTCAGCAAAGTTTCATTCTCT

2410 2430 2450 2470 2490 2510
GTTTGCAACATCCACTTGGTATCTGGAGATTAATTTCTAGTGGAGTAGTTTAGTGCGATAAAATTAGCTTGTTCCACATTTTTATTTCGTAAGCTATGTTAGGCTGGGTCAGATTGGAAC

FIG.5

# FIG. 6

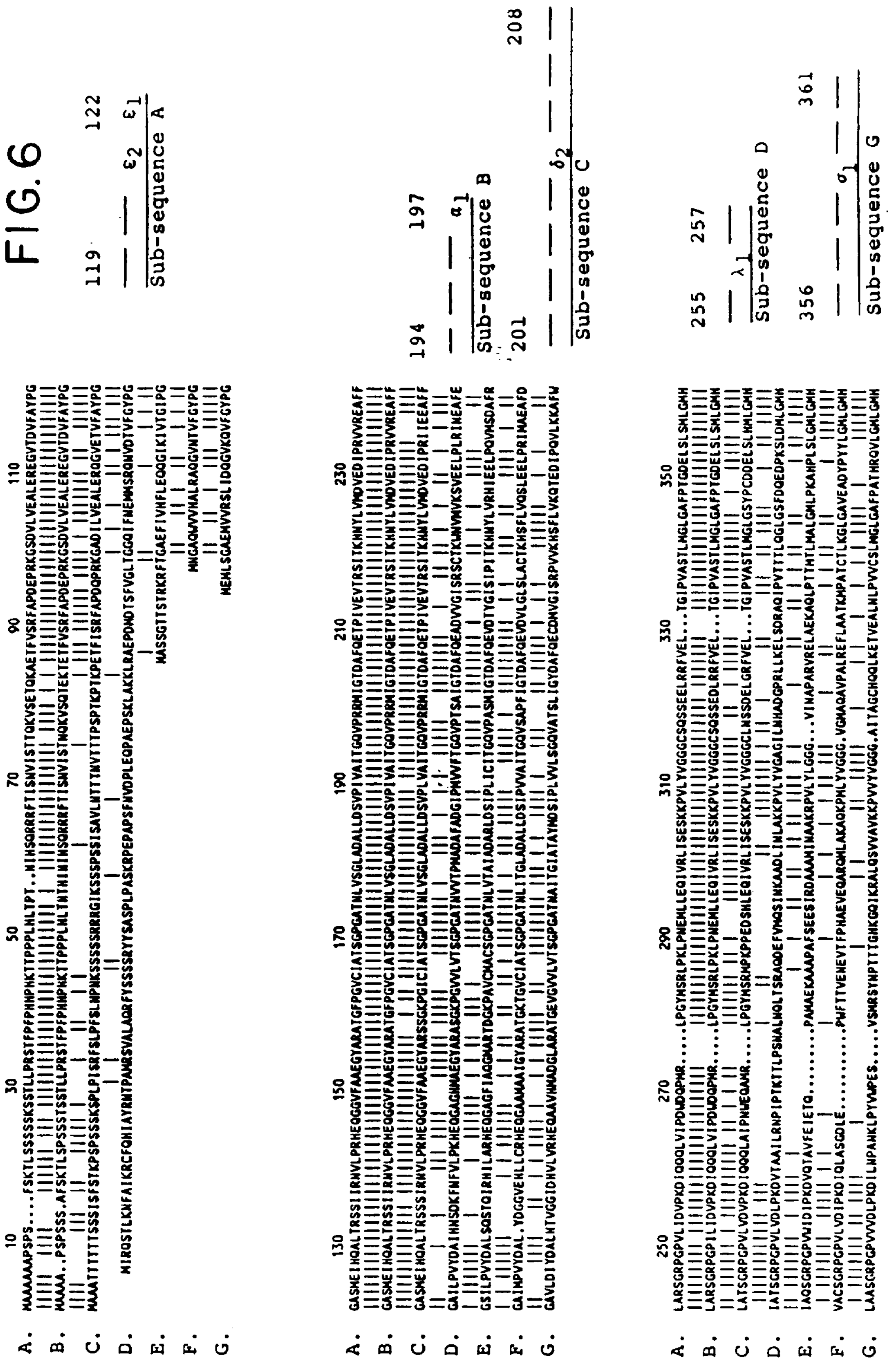

Sub-sequence A
119
122
Sub-sequence B
194
197
Sub-sequence C
201
208
Sub-sequence D
255
257
Sub-sequence G
356
361
A.
B.
C.
D.
E.
F.
G.

# FIG. 6

```
          370                 390                 410                 430                 450                 470
A.  GTVYANYAVDSSDLLLAFGVRFDDRVTGKLEAFASRAKIVHIDIDSAEIGKNKQPHVSICADIKLALQGLNSILESKEGKLKLDFSAWRQELTVQKVKYPLNFKTF.........GDAIP
B.  GTVYANYAVDSSDLLLAFGVRFDDRVTGKLEAFASRAKIVHIDIDSAEIGKNKQPHVSICADIKLALQGLNSILESKEGKLKLDFSAWRQELTEQKVKHPLNFKTF.........GDAIP
C.  GTVYANYAVEHSDLLLAFGVRFDDRVTGKLEAFASRAKIVHIDIDSAEIGKNKTPHVSVCGDVKLALQGMNKVLENRAEELKLDFGVWRNELNVQKQKFPLSFKTF.........GEAIP
D.  GCATANLAVQNADLIIAVGARFDDRVTGNISKFAPEARRAAAEGRGGIINFEVSPKNINKVVQTQIAVEGDATTNLGKMMSKIFPVKERSEWFAQINKWKKEYPYAYMEETPGSKIKPQT
E.  GVRSTNYILQEADLLIVLGARFDDRAIGKTEQFCPNAKIIHVDIDRAQLGKIKQPHVAIQADVDDVLAQLIPLVEAQPRAEWHQLVADLQREFPCPIPKA...............CDPLS
F.  GTKAANFAVQECDLLIAVGARFDDRVTGKLNTFAPHASVIHMDIDPAEMNKLRQAHVALQGDLNALLPALQQPLNQYDWQQHCAQLRDEHSWRYDHP..................GDAIY
G.  GTYEANMTMHNADVIFAVGVRFDDRTTNNLAKYCPNATVLHIDIDPTSISKTVTADIPIVGDARQVLEQMLELLSQESAHQPLDEIRDWWQQIEQWRARQCLKYDTH........SEKIK

          490                 510                 530                 550                 570                 590
A.  PQYAIQVLDELTNGSAIISTGVGQHQMWAAQYYKYRKPRQWLTSGGLGAMGFGLPAAIGAAVGRPDEVVVDIDGDGSFIMNVQELATIKVENLPVKIMLLNNQHLGMVVQWEDRFYKANR
B.  PQYAIQVLDELTNGNAIISTGVGQHQMWAAQYYKYRKPRQWLTSGGLGAMGFGLPAAIGAAVGRPDEVVVDIDGDGSFIMNVQELATIKVENLPVKIMLLNNQHLGMVVQWEDRFYKANR
C.  PQYAIKVLDELTDGKAIISTGVGQHQMWAAQFYNYKKPRQWLSSGGLGAMGFGLPAAIGASVANPDAIVVDIDGDGSFIMNVQELATIRVENLPVKVLLLNNQHLGMVMQWEDRFYKANR
D.  VIKKLSKVANDTGRHVIVTTGVGQHQMWAAQHWTWRNPHTFITSGGLGTMGYGLPAAIGAQVAKPESLVIDIDGDASFNMTLTELSSAVQAGTPVKILILNNEEQGMVTQWQSLFYEHRY
E.  HYGLINAVAACVDDNAIITTDVGQHQMWTAQAYPLNRPRQWLTSGGLGTMGFGLPAAIGAALANPDRKVLCFSGDGSLMMNIQEMATASENQLDVKIILMNNEALGLVHQQQSLFYEQGA
F.  APLLLKQLSDRKPADCVVTTDVGQHQMWAAQHIAHTRPENFITSSGLGTMGFGLPAAVGAQVARPNDTVVCISGDGSFMMNVQELGTVKRKQLPLKIVLLDNQRLGMVRQWQQLFFQERY
G.  PQAVIETLWRLTKGDAYVTSDVGQHQMFAALYYPFDKPRRWINSGGLGSMGFGLPAALGVKMALPEETVVCVTGDGSIQMNIQELSTALQYELPVLVVNLNNRYLGMVKQWQDMIYSGRH

          610                 630                 650                 670                 690
A.  AHTYLGNPSNEAEIFPNMLKFAEACGVPAARVTHRDDLRAAIQKMLDTPGPYLLDVIVPHQEHVLPMIPSGGAFKDVITEGDGRSSY
B.  AHTYLGNPSNEAEIFPNMLKFAEACGVPAARVTHRDDLRAAIQKMLDTPGPYLLDVIVPHQEHVLPMIPSGGAFKDVITEGDGRSSY
C.  AHTFLGDPAQEDEIFPNMLLFAAACGIPAARVTKKADLREAIQTMLDTPGPYLLDVICPHQEHVLPMIPSGGTFNDVITEGDGRIKY
D.  SHTHQL........NPDFIKLAEAMGLKGLRVKKQEELDAKLKEFVSTKGPVLLEVEVDKKVPVLPMVAGGSGLDEFINFDPEVERQQTELRHKRTGGKH
E.  FVATYP.......GKINFMQIAAGFGLETCDLNNEADPQASLQEIINRPGPALIHVRIDAEEKVYPMVPPGAANTEMVGE
F.  SETILT.......DNPDFLMLASAFGIHGQHITRKDQVEAALDTMLNSDGPYLLHVSIDELENVWPLVPPGASNSEMLEKLS
G.  SQSYMQ.....SLPDFVRRGAYGHVGIQISHPHGWKANLARRWNRCAIIAWCLLMLPSMAASTSTRCRFAGAEWMKCG
```

381 385

— — — $\gamma_1$ —

Sub-sequence E

586 595

— — $\beta_8$ — — $\beta_3$ — — — $\beta_7$

Sub-sequence F

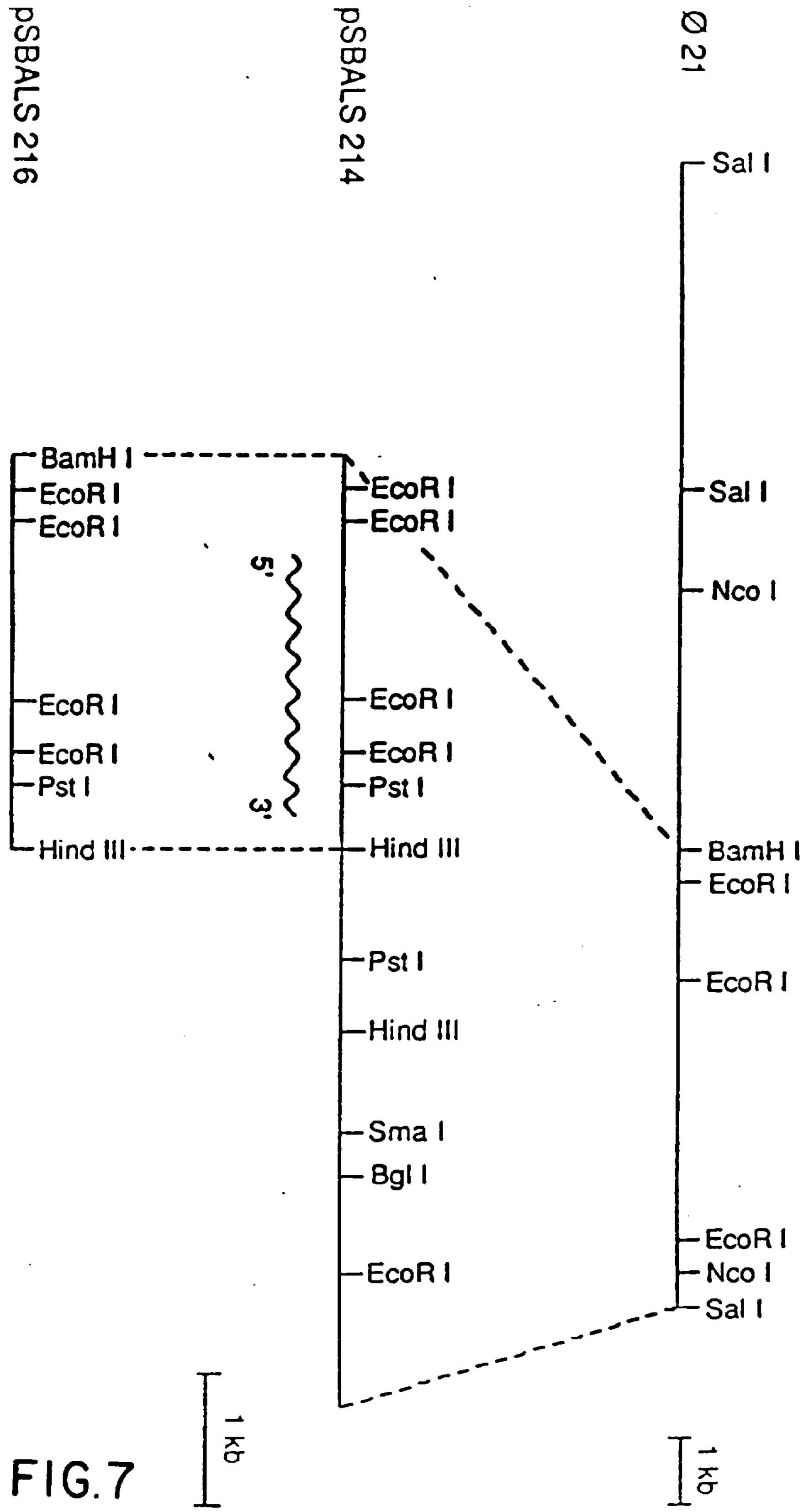
pSBALS 216
pSBALS 214
Ø 21
Sal I
BamH I
EcoR I
EcoR I
EcoR I
EcoR I
Pst I
Hind III
5'
3'
EcoR I
EcoR I
EcoR I
EcoR I
Pst I
Hind III
Pst I
Hind III
Sma I
Bgl I
EcoR I
Sal I
Nco I
BamH I
EcoR I
EcoR I
EcoR I
Nco I
Sal I
1 kb
1 kb

FIG.7

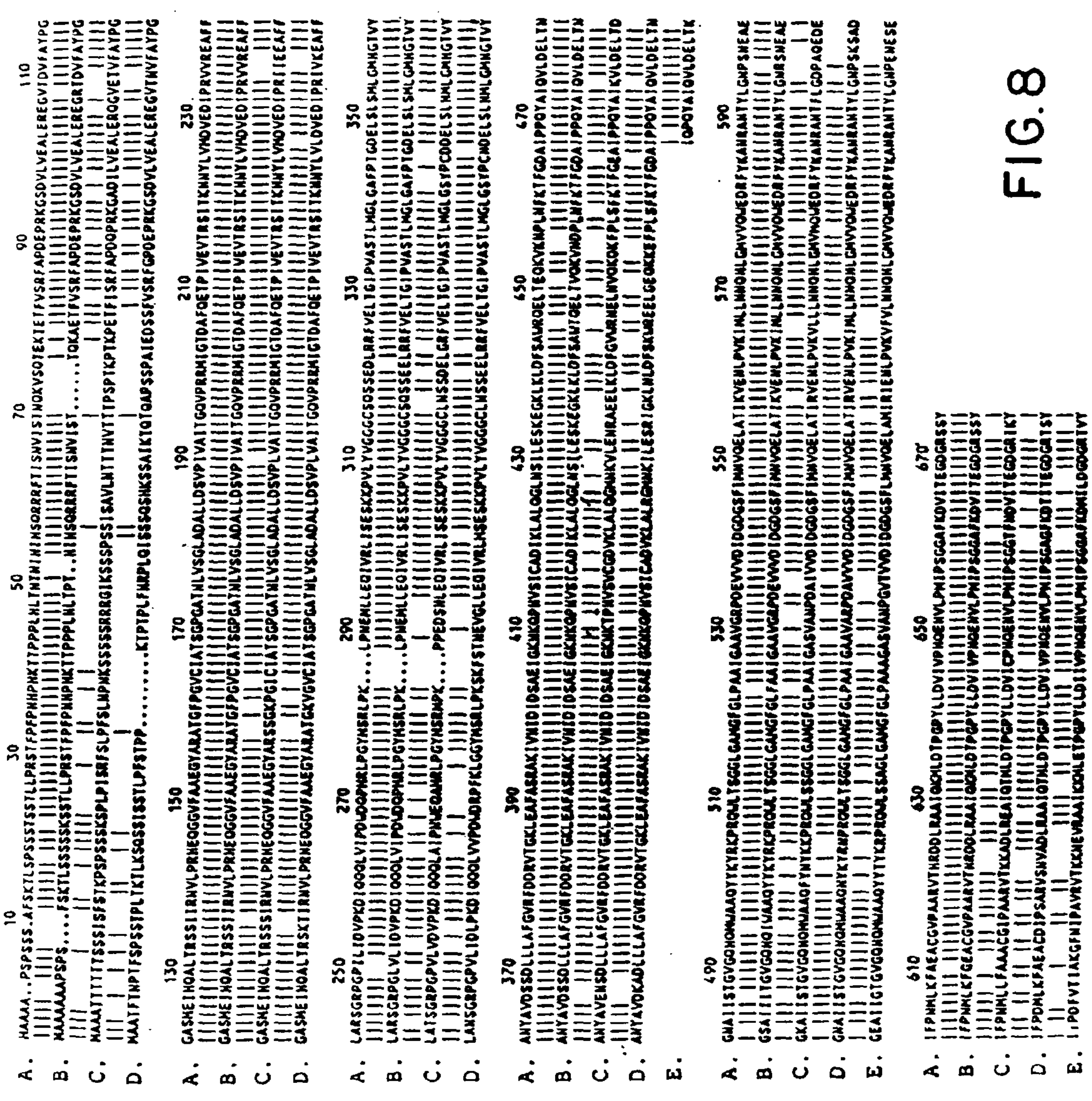

FIG. 8